# EUROPEAN PATENT SPECIFICATION

(11) **EP 1 037 881 B1**
(45) Date of publication and mention of the grant of the patent: **12.11.2003**
(21) Application number: 98963791.3
(22) Date of filing: 04.12.1998
(51) Int. Cl.: C07D 239/56, C07D 401/04

(54) **PROCESS OF PREPARING SUBSTITUTED PYRIMIDINONES**
VERFAHREN ZUR HERSTELLUNG VON SUBSTITUIERTEN PYRIMIDINONEN
PROCEDE SERVANT A PREPARER DES PYRIMIDINONES SUBSTITUEES

(30) Priority: 04.12.1997 US 67513 P; 03.12.1998 US 204569
(43) Date of publication of application: 27.09.2000
(73) Proprietor: Amgen Inc., Thousand Oaks, CA 91320-1799 (US)
(72) Inventor: BURGESS, Laurence, E., Boulder, CO 80301 (US); KOCH, Kevin, Boulder, CO 80301 (US)
(74) Representative: Richardson, Kate
(86) International application number: US9825790
(87) International publication number: WO99028303

(56) References cited:
- US-A- 5 100 897
- US-A- 5 518 994
- T. HIYAMA ET. AL.: "A new synthesis of 3-amino-2-alkenoates" TETRAHEDRON LETTERS, vol. 23, no. 15, April 1982, pages 1597-1600, XP002100269

## Description

### BACKGROUND OF THE INVENTION

The present invention relates to a process useful in the preparation of synthetic intermediates useful in the preparation of compounds (a) which are useful in treating diseases, such as TNF-α, IL-1β, IL-6 and/or IL-8 mediated diseases and other maladies, such as described in U.S. patent application 08/984,774 and corresponding PCT patent application WO 98/24782 (PCT/US97/22390) both filed December 4, 1997, and U.S. patent application 08/985,346 and corresponding PCT patent application WO 98/24780 (PCT/US97/22949) both filed December 4, 1997, (b) which are angiotensin II antagonists, such as described in US 5,100,897, Canadian Patent Application Serial no. 2,020,370, and European Patent Application Serial no. 481, 448, and (c) which are herbicides, such as described in US 5,300,477 and US 5,518,994.

US 5,518,994 discloses the preparation of 2-arylaminopyrimidmone derivatives as herbicides and plant growth regulators.

### BRIEF DESCRIPTION OF THE INVENTION

The present invention relates to processes useful in the preparation of intermediates useful in the preparation of classes of compounds (a) which are useful in treating diseases, such as TNF-α, IL-1β, IL-6 and/or IL-8 mediated diseases and other maladies, such as described in U.S. patent application 08/984,774 and corresponding PCT patent application WO 98/24782 (PCT/US97/22390) both filed December 4, 1997, and U.S. patent application 08/985,346 and corresponding PCT patent application WO 98/24780 (PCT/US97/22949) both filed December 4, 1997, (b) which are angiotensin II antagonists, such as described in US 5,100,897, Canadian Patent Application Serial no. 2,020,370, and European Patent Application Serial, no. 481,448, and (c) which are herbicides, such as described in US 5,300,477 and US 5,518,994.

In particular, the process of the invention permits large scale or bulk preparations of such compounds.

The compounds of the invention are represented by the following general structure: 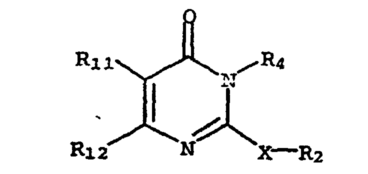 wherein X, R₂, R₄, R₁₁ and R₁₂ are defined below.

The foregoing merely summarizes certain aspects of the invention and is not intended, nor should it be construed, as limiting the invention in any way. All patents and other publications recited herein are hereby incorporated by reference in their entirety.

### DETAILED DESCRIPTION OF THE INVENTION

In accordance with the present invention, there is provided a process for preparing compounds of formula I 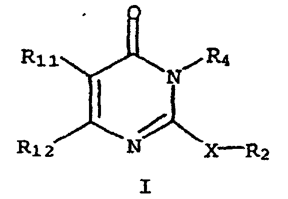 comprising (a) mixing R₁₁-CH₂-C(O)L and R₁₂-CN in a suitable solvent and in the presence of base; and (b) adding R₄-N=C=X; or comprising (a) mixing R₁₁-CH₂-C(O)L and R₁₂-CN in a suitable solvent and in the presence of base; (b) adding R₄-N=C=X; and (c) adding R₂-L'; wherein L, L', X, R₂, R₄, R₁₁ and R₁₂ are defined below.

In accordance with the present invention, there is provided compounds of the formula I wherein
X is O or S; and most preferably, X is S;
R₂ is
(1) a hydrogen radical; or
(2) Q; or
(3) alkyl or alkenyl radical optionally substituted by (a) 1-3 radicals of halo, alkanoyl, aroyl, alkoxycarbonyl, aryloxycarbonyl, aminocarbonyl, alkylaminocarbonyl, dialkylaminocarbonyl, aminosulfonyl, alkylaminosulfonyl, dialkylaminosulfonyl, alkylsulfonyl, arylsulfonyl, heteroarylsulfonyl, hydroxy, alkoxy, cyano, and (b) 1-2 radicals of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, halo, alkyl, trifluoromethoxy or trifluoromethyl radicals; or
(4) heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl, trifluoromethoxy or trifluoromethyl radicals;
preferably, R₂ is
(1) a hydrogen radical; or
(2) Q; or
(3) C₁-C₈ alkyl or C₂-C₈ alkenyl radical optionally substituted by (a) 1-3 radicals of halo, C₁-C₅ alkanoyl, aroyl, C₁-C₄ alkoxycarbonyl, aryloxycarbonyl, aminocarbonyl, C₁-C₄ alkylaminocarbonyl, di-(C₁-C₄ alkyl)aminocarbonyl, aminosulfonyl, C₁-C₄ alkylaminosulfonyl, di-(C₁-C₄ alkyl)aminosulfonyl, C₁-C₄ alkylsulfonyl, arylsulfonyl, heteroarylsulfonyl, hydroxy, C₁-C₄ alkoxy, cyano, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluoromethoxy or trifluoromethyl radicals; or
(4) heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, trifluoromethoxy or trifluoromethyl radicals;
more preferably, R₂ is a hydrogen radical, Q, or a C₁-C₈ alkyl radical optionally substituted by (a) 1-3 radicals of halo, aroyl, C₁-C₄ alkoxycarbonyl, aryloxycarbonyl, C₁-C₄ alkylaminosulfonyl, di-(C₁-C₄ alkyl)aminosulfonyl, C₁-C₄ alkylsulfonyl, arylsulfonyl, heteroarylsulfonyl, C₁-C₄ alkoxy, cyano, and (b) 1-2 radicals of heterocyclyl, aryl or heteroaryl optionally substituted by 1-3 radicals of amino, C₁-C₄ alkylamino, di-(C₁-C₄ alkyl)amino, hydroxy, C₁-C₄ alkoxy, halo, C₁-C₄ alkyl, or trifluoromethyl radicals; or
more preferably, R₂ is a hydrogen radical, Q, C₁-C₄ alkyl radical optionally substituted by 1-3 radicals of halogen; most preferably, R₂ is Q or a methyl or ethyl radical;
Q is a Li⁺, Na⁺, K⁺, Mg⁺², Zn⁺², Cu⁺², or a tetra C₁-C₄ alkylammonium cation; and preferably, Q is a Li⁺, Na⁺, or K⁺ cation;
R₄ is -Z-Y; provided that the total number of aryl, heteroaryl, cycloalkyl and heterocyclyl radicals in -Z-Y is 0-3; preferably, 0-2; more preferably, 0-1;
wherein Z is a
(1) alkyl, alkenyl or alkynyl radical optionally substituted by (a) 1-3 radicals of amino, alkylamino, dialkylamino, alkanoylamino, alkoxycarbonylamino, alkylsulfonylamino, hydroxy, alkoxy, alkylthio or halo, and (b) 1-2 radicals of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, alkyl or haloalkyl; or
(3) aryl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
preferably, Z is a
(1) C₁-C₈ alkyl, C₂-C₈ alkenyl or C₂-C₈ alkynyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
more preferably, Z is a
(1) C₁-C₈ alkyl, C₂-C₈ alkenyl or C₂-C₈ alkynyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or C₁-C₄ haloalkyl of 1-3 halo radicals;
(2) heterocyclyl radical optionally substituted by 1-2 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxyl C₁-C₄ alkylthio, C₁-C₄ alkyl or C₁-C₄ haloalkyl of 1-3 halo radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
more preferably, Z is a
(1) C₁-C₈ alkyl or C₂-C₈ alkenyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, di-(C₁-C₄ alkyl)amino, (C₁-C₄ alkoxy)carbonylamino, hydroxy, C₁-C₄ alkoxy, C₁-C₄ alkylthio or C₁-C₄ alkyl radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₂ haloalkyl of 1-3 halo radicals;
more preferably, Z is a
(1) C₁-C₄ alkyl or C₂-C₅ alkenyl radical optionally substituted by (a) 1-3 radicals of amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio or halo, and (b) 1-2 radicals of heterocyclyl, aryl or heteroaryl optionally substituted by 1-3 radicals of amino, C₁-C₄ alkylamino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of amino, di-(C₁-C₂ alkyl)amino, (C₁-C₄ alkoxy)carbonylamino, hydroxy, C₁-C₂ alkoxy, C₁-C₂ alkylthio or C₁-C₄ alkyl radicals; or
(3) aryl or heteroaryl radical optionally substituted by 1-3 radicals of amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
more preferably, Z is a
(1) C₁-C₄ alkyl or C₂-C₅ alkenyl radical optionally substituted by (a) 1-3 radicals of amino, di-(C₁-C₂ alkyl)amino, (C₁-C₄ alkoxy)carbonylamino, hydroxy, C₁-C₂ alkoxy, C₁-C₂ alkylthio or halo, and (b) 1-2 radicals of aryl or heteroaryl optionally substituted by 1-2 radicals of amino, di-(C₁-C₂ alkyl)amino, acetamido, (C₁-C₄ alkoxy)carbonylamino, hydroxy, C₁-C₂ alkoxy, C₁-C₂ alkylthio, halo, C₁-C₄ alkyl or trifluoromethyl radicals; or
(2) aryl or heteroaryl radical optionally substituted by 1-3 radicals of amino, di-(C₁-C₂ alkyl)amino, acetamido, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
more preferably, Z is a C₁-C₄ alkyl radical optionally substituted by 1-2 radicals of amino, di-(C₁-C₂ alkyl)amino, (C₁-C₄ alkoxy)carbonylamino, hydroxy, C₁-C₂ alkoxy, C₁-C₂ alkylthio, halo or aryl or heteroaryl optionally substituted by 1-2 radicals of hydroxy, C₁-C₂ alkoxy, C₁-C₂ alkylthio, halo, C₁-C₄ alkyl or trifluoromethyl radicals; and
most preferably, Z is a C₁-C₄ alkyl radical optionally substituted by 1-2 radicals of amino, t-butoxycarbonylamino, dimethylamino, hydroxy, methoxy, methylthio or halo radicals;
Y is a
(1) hydrogen radical;
(2) halo or nitro radical;
(3) -C(O)-R₂₀ or -C(NR₅)-NR₅R₂₁ radical;
(4) -OR₂₁, -O-C(O)-R₂₁, -O-C(O)-NR₅R₂₁ or -O-C(O)-NR₂₂-S(O)₂-R₂₀ radical;
(5) -SR₂₁, -S(O)-R₂₀, -S(O)₂-R₂₀, -S(O)₂-NR₅R₂₁, -S(O)₂-NR₂₂-C(O)-R₂₁, -S(O)₂-NR₂₂-C(O)-OR₂₀ or -S(O)₂-NR₂₂-C(O)-NR₅R₂₁ radical; or
(6) -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ or -NR₂₂-S(O)₂-NR₅R₂₁ radical;
preferably, Y is a
(1) hydrogen radical;
(2) halo radical;
(3) -C(O)-R₂₀ or -C(NR₅)-NR₅R₂₁ radical;
(4) -OR₂₁, -O-C(O)-R₂₁ or -O-C(O)-NR₅R₂₁ radical;
(5) -SR₂₁, -S(O)-R₂₀, -S(O)₂-R₂₀ or -S(O)₂-NR₅R₂₁ radical; or
(6) -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ or -NR₂₂-S(O)₂-NR₅R₂₁ radical;
more preferably, Y is a
(1) hydrogen radical;
(2) -C(O)-R₂₀ radical;
(3) -OR₂₁, -SR₂₁, -S(O)-R₂₀, -S(O)₂-R₂₀ or -S(O)₂-NR₅R₂₁ radical; or
(4) -NR₅R₂₁, -NR₂₂-C(O)-R₂₁, -NR₂₂-C(O)-OR₂₀, -NR₂₂-C(O)-NR₅R₂₁, -NR₂₂-S(O)₂-R₂₀ or -NR₂₂-S(O)₂-NR₅R₂₁ radical;
more preferably, Y is a
(1) hydrogen radical;
(2) -C(O)-R₂₀ radical;
(3) -OR₂₁, -SR₂₁, -S(O)-R₂₀, -S(O)₂-R₂₀ or -S(O)₂-NR₅R₂₁ radical; or
(4) -NR₅R₂₁, -NR₂₂-C(O)-R₂₁ or -NR₂₂-S(O)₂-R₂₀ radical;
more preferably, Y is a
(1) -C(O)-R₂₀ radical;
(2) -OR₂₁, -SR₂₁, -S(O)-R₂₀, -S(O)₂-R₂₀ or -S(O)₂-NR₅R₂₁ radical; or
(3) -NR₅R₂₁, -NR₂₂-C(O)-R₂₁ or -NR₂₂-S(O)₂-R₂₀ radical;
most preferably, Y is a -OR₂₁, -SR₂₁ or -NR₅R₂₁ radical;
alternatively, preferably, R₄ is
(1) C₁-C₈ alkyl or C₂-C₈ alkenyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluoromethoxy or trifluoromethyl radicals; or
(2) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, trifluoromethoxy or trifluoromethyl radicals;
more preferably, R₄ is
(1) C₁-C₈ alkyl radical optionally substituted by 1-2 radicals of amino, C₁-C₄ alkylamino, di-(C₁-C₄ alkyl)amino, hydroxy, C₁-C₄ alkoxy or aryl or heteroaryl optionally substituted by 1-3 radicals of amino, C₁-C₄ alkylamino, di-(C₁-C₄ alkyl)amino, C₁-C₄ alkoxy, C₁-C₄ alkylthio, halo, C₁-C₄ alkyl, trifluoromethoxy or trifluoromethyl radicals; or
(2) aryl or heteroaryl radical optionally substituted by 1-3 radicals of amino, C₁-C₄ alkylamino, di-(C₁-C₄ alkyl)amino, C₁-C₄ alkoxy, C₁-C₄ alkylthio, cyano, halo, C₁-C₄ alkyl, trifluoromethoxy or trifluoromethyl radicals;
more preferably, R₄ is a C₁-C₈ alkyl radical optionally substituted by 1-2 radicals of amino, C₁-C₄ alkylamino, di-(C₁-C₄ alkyl)amino, hydroxy, C₁-C₄ alkoxy or aryl or heteroaryl optionally substituted by 1-3 radicals of amino, C₁-C₄ alkylamino, di-(C₁-C₄ alkyl)amino, C₁-C₄ alkoxy, C₁-C₄ alkylthio, halo, C₁-C₄ alkyl, trifluoromethoxy or trifluoromethyl radicals;
more preferably, R₄ is a C₁-C₄ alkyl radical; most preferably, R₄ is a methyl or ethyl radical;
wherein each R₅ is independently
(1) hydrogen radicals;
(2) alkyl, alkenyl or alkynyl radicals optionally substituted by 1-3 radicals of amino, alkylamino, dialkylamino, hydroxy, alkoxy, alkylthio, -SO₃H or halo; or
(3) aryl, heteroaryl, aralkyl, heteroaralkyl, heterocyclyl, heterocyclylalkyl, cycloalkyl or cycloalkylalkyl radicals optionally substituted by 1-3 radicals of amino, alkylamino, dialkylamino, hydroxy, alkoxy, alkylthio, alkyl or haloalkyl;
preferably, each R₅ is independently
(1) hydrogen radicals;
(2) C₁-C₈ alkyl, C₂-C₈ alkenyl or C₂-C₈ alkynyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, -SO₃H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
more preferably, each R₅ is independently
(1) hydrogen radicals;
(2) C₁-C₄ alkyl, C₂-C₅ alkenyl or C₂-C₅ alkynyl radicals. optionally substituted by 1-3 radicals of amino, C₁-C₄ alkylamino, di-(C₁-C₄-alkyl)amino, hydroxy, C₁-C₄ alkoxy, C₁-C₄ alkylthio, -SO₃H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
more preferably, each R₅ is independently
(1) hydrogen radicals;
(2) C₁-C₄ alkyl or C₂-C₅ alkenyl radicals optionally substituted by 1-3 radicals of amino, di-(C₁-C₄-alkyl)amino, hydroxy, C₁-C₄ alkoxy, C₁-C₄ alkylthio, -SO₃H or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₄-alkyl)amino, hydroxy, C₁-C₄ alkoxy, C₁-C₄ alkylthio, C₁-C₄ alkyl or C₁-C₂ haloalkyl of 1-3 halo radicals;
more preferably, each R₅ is independently
(1) hydrogen radical;
(2) C₁-C₄ alkyl radical optionally substituted by 1-3 radicals of amino, di-(C₁-C₂-alkyl)amino, hydroxy, C₁-C₂ alkoxy, C₁-C₂ alkylthio or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₂-alkyl)amino, hydroxy, C₁-C₂ alkoxy, C₁-C₂ alkylthio, methoxy, methylthio, C₁-C₄ alkyl or trifluoromethyl radicals;
more preferably, each R₅ is independently
(1) hydrogen radical;
(2) C₁-C₄ alkyl radical optionally substituted by 1-3 halo radicals; or
(3) phenyl-C₁-C₂-alkyl or heteroaryl-C₁-C₂-alkyl, radicals optionally substituted by 1-3 radicals of amino, dimethylamino, hydroxy, methoxy, methylthio, methyl or trifluoromethyl radicals;
more preferably, each R₅ is independently hydrogen or C₁-C₄ alkyl radical; and most preferably, each R₅ is a hydrogen radical;
wherein each R₂₀ is independently
(1) alkyl, alkenyl or alkynyl radicals optionally substituted by 1-3 radicals of amino, alkylamino, dialkylamino, alkanoylamino, alkoxycarbonylamino, N-(alkoxycarbonyl)-N-(alkyl)amino, aminocarbonylamino, alkylsulfonylamino, hydroxy, alkoxy, alkylthio, alkylsulfinyl, alkylsulfonyl, halo or aralkoxy, aralkylthio, aralkylsulfonyl, cycloalkyl, heterocyclyl, aryl or heteroaryl radicals optionally substituted by 1-3 radicals of amino, alkylamino, dialkylamino, alkanoylamino, alkoxycarbonylamino, alkylsulfonylamino, alkanoyl, hydroxy, alkoxy, alkylthio, alkylsulfinyl, alkylsulfonyl, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, alkoxycarbonyl, hydroxy, alkoxy, alkylthio, cyano, halo, azido, alkyl or haloalkyl;
preferably, each R₂₀ is independently
(1) C₁-C₈ alkyl, C₂-C₈ alkenyl or C₂-C₈ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy) carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
more preferably, each R₂₀ is independently
(1) C₁-C₈ alkyl, C₂-C₅ alkenyl or C₂-C₅ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
more preferably, each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy) carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₂ haloalkyl of 1-3 halo radicals;
more preferably, each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, di-(C₁-C₄ alkyl)amino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-2 radicals of amino, C₁-C₄ alkylamino, di-(C₁-C₄ alkyl)amino, acetamido, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or trifluoromethyl radicals;
more preferably, each R₂₀ is independently
(1) C₁-C₈ alkyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or C₃-C₆ cycloalkyl, heterocyclyl, aryl or heteroaryl radicals optionally substituted by 1-2 radicals of amino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of hydroxy, C₁-C₄ alkoxy, C₁-C₄ alkylthio or C₁-C₄ alkyl; or
(3) aryl or heteroaryl radicals optionally substituted by 1-2 radicals of (C₁-C₄ alkoxy)carbonyl, amino, C₁-C₄ alkylamino, di-(C₁-C₄ alkyl)amino, hydroxy, C₁-C₄ alkoxy, C₁-C₄ alkylthio, cyano, halo, azido, C₁-C₄ alkyl or trifluoromethyl radicals;
more preferably, each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical optionally substituted by 1-2 radicals of hydroxy or C₁-C₄ alkyl; or
(3) aryl or heteroaryl radicals optionally substituted by 1-2 radicals of amino, dimethylamino, hydroxy, methoxy, methylthio, halo, methyl or trifluoromethyl radicals;
more preferably, each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
most preferably, each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
each R₂₂ is independently
(1) hydrogen radical;
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl; or
(3) heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
preferably, each R₂₂ is independently
(1) hydrogen radical;
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals; or
(3) heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
more preferably, each R₂₂ is independently
(1) hydrogen radical; or
(2) C₁-C₄ alkyl radical optionally substituted by a radical of phenyl or heteroaryl optionally substituted by 1-3 radicals of amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₂ haloalkyl of 1-3 halo radicals;
more preferably, each R₂₂ is independently hydrogen or C₁-C₄ alkyl radical; and most preferably, each R₂₂ is independently hydrogen or methyl radical;
R₁₁ and R₁₂ are each independently an alkyl, alkenyl, alkynyl, cycloalkyl, heterocyclyl, aryl or heteroaryl radical optionally substituted by 1-3 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ or -NR₃₃-S(O)₂-NR₃₁R₃₂ radicals; provided that the total number of aryl, heteroaryl, cycloalkyl and heterocyclyl radicals substituted in each of R₁₁ and R₁₂ is 0-1;
preferably, R₁₁ and R₁₂ are each independently an C₁-C₆ alkyl, C₂-C₆ alkenyl, C₂-C₆ alkynyl, C₃-C₈ cycloalkyl, heterocyclyl, aryl or heteroaryl radical optionally substituted by 1-2 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ or -NR₃₃-S(O)₂-NR₃₁R₃₂ radicals; provided that the total number of aryl, heteroaryl, cycloalkyl and heterocyclyl radicals substituted on each of R₁₁ and R₁₂ is 0-1;
more preferably, R₁₁ and R₁₂ are each independently an C₁-C₄ alkyl, C₂-C₄ alkenyl, C₂-C₄ alkynyl, C₃-C₆ cycloalkyl, heterocyclyl, aryl or heteroaryl radical optionally substituted by 1-2 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals; or
(4) -OR₂₉, -SR₂₉, -S(O)-R₃₀, -S(O)₂-R₃₀, -S(O)₂-NR₃₁R₃₂, -NR₃₁R₃₂, -NR₃₃-C(O)-R₂₉ or -NR₃₃-C(O)-OR₃₀ radicals;
more preferably, R₁₁ is an aryl radical and R₁₂ is a heteroaryl radical, wherein the aryl and heteroaryl radicals are optionally substituted by 1-2 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals; or
(4) -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals;
more preferably, R₁₁ is an aryl radical and R₁₂ is a heteroaryl radical, wherein the aryl and heteroaryl radicals are optionally substituted by 1-2 radicals of
(1) R₃₀;
(2) halo radicals; or
(3) -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ or -NR₃₃-C (O) -R₂₉ radicals;
more preferably, R₁₁ is an aryl radical optionally substituted by 1-2 radicals of (1) R₃₀; (2) halo radicals; or (3) -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals; more preferably, R₁₁ is an aryl radical optionally substituted by 1-2 radicals of amino, dimethylamino, acetamido, hydroxy, halo, cyano, methoxy, methylthio, methylsulfinyl, methylsulfonyl, aminocarbonyl, methyl or trifluoromethyl radicals; more preferably, R₁₁ is an unsubstituted phenyl or naphthyl radical or a phenyl radical substituted by 1-2 radicals of amino, dimethylamino, acetamido, hydroxy, halo, cyano, methoxy, methylthio, methylsulfinyl, methylsulfonyl, aminocarbonyl, methyl or trifluoromethyl radicals; and most preferably, R₁₁ is an unsubstituted phenyl radical or a phenyl radical substituted by 1-2 radicals of amino, dimethylamino, acetamido, hydroxy, halo, cyano, methoxy, methylthio, methylsulfonyl, methyl or trifluoromethyl radicals;
more preferably, R₁₂ is a heteroaryl radical optionally substituted by 1-2 radicals of (1) R₃₀; (2) halo radicals; or (3) -C(O)-NR₃₁R₃₂, -OR₂₉, -SR₂₉, -NR₃₁R₃₂ or -NR₃₃-C(O)-R₂₉ radicals; more preferably, R₁₂ is a heteroaryl radical optionally substituted by 1-2 radicals of amino, dimethylamino, acetamido, hydroxy, halo, cyano, methoxy, methyl or trifluoromethyl radicals; more preferably, R₁₂ is a 4-pyridyl, 4-quinolinyl, 4-imidazolyl or 4-pyrimidinyl radical optionally substituted by a radical of amino, dimethylamino, acetamido, hydroxy, halo, cyano, methoxy, methyl or trifluoromethyl radicals; and most preferably, R₁₂ is a 4-pyridyl radical optionally substituted by a radical of amino, dimethylamino, acetamido, hydroxy, halo, cyano, methoxy, methyl or trifluoromethyl radicals;
wherein each R₃₀ is independently
(1) alkyl, alkenyl or alkynyl radicals optionally substituted by 1-3 radicals of -NR₃₁R₃₁, hydroxy, alkoxy, alkylthio, alkylsulfinyl, alkylsulfonyl, cyano, halo or aralkoxy, aralkylthio, aralkylsulfonyl, heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
preferably, each R₃₀ is independently
(1) C₁-C₄ alkyl, C₂-C₄ alkenyl or C₂-C₄ alkynyl radicals optionally substituted by 1-3 radicals of -NR₃₁R₃₁, -CO₂R₂₃, hydroxy, C₁-C₄ alkoxy, C₁-C₄ alkylthio, C₁-C₄ alkylsulfinyl, C₁-C₄ alkylsulfonyl, cyano, halo or aryl-C₁-C₄-alkoxy, aryl-C₁-C₄-alkylthio, aryl-C₁-C₄-alkylsulfonyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
more preferably, each R₃₀ is independently
(1) C₁-C₄ alkyl radical optionally substituted by 1-3 radicals of
   (a) -NR₃₁R₃₁;
   (b) C₁-C₄ alkoxy-carbonyl or phenoxycarbonyl or phenylmethoxycarbonyl optionally substituted by 1-3 radicals of amino, alkylamino, di-(C₁-C₄-alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or trifluoromethyl; or
   (c) hydroxy, C₁-C₄ alkoxy, C₁-C₄ alkylthio, or phenyl-C₁-C₄-alkoxy, phenyl-C₁-C₄-alkylthio, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₄ haloalkyl of 1-3 halo radicals;
(2) C₁-C₄ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
more preferably, each R₃₀ is independently
(1) C₁-C₄ alkyl radical optionally substituted by
   (a) amino, C₁-C₄ alkylamino or di-(C₁-C₄-alkyl)amino radicals; or
   (b) hydroxy, C₁-C₄ alkoxy, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
(2) C₁-C₂ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
more preferably, each R₃₀ is independently
(1) C₁-C₄ alkyl radical optionally substituted by a phenyl or heteroaryl radical optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
(2) trifluoromethyl radical; or
(3) aryl or heteroaryl radicals optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
more preferably, each R₃₀ is independently
(1) C₁-C₄ alkyl radical optionally substituted by a phenyl or heteroaryl radical optionally substituted by 1-3 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
most preferably, R₃₀ is independently
(1) C₁-C₄ alkyl radical optionally substituted by a phenyl or heteroaryl radical optionally substituted by 1-2 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀; and most preferably, R₂₉ is an aryl or heteroaryl radicals optionally substituted by 1-2 radicals of amino, dimethylamino, acetamido, hydroxy, halo, methoxy, methyl or trifluoromethyl radicals;
each R₃₁ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₁ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₁ is independently
(1) hydrogen radicals; or
(2) C₁-C₄ alkyl radical optionally substituted by an phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyl or trifluoromethyl radicals;
more preferably, each R₃₁ is independently hydrogen or C₁-C₄ alkyl radicals; and most preferably, each R₃₁ is independently hydrogen, methyl or ethyl radicals;
each R₃₂ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₂ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₂ is independently
(1) hydrogen radicals;
(2) C₁-C₄ alkyl radical optionally substituted by an C₃-C₆ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₆ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₂ is independently
(1) hydrogen radicals;
(2) C₁-C₄ alkyl radical optionally substituted by phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals; or
(3) phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals;
more preferably, each R₃₂ is independently
(1) hydrogen radicals;
(2) C₁-C₄ alkyl radical or C₁-C₂ alkyl radical substituted by phenyl or heteroaryl radical optionally substituted by 1-3 radicals of amino, dimethylamino, acetamido, hydroxy, methoxy, methyl or trifluoromethyl radicals; or
(3) phenyl or heteroaryl radical optionally substituted by 1-3 radicals of amino, dimethylamino, acetamido, hydroxy, methoxy, methyl or trifluoromethyl radicals;
most preferably, R₃₂ is independently
(1) hydrogen or C₁-C₄ alkyl radical; or
(2) phenyl or heteroaryl radical optionally substituted by 1-2 radicals of amino, dimethylamino, acetamido, hydroxy, methoxy, methyl or trifluoromethyl radicals; and
wherein each R₃₃ is independently
(1) hydrogen radical; or
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
preferably, each R₃₃ is independently
(1) hydrogen radical; or
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
more preferably, each R₃₃ is independently hydrogen or C₁-C₄ alkyl radical; and most preferably, each R₃₃ is independently hydrogen or methyl radical.
L and L' are "leaving groups" a each independently a halo, C₁-C₄ alkylsulfate, arylsulfate, trifluoromethylsulfate, C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halo radicals, arylcarbonyloxy, N-oxosuccinate, N-oxobenzotriazole or cyanide radical;
preferably, L is a C₁-C₄ alkoxy, aryloxy, C₁-C₄ alkylcarbonyloxy optionally substituted by 1-3 halo radicals, arylcarbonyloxy, N-oxosuccinate, N-oxobenzotriazole or cyanide radical; more preferably L is a C₁-C₄ alkoxy, aryloxy, N-oxosuccinate or N-oxobenzotriazole radical; most preferably L is C₁-C₄ alkoxy radical;
preferably, L' is a halo, C₁-C₄ alkylsulfate, arylsulfate or trifluoromethylsulfate radical; more preferably L' is a halo, trifluoromethylsulfate or mesylate radical; most preferably L' is iodide;

The compounds of this invention may have in general several asymmetric centers and are typically depicted in the form of racemic mixtures. This invention is intended to encompass racemic mixtures, partially racemic mixtures and separate enantiomers and diasteromers.

Compounds of interest include the following: 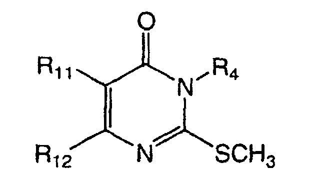 wherein R₄ is methyl, ethyl, benzyl, phenyl or 4-pyridyl and R₁₁ and R₁₂ are one of the combinations given in the following table:

| R¹¹ | R¹² |
|---|---|
| Phenyl | 4-methylsulfonylphenyl |
| 4-fluorophenyl | 4-aminosulfonylphenyl |
| 3-fluorophenyl | 4-(CF₃C(O)NHSO₂)phenyl |
| 2-fluorophenyl | 4-(CH₃NHSO₂)phenyl |
| 4-chlorophenyl | 4-chlorophenyl |
| 3-chlorophenyl | 3-chlorophenyl |
| 2-chlorophenyl | 2-chlorophenyl |
| 4-tolyl | 4-tolyl |
| 3-tolyl | 3-tolyl |
| 2-tolyl | 2-tolyl |
| 4-trifluoromethylphenyl | 4-trifluoromethylphenyl |
| 3-trifluoromethylphenyl | 3-trifluoromethylphenyl |
| 2,6-dichlorophenyl | 2,6-dichlorophenyl |
| 2,6-dimethylphenyl | 2,6-dimethylphenyl |
| 4-methylsulfonylphenyl | 3,4-dichlorophenyl |
| 4-aminosulfonylphenyl | 3,4-dimethylphenyl |
| 4-(CF₃C(O)NHSO₂)phenyl | 2,4-dichlorophenyl |
| 4-(CH₃NHSO₂)phenyl | 2,4-dimethylphenyl |
| 4-methylsulfonylphenyl | 4-pyridyl |
| 4-aminosulfonylphenyl | 4-pyrimidinyl |
| 4-(CF₃C(O)NHSO₂)phenyl | 2-thienyl |
| 4-(CH₃NHSO₂)phenyl | 3-benzofuryl |
| 4-pyridyl | 4-methylsulfonylphenyl |
| 4-pyrimidinyl | 4-aminosulfonylphenyl |
| 2-furyl | 4-(CF₃C(O)NHSO₂)phenyl |
| 3-benzothienyl | 4-(CH₃NHSO₂)phenyl |
| Phenyl | 4-pyridyl |
| 4-fluorophenyl | 4-pyridyl |
| 4-pyridyl | 3-fluorophenyl |
| 2-fluorophenyl | 4-pyridyl |
| 4-pyridyl | 4-chlorophenyl |
| 3 -chlorophenyl | 4-pyridyl |
| 2-chlorophenyl | 4-pyridyl |
| 4-tolyl | 4-pyridyl |
| 3-tolyl | 4-pyridyl |
| 2-tolyl | 4-pyridyl |
| 4-trifluoromethylphenyl | 4-pyridyl |
| 3-trifluoromethylphenyl | 4-pyridyl |
| 2,6-dichlorophenyl | 4-pyridyl |
| 2,6-dimethylphenyl | 4-pyridyl |
| 3,4-dichlorophenyl | 4-pyridyl |
| 4-pyridyl | 3,4-dimethylphenyl |
| 4-pyridyl | 2,4-dichlorophenyl |
| 4-pyridyl | 2,4-dimethylphenyl |
| Phenyl | 2-nitro-4-pyridyl |
| 2-nitro-4-pyridyl | 4-fluorophenyl |
| 3-fluorophenyl | 2-nitro-4-pyridyl |
| 2-amino-4-pyridyl | 2-fluorophenyl |
| 4-chlorophenyl | 2-nitro-4-pyridyl |
| 3-chlorophenyl | 2-nitro-4-pyridyl |
| 2-chlorophenyl | 2-nitro-4-pyridyl |
| 4-tolyl | 2-amino-4-pyridyl |
| 2-nitro-4-pyridyl | 3-tolyl |
| 2-tolyl | 2-nitro-4-pyridyl |
| 4-trifluoromethylphenyl | 2-nitro-4-pyridyl |
| 3-trifluoromethylphenyl | 2-amino-4-pyridyl |
| 2,6-dichlorophenyl | 2-amino-4-pyridyl |
| 4-quinolyl | 2,6-dimethyl phenyl |
| 4-quinolyl | 3,4-dichlorophenyl |
| 4-quinolyl | 3,4-dimethylphenyl |
| 2,4-dichlorophenyl | 4-quinolyl |
| 2,4-dimethylphenyl | 4-quinolyl |
| Phenyl | 2-acetamido-4-pyridyl |
| 4-fluorophenyl | 2-acetamido-4-pyridyl |
| 3-fluorophenyl | 2-acetamido-4-pyridyl |
| 2-acetamido-4-pyridyl | 2-fluorophenyl |
| 2-acetamido-4-pyridyl | 4-chlorophenyl |
| 2-acetamido-4-pyridyl | 3-chlorophenyl |
| 2-chlorophenyl | 2-acetamido-4-pyridyl |
| 4-tolyl | 2-acetamido-4-pyridyl |
| 2-acetamido-4-pyridyl | 3-tolyl |
| 2-tolyl | 2-acetamido-4-pyridyl |
| 4-trifluoromethylphenyl | 2-acetamido-4-pyridyl |
| 3-trifluoromethylphenyl | 2-acetamido-4-pyridyl |
| 6-isoquinolinyl | 2,6-dichlorophenyl |
| 6-isoquinolinyl | 2,6-dimethyl phenyl |
| 6-isoquinolinyl | 3,4-dichlorophenyl |
| 6-isoquinolinyl | 3,4-dimethyl phenyl |
| 2,4-dichlorophenyl | 6-isoquinolinyl |
| 2,4-dimethyl phenyl | 6-isoquinolinyl |
| Phenyl | 2-nitro-4-pyrimidinyl |
| 4-fluorophenyl | 4-pyrimidinyl |
| 3-fluorophenyl | 2-amino-4-pyrimidinyl |
| 2-nitro-4-pyrimidinyl | 2-fluorophenyl |
| 4-pyrimidinyl | 4-chlorophenyl |
| 3-chlorophenyl | 2-amino-4-pyrimidinyl |
| 2-chlorophenyl | 4-pyrimidinyl |
| 4-tolyl | 2-amino-4-pyrimidinyl |
| 3-tolyl | 4-pyrimidinyl |
| 2-nitro-4-pyrimidinyl | 2-tolyl |
| 4-pyrimidinyl | 4-trifluoromethylphenyl |
| 3-trifluoromethylphenyl | 2-amino-4-pyrimidinyl |
| 2,6-dichlorophenyl | 4-pyrimidinyl |
| 2,6-dimethyl phenyl | 4-pyrimidinyl |
| 2-nitro-4-pyrimidinyl | 3,4-dichlorophenyl |
| 4-pyrimidinyl | 3,4-dimethyl phenyl |
| 2,4-dichlorophenyl | 2-nitro-4-pyrimidinyl |
| 2,4-dimethyl phenyl | 2-nitro-4-pyrimidinyl |

### Additional preferred compounds are listed in the Examples, infra.

As utilized herein, the following terms shall have the following meanings:
"Alkyl", alone or in combination, means a straight-chain or branched-chain alkyl radical containing preferably 1-15 carbon atoms (C₁-C₁₅), more preferably 1-8 carbon atoms (C₁-C₈), even more preferably 1-6 carbon atoms (C₁-C₆), yet more preferably 1-4 carbon atoms (C₁-C₄), still more preferably 1-3 carbon atoms (C₁-C₃), and most preferably 1-2 carbon atoms (C₁-C₂). Examples of such radicals include methyl, ethyl, n-propyl, isopropyl, n-butyl, isobutyl, sec-butyl, tert-butyl, pentyl, isoamyl, hexyl, octyl and the like.
"Hydroxyalkyl", alone or in combination, means an alkyl radical as defined above wherein at least one hydrogen radical is replaced with a hydroxyl radical, preferably 1-3 hydrogen radicals are replaced by hydroxyl radicals, more preferably 1-2 hydrogen radicals are replaced by hydroxyl radicals, and most preferably one hydrogen radical is replaced by a hydroxyl radical. Examples of such radicals include hydroxymethyl, 1-, 2-hydroxyethyl, 1-, 2-, 3-hydroxypropyl, 1,3-dihydroxy-2-propyl, 1,3-dihydroxybutyl, 1,2,3,4,5,6-hexahydroxy-2-hexyl and the like.
"Alkenyl", alone or in combination, means a straight-chain or branched-chain hydrocarbon radical having one or more double bonds, preferably 1-2 double bonds and more preferably one double bond, and containing preferably 2-15 carbon atoms (C₂-C₁₅), more preferably 2-8 carbon atoms (C₂-C₈), even more preferably 2-6 carbon atoms (C₂-C₆), yet more preferably 2-4 carbon atoms (C₂-C₄), and still more preferably 2-3 carbon atoms (C₂-C₃). Examples of such alkenyl radicals include ethenyl, propenyl, 2-methylpropenyl, 1,4-butadienyl and the like.
"Alkoxy", alone or in combination, means a radical of the type "R-O-" wherein "R" is an alkyl radical as defined above and "O" is an oxygen atom. Examples of such alkoxy radicals include methoxy, ethoxy, n-propoxy, isopropoxy, n-butoxy, iso-butoxy, sec-butoxy, tert-butoxy and the like.
"Alkoxycarbonyl", alone or in combination, means a radical of the type "R-O-C(O)-" wherein "R-O-" is an alkoxy radical as defined above and "C(O)" is a carbonyl radical.
"Alkoxycarbonylamino", alone or in combination, means a radical of the type "R-O-C(O)-NH-" wherein "R-O-C(O)" is an alkoxycarbonyl radical as defined above, wherein the amino radical may optionally be substituted, such as with alkyl, aryl, aralkyl, cycloalkyl, cycloalkylalkyl and the like.
"Alkylthio", alone or in combination, means a radical of the type "R-S-" wherein "R" is an alkyl radical as defined above and "S" is a sulfur atom. Examples of such alkylthio radicals include methylthio, ethylthio, n-propylthio, isopropylthio, n-butylthio, iso-butylthio, sec-butylthio, tert-butylthio and the like.
"Alkylsulfinyl", alone or in combination, means a radical of the type "R-S(O)-" wherein "R" is an alkyl radical as defined above and "S(O)" is a mono-oxygenated sulfur atom. Examples of such alkylsulfinyl radicals include methylsulfinyl, ethylsulfinyl, n-propylsulfinyl, isopropylsulfinyl, n-butylsulfinyl, iso-butylsulfinyl, sec-butylsulfinyl, test-butylsulfinyl and the like.
"Alkylsulfonyl", alone or in combination, means a radical of the type "R-S(O)₂-" wherein "R" is an alkyl radical as defined above and "S(O)₂" is a di-oxygenated sulfur atom. Examples of such alkylsulfonyl radicals include methylsulfonyl, ethylsulfonyl, n-propylsulfonyl, isopropylsulfonyl, n-butylsulfonyl, iso-butylsulfonyl, sec-butylsulfonyl, tert-butylsulfonyl and the like.
"Aryl", alone or in combination, means a phenyl or biphenyl radical, which is optionally benzo fused or heterocyclo fused and which is optionally substituted with one or more substituents selected from alkyl, alkoxy, halogen, hydroxy, amino, azido, nitro, cyano, haloalkyl, carboxy, alkoxycarbonyl, cycloalkyl, alkanoylamino, amido, amidino, alkoxycarbonylamino, N-alkylamidino, alkylamino, dialkylamino, aminoalkyl, alkylaminoalkyl, dialkylaminoalkyl, N-alkylamido, N,N-dialkylamido, aralkoxycarbonylamino, alkylthio, alkylsulfinyl, alkylsulfonyl, oxo and the like.
   Examples of aryl radicals are phenyl, o-tolyl, 4-methoxyphenyl, 2-(tert-butoxy)phenyl, 3-methyl-4-methoxyphenyl, 2-CF₃-phenyl, 2-fluorophenyl, 2-chlorophenyl, 3-nitrophenyl, 3-aminophenyl, 3-acetamidophenyl, 2-amino-3-(aminomethyl)phenyl, 6-methyl-3-acetamidophenyl, 6-methyl-2-aminophenyl, 6-methyl-2,3-diaminophenyl, 2-amino-3-methylphenyl, 4,6-dimethyl-2-aminophenyl, 4-hydroxyphenyl, 3-methyl-4-hydroxyphenyl, 4-(2-methoxyphenyl)phenyl, 2-amino-1-naphthyl, 2-naphthyl, 3-amino-2-naphthyl, 1-methyl-3-amino-2-naphthyl, 2,3-diamino-l-naphthyl, 4, 8-dimethoxy-2-naphthyl and the like.
"Aralkyl" and "arylalkyl", alone or in combination, means an alkyl radical as defined above in which at least one hydrogen atom, preferably 1-2, is replaced by an aryl radical as defined above, such as benzyl, 1-, 2-phenylethyl, dibenzylmethyl, hydroxyphenylmethyl, methylphenylmethyl, diphenylmethyl, dichlorophenylmethyl, 4-methoxyphenylmethyl and the like.
"Aralkoxy", alone or in combination, means an alkoxy radical as defined above in which at least one hydrogen atom, preferably 1-2, is replaced by an aryl radical as defined above, such as benzyloxy, 1-, 2-phenylethoxy, dibenzylmethoxy, hydroxyphenylmethoxy, methylphenylmethoxy, dichlorophenylmethoxy, 4-methoxyphenylmethoxy and the like.
"Aralkoxycarbonyl", alone or in combination, means a radical of the type "R-O-C(O)-'' wherein "R-O-" is an aralkoxy radical as defined above and "-C(O)-" is a carbonyl radical.
"Alkanoyl", alone or in combination, means a radical of the type "R-C(O)-" wherein "R" is an alkyl radical as defined above and "-C(O)-" is a carbonyl radical. Examples of such alkanoyl radicals include acetyl, trifluoroacetyl, hydroxyacetyl, propionyl, butyryl, valeryl, 4-methylvaleryl, and the like.
"Alkanoylamino", alone or in combination, means a radical of the type "R-C(O)-NH-" wherein "R-C(O)-" is an alkanoyl radical as defined above, wherein the amino radical may optionally be substituted, such as with alkyl, aryl, aralkyl, cycloalkyl, cycloalkylalkyl and the like.
"Aminocarbonyl", alone or in combination, means an amino substituted carbonyl (carbamoyl) radical, wherein the amino radical may optionally be mono- or di-substituted, such as with alkyl, aryl, aralkyl, cycloalkyl, cycloalkylalkyl, alkanoyl, alkoxycarbonyl, aralkoxycarbonyl and the like.
"Aminosulfonyl", alone or in combination, means an amino substituted sulfonyl radical.
"Benzo", alone or in combination, means the divalent radical C₆H₄= derived from benzene. "Benzo fused" forms a ring system in which benzene and a cycloalkyl or aryl group have two carbons in common, for example tetrahydronaphthylene and the like.
"Bicyclic" as used herein is intended to include both fused ring systems, such as naphthyl and β-carbolinyl, and substituted ring systems, such as biphenyl, phenylpyridyl and diphenylpiperazinyl.
"Cycloalkyl", alone or in combination, means a saturated or partially saturated, preferably one double bond, monocyclic, bicyclic or tricyclic carbocyclic alkyl radical, preferably monocyclic, containing preferably 5-12 carbon atoms (C₅-C₁₂), more preferably 5-10 carbon atoms (C₅-C₁₀), even more preferably 5-7 carbon atoms (C₅-C₇), which is optionally benzo fused or heterocyclo fused and which is optionally substituted as defined herein with respect to the definition of aryl. Examples of such cycloalkyl radicals include cyclopentyl, cyclohexyl, dihydroxycyclohexyl, ethylenedioxycyclohexyl, cycloheptyl, octahydronaphthyl, tetrahydronaphthyl, octahydroquinolinyl, dimethoxytetrahydronaphthyl, 2,3-dihydro-lH-indenyl, azabicyclo[3.2.1]octyl and the like.
"Heteroatoms" means nitrogen, oxygen and sulfur heteroatoms.
"Heterocyclo fused" forms a ring system in which a heterocyclyl or heteroaryl group of 5-6 ring members and a cycloalkyl or aryl group have two carbons in common, for example indole, isoquinoline, tetrahydroquinoline, methylenedioxybenzene and the like.
"Heterocyclyl" means a saturated or partially unsaturated, preferably one double bond, monocyclic or bicyclic, preferably monocyclic, heterocycle radical containing at least one, preferably 1 to 4, more preferably 1 to 3, even more preferably 1-2, nitrogen, oxygen or sulfur atom ring member and having preferably 3-8 ring members in each ring, more preferably 5-8 ring members in each ring and even more preferably 5-6 ring members in each ring. "Heterocyclyl" is intended to include sulfone and sulfoxide derivatives of sulfur ring members and N-oxides of tertiary nitrogen ring members, and carbocyclic fused, preferably 3-6 ring carbon atoms and more preferably 5-6 ring carbon atoms, and benzo fused ring systems. "Heterocyclyl" radicals may optionally be substituted on at least one, preferably 1-4, more preferably 1-3, even more preferably 1-2, carbon atoms by halogen, alkyl, alkoxy, hydroxy, oxo, thioxo, aryl, aralkyl, heteroaryl, heteroaralkyl, amidino, N-alkylamidino, alkoxycarbonylamino, alkylsulfonylamino and the like, and/or on a secondary nitrogen atom by hydroxy, alkyl, aralkoxycarbonyl, alkanoyl, alkoxycarbonyl, heteroaralkyl, aryl or aralkyl radicals. More preferably, "heterocyclyl", alone or in combination, is a radical of a monocyclic or bicyclic saturated heterocyclic ring system having 5-8 ring members per ring, wherein 1-3 ring members are oxygen, sulfur or nitrogen heteroatoms, which is optionally partially unsaturated or benzo-fused and optionally substituted by 1-2 oxo or thioxo radicals. Examples of such heterocyclyl radicals include pyrrolidinyl, piperidinyl, piperazinyl, morpholinyl, thiamorpholinyl, 4-benzyl-piperazin-l-yl,. pyrimidinyl, tetrahydrofuryl, pyrazolidonyl, pyrazolinyl, pyridazinonyl, pyrrolidonyl, tetrahydrothienyl and its sulfoxide and sulfone derivatives, 2,3-dihydroindolyl, tetrahydroquinolinyl, 1,2,3,4-tetrahydroisoquinolinyl, 1,2,3,4-tetrahydro-1-oxo-isoquinolinyl, 2,3-dihydrobenzofuryl, benzopyranyl, methylenedioxyphenyl, ethylenedioxyphenyl and the like.
"Heteroaryl" means a monocyclic or bicyclic, preferably monocyclic, aromatic heterocycle radical, having at least one, preferably 1 to 4, more preferably 1 to 3, even more preferably 1-2, nitrogen, oxygen or sulfur atom ring members and having preferably 5-6 ring members in each ring, which is optionally saturated carbocyclic fused, preferably 3-4 carbon atoms (C₃-C₄) to form 5-6 ring membered rings and which is optionally substituted as defined above with respect to the definitions of aryl. Examples of such heteroaryl groups include imidazolyl, 1-benzyloxycarbonylimidazol-4-yl, pyrrolyl, pyrazolyl, pyridyl, 3-(2-methyl)pyridyl, 3-(4-trifluoromethyl)pyridyl, pyrimidinyl, 5-(4-trifluoromethyl)pyrimidinyl, pyrazinyl, triazolyl, furyl, thienyl, oxazolyl, thiazolyl, indolyl, quinolinyl, 5,6,7,8-tetrahydroquinolyl, 5,6,7,8-tetrahydroisoquinolinyl, quinoxalinyl, benzothiazolyl, benzofuryl, benzimidazolyl, benzoxazolyl and the like.
"Heteroaralkyl" and "heteroarylalkyl," alone or in combination, means an alkyl radical as defined above in which at least one hydrogen atom, preferably 1-2, is replaced by a heteroaryl radical as defined above, such as 3-furylpropyl, 2-pyrrolyl propyl, chloroquinolinylmethyl, 2-thienylethyl, pyridylmethyl, 1-imidazolylethyl and the like.
"Halogen" and "halo", alone or in combination, means fluoro, chloro, bromo or iodo radicals.
"Haloalkyl", alone or in combination, means an alkyl radical as defined above in which at least one hydrogen atom, preferably 1-3, is replaced by a halogen radical, more preferably fluoro or chloro radicals. Examples of such haloalkyl radicals include 1,1,1-trifluoroethyl, chloromethyl, 1-bromoethyl, fluoromethyl, difluoromethyl, trifluoromethyl, bis(trifluoromethyl)methyl and the like.
Cation shall mean a positively charged element or molecule capable of pairing with a negative charge residing on the sulfur atom, or delocalized within the ring system, of a negatively charged compound of formula (I). Examples of such cations include, but are not limited to, alkaline, alkaline earth, ammonium, and quaternaryl ammonium cations and the like. Specific examples include: Li⁺, Na⁺, K⁺, Mg⁺², Zn⁺², Cu⁺², or tetra C₁-C₄ alkylammonium cations, preferably, Li⁺, Na⁺, or K⁺.
"Cytokine" means a secreted protein that affects the functions of other cells, particularly as it relates to the modulation of interactions between cells of the immune system or cells involved in the inflammatory response. Examples of cytokines include but are not limited to interleukin 1 (IL-1), preferably IL-1β, interleukin 6 (IL-6), interleukin 8 (IL-8) and TNF, preferably TNF-α (tumor necrosis factor-α).
"TNF, IL-1, IL-6, and/or IL-8 mediated disease or disease state" means all disease states wherein TNF, IL-1, IL-6, and/or IL-8 plays a role, either directly as TNF, IL-1, IL-6, and/or IL-8 itself, or by TNF, IL-1, IL-6, and/or IL-8 inducing another cytokine to be released. For example, a disease state in which IL-1' plays a major role, but in which the production of or action of IL-1 is a result of TNF, would be considered mediated by TNF.
"Leaving group" generally refers to groups readily displaceable by a nucleophile, such as an amine, a thiol or an alcohol nucleophile. Such leaving groups are well known in the art. Examples of such leaving groups include, but are not limited to, N-hydroxysuccinimide, N-hydroxybenzotriazole, halides, triflates, tosylates and the like. Preferred leaving groups are indicated herein where appropriate.
"Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. Preferred protecting groups are indicated herein where appropriate. Examples of amino protecting groups include, but are not limited to, aralkyl, substituted aralkyl, cycloalkenylalkyl and substituted cycloalkenyl alkyl, allyl, substituted allyl, acyl, alkoxycarbonyl, aralkoxycarbonyl, silyl and the like. Examples of aralkyl include, but are not limited to, benzyl, ortho-methylbenzyl, trityl and benzhydryl, which can be optionally substituted with halogen, alkyl, alkoxy, hydroxy, nitro, acylamino, acyl and the like, and salts, such as phosphonium and ammonium salts. Examples of aryl groups include phenyl, naphthyl, indanyl, anthracenyl, 9-(9-phenylfluorenyl), phenanthrenyl, durenyl and the like. Examples of cycloalkenylalkyl or substituted cycloalkylenylalkyl radicals, preferably have 6-10 carbon atoms, include, but are not limited to, cyclohexenyl methyl and the like. Suitable acyl, alkoxycarbonyl and aralkoxycarbonyl groups include benzyloxycarbonyl, t-butoxycarbonyl, iso-butoxycarbonyl, benzoyl, substituted benzoyl, butyryl, acetyl, tri-fluoroacetyl, tri-chloro acetyl, phthaloyl and the like. A mixture of protecting groups can be used to protect the same amino group, such as a primary amino group can be protected by both an aralkyl group and an aralkoxycarbonyl group. Amino protecting groups can also form a heterocyclic ring with the nitrogen to which they are attached, for example, 1,2-bis(methylene)benzene, phthalimidyl, succinimidyl, maleimidyl and the like and where these heterocyclic groups can further include adjoining aryl and cycloalkyl rings. In addition, the heterocyclic groups can be mono-, di- or tri-substituted, such as nitrophthalimidyl. Amino groups may also be protected against undesired reactions, such as oxidation, through the formation of an addition salt, such as hydrochloride, toluenesulfonic acid, trifluoroacetic acid and the like. Many of the amino protecting groups are also suitable for protecting carboxy, hydroxy and mercapto groups. For example, aralkyl groups. Alkyl groups are also sutiable groups for protecting hydroxy and mercapto groups, such as tert-butyl.

Silyl protecting groups are silicon atoms optionally substituted by one or more alkyl, aryl and aralkyl groups. Suitable silyl protecting groups include, but are not limited to, trimethylsilyl, triethylsilyl, tri-isopropylsilyl, tertbutyldimethylsilyl, dimethylphenylsilyl, 1,2-bis(dimethylsilyl)benzene, 1,2-bis(dimethylsilyl) ethane and diphenylmethylsilyl. Silylation of an amino groups provide mono- or di-silylamino groups. Silylation of aminoalcohol compounds can lead to a N,N,O-tri-silyl derivative. Removal of the silyl function from a silyl ether function is readily accomplished by treatment with, for example, a metal hydroxide or ammonium flouride reagent, either as a discrete reaction step or in situ during a reaction with the alcohol group. Suitable silylating agents are, for example, trimethylsilyl chloride, tert-buty-dimethylsilyl chloride, phenyldimethylsilyl chloride, diphenylmethyl silyl chloride or their combination products with imidazole or DMF. Methods for silylation of amines and removal of silyl protecting groups are well known to those skilled in the art. Methods of preparation of these amine derivatives from corresponding amino acids, amino acid amides or amino acid esters are also well known to those skilled in the art of organic chemistry including amino acid/amino acid ester or aminoalcohol chemistry.

Protecting groups are removed under conditions which will not affect the remaining portion of the molecule. These methods are well known in the art and include acid hydrolysis, hydrogenolysis and the like. A preferred method involves removal of a protecting group, such as removal of a benzyloxycarbonyl group by hydrogenolysis utilizing palladium on carbon in a suitable solvent system such as an alcohol, acetic acid, and the like or mixtures thereof. A t-butoxycarbonyl protecting group can be removed utilizing an inorganic or organic acid, such as HCl or trifluoroacetic acid, in a suitable solvent system, such as dioxane or methylene chloride. The resulting amino salt can readily be neutralized to yield the free amine. Carboxy protecting group, such as methyl, ethyl, benzyl, tert-butyl, 4-methoxyphenylmethyl and the like, can be removed under hydroylsis and hydrogenolysis conditions well known to those skilled in the art.

The symbols used above have the following meanings:

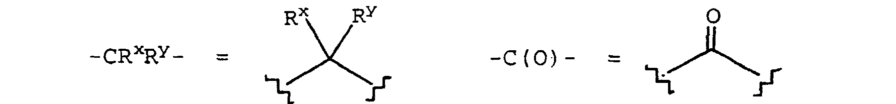

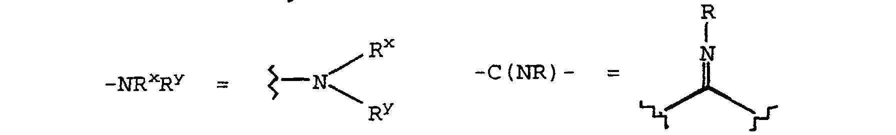

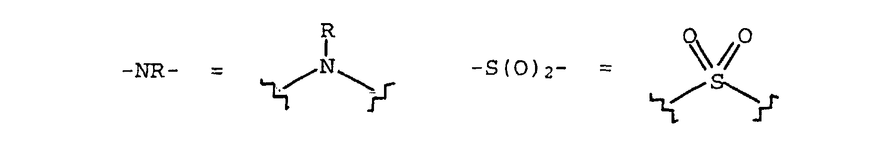

### Process of the Invention:

The synthesis of pyrimidinones of formula I within the instant invention is performed in one reaction vessel in high overall yield as shown in Scheme 1. Moreover the synthetic methodology allows for the incorporation of an R⁴ substituent with high regioselectivity. 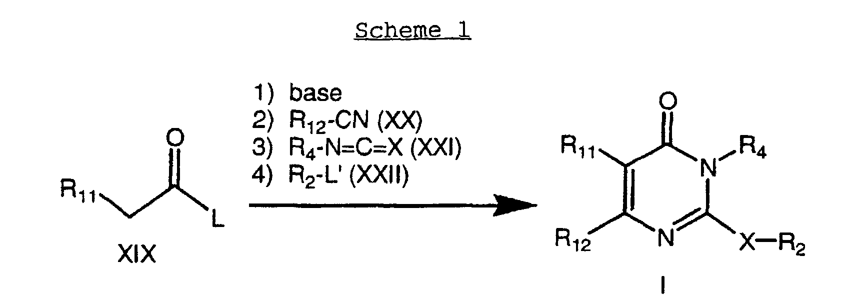 As shown in Scheme 1, an acetic acid derivative XIX, preferably a substituted acetic acid ester (L = alkoxy), is treated with an appropriate base, such as potassium tert-butoxide. The reaction is performed in an appropriate solvent containing a proton source, such as tert-butanol, and a co-solvent as needed. Such cosolvents may be DMSO, tetra-alkylureas, DMF, DMA, tert-butylmethyl ether, and the like. Nitrile XX (R₁₂-CN) is then added to the reaction mixture and the mixture is stirred for an appropriate amount of time, preferably from about 30 min to about 24 h. Slow addition of isocyanate (X=O) or isothiocyanate (X=S) XXI (R₄-N=C=X), at a reaction temperature of preferably from about -20°C to about 25°C provides the cyclized product II where R₂ is an appropriate cation, such as K⁺. The anionic intermediate may be treated with acid to obtain compounds of formula I where R₂ is H. The anionic intermediate may be treated with an appropriate electrophile, R₂-L', where L' is an appropriate leaving group, such as halogen, mesylate, tosylate and the like, to obtain compounds of formula I where R₂ is other than H as defined above.

The starting materials, R₂-L', R¹¹-CH₂-C(O)-L and R¹²-CN, are commerically available or readily prepared from commercially available starting materials using processes and reagents well known in the art. N-substituted isocyanates or N-substituted isothiocynates (R₄-N=C=X) are commercially available or are readily prepared by treatment of R₄-L, where L is an appropriate leaving group (such as halogen, mesylate, tosylate and the like), with sodium or potassium cyanate or thiocyanate; from the corresponding amino (R₄-NH2) compounds, for example, by reaction with phosgene, thiophosgene, carbon disulfide and the like in an appropriate solvent; from the corresponding acyl azide (R₄-C(O)-N₃) compounds (Curtius Rearrangement) in an appropriate solvent; and the like.

The following Examples are presented for illustrative purposes only and are not intended, nor should they be construed, as limiting the invention in any manner. Those skilled in the art will appreciate that modifications and variations of the compounds disclosed herein can be made without violating the spirit or scope of the present invention.

### EXAMPLES

### Example 1

### Procedure for the preparation of 3-methyl-2-methylthio-5- (3-methylphenyl)-6- (4-pyridyl)-4(3H)-pyrimidinone

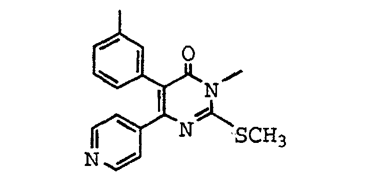

A solution of potassium t-butoxide (1M in t-butanol, 11, 1 mol) was added dropwise to a stirred solution of ethyl 3-methylphenyl acetate (178 g, 1 mol) in *N,N*-dimethylformamide (2 1). A solution of 4-cyanopyridine (104.11 g, 1 mol) in *N,N*-dimethylformamide (1 1) was pumped into the reaction mixture over a period of about 4.5 h. The mixture was then stirred at room temperature for 3 h, before the dropwise addition of a solution of methyl isothiocyanate (68.4 ml, 1 mol) in *N*,*N*-dimethylformamide (50 ml) over a period of 10 min. After stirring for 1 h at room temperature, the reaction mixture was cooled to 3°C and methyl iodide (62.3 ml, 1 mol) was added dropwise over a period of 10 min. Stirring was continued at room temperature overnight. The mixture was cooled to 3°C and water (4 1) was pumped into the reaction mixture over a period of 6 h. The precipitate was removed by filtration, washed with water and dried in a vacuum oven to give 3-Methyl-2-methylthio-5-(3-methylphenyl)-6-(4-pyridyl)-4(3H)-pyrimidinone as a solid: MS (*m*/*z*) : 324 (M+H)⁺ ; C₁₈H₁₇N₃OS requir. 323.4.

### Example 2

### Procedure for the preparation of 3-methyl-2-methylthio-5- (3-trifluoromethylphenyl)-6-(4-pyridyl)-4(3H)-pyrimidinone

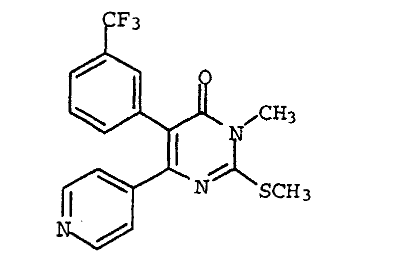

Step A: Ethyl 3-trifluoromethylphenylacetate: To a mixture of 3-trifluoromethylphenylacetic acid (500 gm, 2.45 mol) in absolute ethanol (11) was added conc. sulfuric acid (10 mL) at room temperature. The reation was heated to reflux for 12 hr, cooled to room teperature and the ethanol was removed *in vacuo*. The acid was neutralized by the addition of 500 mL of saturated NaHCO₃ and extracted with ethyl actetate. The combined organic extracts were dried (Mg₂SO₄), filtered and concentrated to give the Step A compound: Silica gel TLC eluted with 20% ethyl actetate/hexanes; Rf = 0.65.
Step B: 3-methyl-2-methylthio-5-(3-trifluoromethylphenyl)-6-(4-pyridyl)-4(3H)-pyrimidinone; A solution of potassium t-butoxide (1M in t-butanol, 323 mL, 323 mmol) was added dropwise to a heated (40 °C), stirred solution of ethyl 3-trifluoromethylphenyl acetate (75 g, 323 mol) in *N*,*N*-dimethylformamide (431 mL). A solution of 4-cyanopyridine (33.6 g, 323 mmol) in *N,N*-dimethylformamide (431 mL) was pumped into the reaction at a rate of 1.4 mL/min. After complete addition, the mixture was stirred for 30 min, and cooled to room temperature before the dropwise addition of a solution of methyl isothiocyanate (22.1 mL, 323 mmol) over a period of 50 min. After stirring for 50 min at room temperature, the reaction mixture was cooled to 3°C and methyl iodide (20.1 mL, 323 mol) was added dropwise over a period of 10 min. Stirring was continued at room temperature overnight. The solvents were removed in vacuo, and the residue was pre-absorbed on silica gel, loaded on a column, and the product was isolated by chromatography (SiO₂, gradient of 10% to 40% acetone/hexane) to give 3-methyl-2-methylthio-5-(3-trifluoromethylphenyl)-6-(4-pyridyl)-4(3H)-pyrimidinone: silica gel TLC eluted with 10% methanol/methylene chloride, Rf = 0.6.

Although the above examples illustrate syntheses in which R¹¹ is a substituted phenyl and R¹² is 4-pyridyl, this approach may be equally applied to any other aryl or heteroaryl ring within the definitions of R¹¹ and R¹² by the appropriate choice of the starting material. Some of the sustituents of R¹¹ and R¹² may require appropriate protection prior to reaction, such as hydroxy, thiol, carboxy, amido, amino and the like radicals may be reacted analogously preferably after appropriate protection. For example, a hydroxy, thiol or carboxy radical may be protected with a benzyl radical that can be remove after reaction, or an amine or amide nitrogen may be protected with e.g. a *tert*-butyldimethylsilyloxymethyl group (Benneche et al., *Acta* *Chem. Scand. B* 42 384-389 (1988)), a *tert*butyldimethylsilyl group, a benzyloxymethyl group, a benzyl group or the like (P₁). Removal of the protecting group P₁ of the resulting pyrimidinone with a suitable reagent (*e.g*., tetrabutylammonium fluoride in the case where P₁ is t-butyldimethyl-silyloxymethyl).

The foregoing is merely illustrative of the invention and is not intended to limit the invention to the disclosed compounds. Variations and changes which are obvious to one skilled in the art are intended to be within the scope and nature of the invention which are defined in the appended claims.

From the foregoing description, one skilled in the art can easily ascertain the essential characteristics of this invention, and can make various changes and modifications of the invention to adapt it to various usages and conditions.

## Claims

1. A process of preparing compounds of formula I 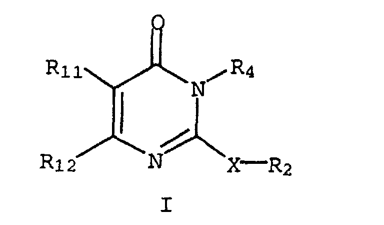 comprising
(a) mixing R₁₁-CH₂-C(O)L and R₁₂-CN in a suitable solvent and in the presence of base; and
(b) adding R₄-N=C=X;
wherein L is a halo, C₁-C₄ alkylsulfate, arylsulfate, trifluoromethylsulfate, C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halogen radicals, arylcarbonyloxy, N-oxosuccinate, N-oxobenzotriazole, or cyanide radical;
X is S or O;
R₂ is a hydrogen radical or Q;
Q is a Li⁺, Na⁺, K⁺, Mg⁺², Zn⁺², Cu⁺², or a tetra C₁-C₄ alkylammonium cation;
R₄ is -Z-Y; provided that the total number of aryl, heteroaryl, cycloalkyl and heterocyclyl radicals in -Z-Y is 0-3;
wherein Z is a
(1) alkyl, alkenyl or alkynyl radical optionally substituted by (a) 1-3 radicals of amino, alkylamino, dialkylamino, alkanoylamino, alkoxycarbonylamino, alkylsulfonylamino, hydroxy, alkoxy, alkylthio or halo, and (b) 1-2 radicals of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, alkyl or haloalkyl; or
(3) aryl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
Y is a
(1) hydrogen radical;
(2) halo or nitro radical;
(3) -C(O)-R₂₀ or -C(NR₅)-NR₅R₂₁ radical;
(4) -OR₂₁, -O-C(O)-R₂₁, -O-C(O)-NR₅R₂₁ or -O-C(O)-NR₂₂-S(O)₂-R₂₀ radical;
(5) -SR₂₁, -S(O)-R₂₀, -S(O)₂-R₂₀, -S(O)₂-NR₅R₂₁, -S(O)₂-NR₂₂-C(O)-R₂₁, -S(O)₂-NR₂₂-C(O)-OR₂₀ or -S(O)₂-NR₂₂-C(O)-NR₅R₂₁ radical; or
(6) -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ or -NR₂₂-S(O)₂-NR₅R₂₁ radical;
wherein each R₅ is independently
(1) hydrogen radicals;
(2) alkyl, alkenyl or alkynyl radicals optionally substituted by 1-3 radicals of amino, alkylamino, dialkylamino, hydroxy, alkoxy, alkylthio, -SO₃H or halo; or
(3) aryl, heteroaryl, aralkyl, heteroaralkyl, heterocyclyl, heterocyclylalkyl, cycloalkyl or cycloalkylalkyl radicals optionally substituted by 1-3 radicals of amino, alkylamino, dialkylamino, hydroxy, alkoxy, alkylthio, alkyl or haloalkyl;
wherein each R₂₀ is independently
(1) alkyl, alkenyl or alkynyl radicals optionally substituted by 1-3 radicals of amino, alkylamino, dialkylamino, alkanoylamino, alkoxycarbonylamino, N-(alkoxycarbonyl)-N-(alkyl)amino, aminocarbonylamino, alkylsulfonylamino, hydroxy, alkoxy, alkylthio, alkylsulfinyl, alkylsulfonyl, halo or aralkoxy, aralkylthio, aralkylsulfonyl, cycloalkyl, heterocyclyl, aryl or heteroaryl radicals optionally substituted by 1-3 radicals of amino, alkylamino, dialkylamino, alkanoylamino, alkoxycarbonylamino, alkylsulfonylamino, alkanoyl, hydroxy, alkoxy, alkylthio, alkylsulfinyl, alkylsulfonyl, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, alkoxycarbonyl, hydroxy, alkoxy, alkylthio, cyano, halo, azido, alkyl or haloalkyl;
each R₂₁ is independently hydrogen radical or R₂₀;
each R₂₂ is independently
(1) hydrogen radical;
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl; or
(3) heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
R₁₁ and R₁₂ are each independently an alkyl, alkenyl, alkynyl, cycloalkyl, heterocyclyl, aryl or heteroaryl radical optionally substituted by 1-3 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ or -NR₃₃-S(O)₂-NR₃₁R₃₂ radicals;
provided that the total number of aryl, heteroaryl, cycloalkyl and heterocyclyl radicals substituted in each of R₁₁ and R₁₂ is 0-1;
wherein each R₃₀ is independently
(1) alkyl, alkenyl or alkynyl radicals optionally substituted by 1-3 radicals of -NR₃₁R₃₁, hydroxy, alkoxy, alkylthio, alkylsulfinyl, alkylsulfonyl, cyano, halo or aralkoxy, aralkylthio, aralkylsulfonyl, heterocyclyl, aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl or haloalkyl;
(2) heterocyclyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl or heteroaryl radicals optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl or haloalkyl;
each R₂₉ is independently hydrogen radical or R₃₀;
each R₃₁ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl;
each R₃₂ is independently
(1) hydrogen radicals;
(2) alkyl radical optionally substituted by an cycloalkyl, aryl, heterocyclyl or heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; or
(3) aryl, heteroaryl, heterocyclyl or cycloalkyl radical optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl; and
wherein each R₃₃ is independently
(1) hydrogen radical; or
(2) alkyl radical optionally substituted by a radical of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, cyano, alkyl or haloalkyl.

2. The process of claim 1 wherein
Z is a
(1) C₁-C₈ alkyl, C₂-C₈ alkenyl or C₂-C₈ alkynyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
each R₅ is independently
(1) hydrogen radicals;
(2) C₁-C₈ alkyl, C₂-C₈ alkenyl or C₂-C₈ alkynyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, -SO₃H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
each R₂₀ is independently
(1) C₁-C₈ alkyl, C₂-C₈ alkenyl or C₂-C₈ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl) amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
each R₂₁ is independently hydrogen radical or R₂₀;
each R₂₂ is independently
(1) hydrogen radical;
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals; or
(3) heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
R₁₁ and R₁₂ are each independently an C₁-C₆ alkyl, C₂-C₆ alkenyl, C₂-C₆ alkynyl, C₃-C₈ cycloalkyl, heterocyclyl, aryl or heteroaryl radical optionally substituted by 1-2 radicals of
(1) R₃₀;
(2) halo radicals;
(3) -C(O)-R₃₀, -C(O)-OR₂₉, -C(O)-NR₃₁R₃₂ or -C(NR₃₁)-NR₃₁R₃₂ radicals;
(4) -OR₂₉, -O-C(O)-R₂₉, -O-C(O)-NR₃₁R₃₂ or -O-C(O)-NR₃₃-S(O)₂-R₃₀ radicals;
(5) -SR₂₉, -S(O)-R₃₀, -S(O)₂-R₃₀, -S(O)₂-NR₃₁R₃₂, -S(O)₂-NR₃₃-C(O)-R₃₀, -S(O)₂-NR₃₃-C(O)-OR₃₀ or -S(O)₂-NR₃₃-C(O)-NR₃₁R₃₂ radicals; or
(6) -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ or -NR₃₃-S(O)₂-NR₃₁R₃₂ radicals;
provided that the total number of aryl, heteroaryl, cycloalkyl and heterocyclyl radicals substituted on each of R₁₁ and R₁₂ is 0-1;
each R₃₀ is independently
(1) C₁-C₄ alkyl, C₂-C₄ alkenyl or C₂-C₄ alkynyl radicals optionally substituted by 1-3 radicals of -NR₃₁R₃₁, hydroxy, C₁-C₄ alkoxy, C₁-C₄ alkylthio, C₁-C₄ alkylsulfinyl, C₁-C₄ alkylsulfonyl, cyano, halo or aryl-C₁-C₄-alkoxy, aryl-C₁-C₄-alkylthio, aryl-C₁-C₄-alkylsulfonyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
each R₂₉ is independently hydrogen radical or R₃₀;
each R₃₁ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals;
each R₃₂ is independently
(1) hydrogen radicals;
(2) C₁-C₄ alkyl radical optionally substituted by an C₃-C₈ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₈ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; and
each R₃₃ is independently
(1) hydrogen radical; or
(2) C₁-C₄ alkyl radical optionally substituted by a radical of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals.

3. The process of claim 2 wherein
Z is a
(1) C₁-C₈ alkyl, C₂-C₈ alkenyl or C₂-C₈ alkynyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl or C₁-C₄ haloalkyl of 1-3 halo radicals;
(2) heterocyclyl radical optionally substituted by 1-2 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₄ haloalkyl of 1-3 halo radicals;
Y is a
(1) hydrogen radical;
(2) halo radical;
(3) -C(O)-R₂₀ or -C(NR₅)-NR₅R₂₁ radical;
(4) -OR₂₁, -O-C(O)-R₂₁ or -O-C(O)-NR₅R₂₁ radical;
(5) -SR₂₁, -S(O)-R₂₀, -S(O)₂-R₂₀ or -S(O)₂-NR₅R₂₁ radical; or
(6) -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ or -NR₂₂-S(O)₂-NR₅R₂₁ radical;
each R₅ is independently
(1) hydrogen radicals;
(2) C₁-C₄ alkyl, C₂-C₅ alkenyl or C₂-C₅ alkynyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, -SO₃H or halo; or
(3) aryl, heteroaryl, aryl-C₁-C₄-alkyl, heteroaryl-C₁-C₄-alkyl, heterocyclyl, heterocyclyl-C₁-C₄-alkyl, C₃-C₈ cycloalkyl or C₃-C₈-cycloalkyl-C₁-C₄-alkyl radicals optionally substituted by 1-3 radicals of amino, C₁-C₄ alkylamino, di-(C₁-C₄-alkyl)amino, hydroxy, C₁-C₄ alkoxy, C₁-C₄ alkylthio, C₁-C₄ alkyl or C₁-C₄ haloalkyl of 1-3 halo radicals;
each R₂₀ is independently
(1) C₁-C₈ alkyl, C₂-C₅ alkenyl or C₂-C₅ alkynyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₈ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl or C₁-C₄ haloalkyl of 1-3 halo radicals;
(2) heterocyclyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl or C₁-C₄ haloalkyl of 1-3 halo radicals; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₄ haloalkyl of 1-3 halo radicals;
each R₂₁ is independently hydrogen radical or R₂₀;
R₁₁ and R₁₂ are each independently an C₁-C₄ alkyl, C₂-C₄ alkenyl, C₂-C₄ alkynyl, C₃-C₆ cycloalkyl, heterocyclyl, aryl or heteroaryl radical optionally substituted by 1-2 radicals of
(1) R30;
(2) halo radicals;
(3) -C(O)-R₃₀, -C(O)-OR₂₉, -C(O)-NR₃₁R₃₂ or -C(NR₃₁)-NR₃₁R₃₂ radicals; or
(4) -OR₂₉, -SR₂₉, -S(O)-R₃₀, -S(O)₂-R₃₀, -S(O)₂-NR₃₁R₃₂, -NR₃₁R₃₂, -NR₃₃-C(O)-R₂₉ or -NR₃₃-C(O)-OR₃₀ radicals;
each R₃₀ is independently
(1) C₁-C₄ alkyl radical optionally substituted by 1-3 radicals of
(a) -NR₃₁R₃₁;
(b) C₁-C₄ alkoxy-carbonyl or phenoxycarbonyl or phenylmethoxycarbonyl optionally substituted by 1-3 radicals of amino, alkylamino, di-(C₁-C₄-alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or trifluoromethyl; or
(c) hydroxy, C₁-C₄ alkoxy, C₁-C₄ alkylthio, or phenyl-C₁-C₄-alkoxy, phenyl-C₁-C₄-alkylthio, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₄ haloalkyl of 1-3 halo radicals;
(2) C₁-C₄ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
each R₂₉ is independently hydrogen radical or R₃₀;
each R₃₁ is independently
(1) hydrogen radicals; or
(2) C₁-C₄ alkyl radical optionally substituted by an phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyl or trifluoromethyl radicals; and
each R₃₂ is independently
(1) hydrogen radicals;
(2) C₁-C₄ alkyl radical optionally substituted by an C₃-C₆ cycloalkyl, aryl, heterocyclyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals; or
(3) aryl, heteroaryl, heterocyclyl or C₃-C₆ cycloalkyl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl or C₁-C₄ haloalkyl of 1-3 halo radicals.

4. The process of claim 3 wherein
R₄ is -Z-Y; provided that the total number of aryl, heteroaryl, cycloalkyl and heterocyclyl radicals in -Z-Y is 0-2;
Z is a
(1) C₁-C₈ alkyl or C₂-C₈ alkenyl radical optionally substituted by (a) 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or halo, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, di-(C₁-C₄ alkyl)amino, (C₁-C₄ alkoxy)carbonylamino, hydroxy, C₁-C₄ alkoxy, C₁-C₄ alkylthio or C₁-C₄ alkyl radicals; or
(3) aryl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl or C₁-C₂ haloalkyl of 1-3 halo radicals;
Y is a
(1) hydrogen radical;
(2) -C(O)-R₂₀ radical;
(3) -OR₂₁, -SR₂₁, -S(O)-R₂₀, -S(O)₂-R₂₀ or -S(O)₂-NR₅R₂₁ radical; or
(4) -NR₅R₂₁, -NR₂₂-C(O)-R₂₁, -NR₂₂-C(O)-OR₂₀, -NR₂₂-C(O)-NR₅R₂₁, -NR₂₂-S(O)₂-R₂₀ or -NR₂₂-S(O)₂-NR₅R₂₁ radical;
each R₅ is independently
(1) hydrogen radicals;
(2) C₁-C₄ alkyl or C₂-C₅ alkenyl radicals optionally substituted by 1-3 radicals of amino, di-(C₁-C₄-alkyl)amino, hydroxy, C₁-C₄ alkoxy, C₁-C₄ alkylthio, -SO₃H or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₄-alkyl)amino, hydroxy, C₁-C₄ alkoxy, C₁-C₄ alkylthio, C₁-C₄ alkyl or C₁-C₂ haloalkyl of 1-3 halo radicals;
each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or C₁-C₂ haloalkyl of 1-3 halo radicals;
each R₂₁ is independently hydrogen radical or R₂₀;
each R₂₂ is independently
(1) hydrogen radical; or
(2) C₁-C₄ alkyl radical optionally substituted by a radical of phenyl or heteroaryl optionally substituted by 1-3 radicals of amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or C₁-C₂ haloalkyl of 1-3 halo radicals;
R₁₁ is an aryl radical and R₁₂ is a heteroaryl radical,
wherein the aryl and heteroaryl radicals are optionally substituted by 1-2 radicals of
(1) R₃₀;
(2) halo radicals; or
(3) -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals;
each R₃₀ is independently
(1) C₁-C₄ alkyl radical optionally substituted by
(a) amino, C₁-C₄ alkylamino or di-(C₁-C₄-alkyl)amino radicals; or
(b) hydroxy, C₁-C₄ alkoxy, heterocyclyl, phenyl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
(2) C₁-C₂ haloalkyl of 1-3 halo radical; or
(3) aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl or trifluoromethyl radicals;
each R₂₉ is independently hydrogen radical or R₃₀;
each R₃₂ is independently
(1) hydrogen radicals;
(2) C₁-C₄ alkyl radical optionally substituted by phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals; or
(3) phenyl or heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl or trifluoromethyl radicals; and
each R₃₃ is independently hydrogen or C₁-C₄ alkyl radical.

5. The process of claim 4 wherein
L is C₁-C₄ alkoxy or aryloxy, optionally substituted by 1-3 radicals of halogen;
X is S;
R₂ is a hydrogen radical, Na⁺ or K⁺;
Z is a
(1) C₁-C₄ alkyl or C₂-C₅ alkenyl radical optionally substituted by (a) 1-3 radicals of amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio or halo, and (b) 1-2 radicals of heterocyclyl, aryl or heteroaryl optionally substituted by 1-3 radicals of amino, C₁-C₄ alkylamino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl or trifluoromethyl radicals;
(2) heterocyclyl radical optionally substituted by 1-2 radicals of amino, di-(C₁-C₂ alkyl)amino, (C₁-C₄ alkoxy)carbonylamino, hydroxy, C₁-C₂ alkoxy, C₁-C₂ alkylthio or C₁-C₄ alkyl radicals; or
(3) aryl or heteroaryl radical optionally substituted by 1-3 radicals of amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
Y is a
(1) hydrogen radical;
(2) -C(O)-R₂₀ radical;
(3) -OR₂₁, -SR₂₁, -S(O)-R₂₀, -S(O)₂-R₂₀ or -S(O)₂-NR₅R₂₁ radical; or
(4) -NR₅R₂₁, -NR₂₂-C(O)-R₂₁ or -NR₂₂-S(O)₂-R₂₀ radical;
each R₅ is independently
(1) hydrogen radical;
(2) C₁-C₄ alkyl radical optionally substituted by 1-3 radicals of amino, di-(C₁-C₂-alkyl)amino, hydroxy, C₁-C₂ alkoxy, C₁-C₂ alkylthio or halo; or
(3) phenyl-C₁-C₂-alkyl, heteroaryl-C₁-C₂-alkyl, heterocyclyl-C₁-C₂-alkyl or C₃-C₆-cycloalkyl-C₁-C₂-alkyl radicals optionally substituted by 1-3 radicals of amino, di-(C₁-C₂-alkyl)amino, hydroxy, C₁-C₂ alkoxy, C₁-C₂ alkylthio, methoxy, methylthio, C₁-C₄ alkyl or trifluoromethyl radicals;
each R₂₀ is independently
(1) C₁-C₈ alkyl or C₂-C₅ alkenyl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo or aryl-C₁-C₄-alkoxy, aryl-C₁-C₄-alkylthio, aryl-C₁-C₄-alkylsulfonyl, C₃-C₆ cycloalkyl, heterocyclyl, aryl or heteroaryl radicals optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl or C₁-C₂ haloalkyl of 1-3 halo radicals;
(2) heterocyclyl radical optionally substituted by 1-2 radicals of amino, di-(C₁-C₄ alkyl)amino, (C₁-C₄ alkoxy)carbonylamino, hydroxy, C₁-C₄ alkoxy, C₁-C₄ alkylthio or C₁-C₄ alkyl; or
(3) aryl or heteroaryl radicals optionally substituted by 1-2 radicals of amino, C₁-C₄ alkylamino, di-(C₁-C₄ alkyl)amino, acetamido, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl or trifluoromethyl radicals;
each R₂₁ is independently hydrogen radical or R₂₀;
R₁₁ is an aryl radical optionally substituted by 1-2 radicals of (1) R₃₀; (2) halo radicals; or (3) -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals;
R₁₂ is a heteroaryl radical optionally substituted by 1-2 radicals of (1) R₃₀; (2) halo radicals; or (3) -C(O)-NR₃₁R₃₂, -OR₂₉, -SR₂₉, -NR₃₁R₃₂ or -NR₃₃-C(O)-R₂₉ radicals;
each R₃₀ is independently
(1) C₁-C₄ alkyl radical optionally substituted by a phenyl or heteroaryl radical optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
(2) trifluoromethyl radical; or
(3) aryl or heteroaryl radicals optionally substituted by 1-3 radicals of amino, di-(C₁-C₂ alkyl)amino, acetamido, hydroxy, C₁-C₂ alkoxy, halo, C₁-C₄ alkyl or trifluoromethyl radicals;
each R₂₉ is independently hydrogen radical or R₃₀;
each R₃₁ is independently hydrogen or C₁-C₄ alkyl radicals; and
each R₃₂ is independently
(1) hydrogen radicals;
(2) C₁-C₄ alkyl radical or C₁-C₂ alkyl radical substituted by phenyl or heteroaryl radical optionally substituted by 1-3 radicals of amino, dimethylamino, acetamido, hydroxy, methoxy, methyl or trifluoromethyl radicals; or
(3) phenyl or heteroaryl radical optionally substituted by 1-3 radicals of amino, dimethylamino, acetamido, hydroxy, methoxy, methyl or trifluoromethyl radicals.

6. The process of claim 5 wherein
R₄ is -Z-Y; provided that the total number of aryl, heteroaryl, cycloalkyl and heterocyclyl radicals in -Z-Y is 0-1;
Z is a
(1) C₁-C₄ alkyl or C₂-C₅ alkenyl radical optionally substituted by (a) 1-3 radicals of amino, di-(C₁-C₂ alkyl)amino, (C₁-C₄ alkoxy)carbonylamino, hydroxy, C₁-C₂ alkoxy, C₁-C₂ alkylthio or halo, and (b) 1-2 radicals of aryl or heteroaryl optionally substituted by 1-2 radicals of amino, di-(C₁-C₂ alkyl)amino, acetamido, (C₁-C₄ alkoxy)carbonylamino, hydroxy, C₁-C₂ alkoxy, C₁-C₂ alkylthio, halo, C₁-C₄ alkyl or trifluoromethyl radicals; or
(2) aryl or heteroaryl radical optionally substituted by 1-3 radicals of amino, di-(C₁-C₂ alkyl)amino, acetamido, (C₁-C₄ alkoxy)carbonylamino, hydroxy, C₁-C₂ alkoxy, C₁-C₂ alkylthio, cyano, halo, C₁-C₄ alkyl or trifluoromethyl radicals;
each R₅ is independently
(1) hydrogen radical;
(2) C₁-C₄ alkyl radical optionally substituted by 1-3 halo radicals; or
(3) phenyl-C₁-C₂-alkyl or heteroaryl-C₁-C₂-alkyl, radicals optionally substituted by 1-3 radicals of amino, dimethylamino, hydroxy, methoxy, methylthio, methyl or trifluoromethyl radicals;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical optionally substituted by 1-2 radicals of hydroxy or C₁-C₄ alkyl; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
each R₂₂ is independently hydrogen or C₁-C₄ alkyl radical;
R₁₁ is an aryl radical and R₁₂ is a heteroaryl radical, wherein the aryl and heteroaryl radicals are optionally substituted by 1-2 radicals of
(1) R₃₀;
(2) halo radicals; or
(3) -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ or -NR₃₃-C(O)-R₂₉ radicals;
R₁₁ is an aryl radical optionally substituted by 1-2 radicals of amino, dimethylamino, acetamido, hydroxy, halo, cyano, methoxy, methylthio, methylsulfinyl, methylsulfonyl, aminocarbonyl, methyl or trifluoromethyl radicals;
R₁₂ is a heteroaryl radical optionally substituted by 1-2 radicals of amino, dimethylamino, acetamido, hydroxy, halo, cyano, methoxy, methyl or trifluoromethyl radicals;
each R₃₀ is independently
(1) C₁-C₄ alkyl radical optionally substituted by a phenyl or heteroaryl radical optionally substituted by 1-3 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀;
R₃₂ is independently
(1) hydrogen or C₁-C₄ alkyl radical; or
(2) phenyl or heteroaryl radical optionally substituted by 1-2 radicals of amino, dimethylamino, acetamido, hydroxy, methoxy, methyl or trifluoromethyl radicals; and
each R₃₃ is independently hydrogen or methyl radical.

7. The process of claim 6 wherein
Z is a C₁-C₄ alkyl radical optionally substituted by 1-2 radicals of amino, di-(C₁-C₂ alkyl)amino, (C₁-C₄ alkoxy)carbonylamino, hydroxy, C₁-C₂ alkoxy, C₁-C₂ alkylthio, halo or aryl or heteroaryl optionally substituted by 1-2 radicals of hydroxy, C₁-C₂ alkoxy, C₁-C₂ alkylthio, halo, C₁-C₄ alkyl or trifluoromethyl radicals;
Y is a
(1) -C(O)-R₂₀ radical;
(2) -OR₂₁, -SR₂₁, -S(O)-R₂₀, -S(O)₂-R₂₀ or -S(O)₂-NR₅R₂₁ radical; or
(3) -NR₅R₂₁, -NR₂₂-C(O)-R₂₁ or -NR₂₂-S(O)₂-R₂₀ radical;
each R₅ is independently hydrogen or C₁-C₄ alkyl radical;
each R₂₀ is independently
(1) C₁-C₆ alkyl radicals optionally substituted by 1-3 radicals of amino, methylamino, dimethylamino, t-butoxycarbonylamino, N-((t-butoxy)carbonyl)-N-(methyl)amino, aminocarbonylamino, hydroxy, butoxy, methoxy, butylthio, methylthio, methylsulfinyl, methylsulfonyl, halo or C₅-C₆ cycloalkyl, heterocyclyl, phenyl or heteroaryl radicals optionally substituted by 1-2 radicals of amino, dimethylamino, acetamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
R₁₁ is an unsubstituted phenyl or naphthyl radical or a phenyl radical substituted by 1-2 radicals of amino, dimethylamino, acetamido, hydroxy, halo, cyano, methoxy, methylthio, methylsulfinyl, methylsulfonyl, aminocarbonyl, methyl or trifluoromethyl radicals;
R₁₂ is a 4-pyridyl, 4-quinolinyl, 4-imidazolyl or 4-pyrimidinyl radical optionally substituted by a radical of amino, dimethylamino, acetamido, hydroxy, halo, cyano, methoxy, methyl or trifluoromethyl radicals;
R₃₀ is independently
(1) C₁-C₄ alkyl radical optionally substituted by a phenyl or heteroaryl radical optionally substituted by 1-2 radicals of amino, dimethylamino, acetamido, hydroxy, halo, methoxy, methyl or trifluoromethyl radicals;
(2) trifluoromethyl radical; or
(3) aryl or heteroaryl radicals optionally substituted by 1-3 radicals of amino, dimethylamino, acetamido, hydroxy, halo, methoxy, methyl or trifluoromethyl radicals;
each R₂₉ is independently hydrogen radical or R₃₀; and
each R₃₁ is independently hydrogen, methyl or ethyl radicals.

8. The process of claim 7 wherein
Z is a C₁-C₄ alkyl radical optionally substituted by 1-2 radicals of amino, t-butoxycarbonylamino, dimethylamino, hydroxy, methoxy, methylthio or halo radicals;
Y is a -OR₂₁, -SR₂₁ or -NR₅R₂₁ radical;
each R₅ is a hydrogen radical;
each R₂₀ is independently
(1) C₁-C₆ alkyl radicals optionally substituted by 1-3 radicals of amino, methylamino, dimethylamino, hydroxy or phenyl or heteroaryl radicals optionally substituted by 1-2 radicals of amino, dimethylamino, hydroxy, methoxy, methylthio, halo, methyl or trifluoromethyl radicals;
(2) heterocyclyl radical; or
(3) aryl or heteroaryl radicals optionally substituted by 1-2 radicals of amino, dimethylamino, hydroxy, methoxy, methylthio, halo, methyl or trifluoromethyl radicals;
each R₂₁ is independently hydrogen radical or R₂₀;
each R₂₂ is independently hydrogen or methyl radical;
R₁₁ is an unsubstituted phenyl radical or a phenyl radical substituted by 1-2 radicals of amino, dimethylamino, acetamido, hydroxy, halo, cyano, methoxy, methylthio, methylsulfonyl, methyl or trifluoromethyl radicals;
R₁₂ is a 4-pyridyl radical optionally substituted by a radical of amino, dimethylamino, acetamido, hydroxy, halo, cyano, methoxy, methyl or trifluoromethyl radicals; and
R₂₉ is an aryl or heteroaryl radicals optionally, substituted by 1-2 radicals of amino, dimethylamino, acetamido, hydroxy, halo, methoxy, methyl or trifluoromethyl radicals.

9. A process according to Claim 1
comprising the additional step of:
(c) adding R₂-L' ;
wherein L and L' are each independently a halo, C₁-C₄ alkylsulfate, arylsulfate, trifluoromethylsulfate, C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halogen radicals, arylcarbonyloxy, N-oxosuccinate, N-oxobenzotriazole, or cyanide radical;
and wherein
R₂ is alkyl or alkenyl radical optionally substituted by (a) 1-3 radicals of halo, alkanoyl, aroyl, alkoxycarbonyl, aryloxycarbonyl, aminocarbonyl, alkylaminocarbonyl, dialkylaminocarbonyl, aminosulfonyl, alkylaminosulfonyl, dialkylaminosulfonyl, alkylsulfonyl, arylsulfonyl, heteroarylsulfonyl, hydroxy, alkoxy, cyano, and (b) 1-2 radicals of heterocyclyl, aryl or heteroaryl optionally substituted by 1-3 radicals of amino, alkylamino, dialkylamino, alkanoylamino, alkoxycarbonylamino, alkylsulfonylamino, hydroxy, alkoxy, alkylthio, halo, alkyl, trifluoromethoxy or trifluoromethyl radicals; or (4) heteroaryl radical optionally substituted by 1-3 radicals of amino, alkylamino, dialkylamino, alkanoylamino, alkoxycarbonylamino, alkylsulfonylamino, hydroxy, alkoxy, alkylthio, cyano, halo, alkyl, trifluoromethoxy or trifluoromethyl radicals.

10. The process according to Claim 2, comprising the additional step of:
(c) adding R₂-L'; wherein L and L' are each independently a halo, C₁-C₄ alkylsulfate, arylsulfate, trifluoromethylsulfate, C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halogen radicals, arylcarbonyloxy, N-oxosuccinate, N-oxobenzotriazole, or cyanide radical;
and wherein R₂ is C₁-C₈ alkyl or C₂-C₈ alkenyl radical optionally substituted by (a) 1-3 radicals of halo, C₁-C₅ alkanoyl, aroyl, C₁-C₄ alkoxycarbonyl, aryloxycarbonyl, aminocarbonyl, C₁-C₄ alkylaminocarbonyl, di-(C₁-C₄ alkyl)aminocarbonyl, aminosulfonyl, C₁-C₄ alkylaminosulfonyl, di-(C₁-C₄ alkyl)aminosulfonyl, C₁-C₄ alkylsulfonyl, arylsulfonyl, heteroarylsulfonyl, hydroxy, C₁-C₄ alkoxy, cyano, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluoromethoxy or trifluoromethyl radicals; or (4) heteroaryl radical optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, trifluoromethoxy or trifluoromethyl radicals

11. The process according to Claim 3, comprising the additional step of: (c) adding R₂-L'; wherein L and L' are each independently a halo, C₁-C₄ alkylsulfate, arylsulfate, trifluoromethylsulfate; C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halogen radicals, arylcarbonyloxy, N-oxosuccinate, N-oxobenzotriazole, or cyanide radical; and wherein R₂ is C₁-C₈ alkyl or C₂-C₈ alkenyl radical optionally substituted by (a) 1-3 radicals of halo, C₁-C₅ alkanoyl, aroyl, C₁-C₄ alkoxycarbonyl, aryloxycarbonyl, aminocarbonyl, C₁-C₄ alkylaminocarbonyl, di-(C₁-C₄ alkyl)aminocarbonyl, aminosulfonyl, C₁-C₄ alkylaminosulfonyl, di-(C₁-C₄ alkyl)aminosulfonyl, C₁-C₄ alkylsulfonyl, arylsulfonyl, heteroarylsulfonyl, hydroxy, C₁-C₄ alkoxy, cyano, and (b) 1-2 radicals of heterocyclyl, aryl or heteroaryl optionally substituted by 1-3 radicals of amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluoromethoxy or trifluoromethyl radicals; or (4) heteroaryl radical optionally substituted by 1-3 radicals of amino, C₁-C₄ alkylamino; di-(C₁-C₄ alkyl)amino, C₁-C₅ alkanoylamino, (C₁-C₄ alkoxy) carbonylamino, C₁-C₄ alkylsulfonylamino, hydroxy, C₁-C₄ alkoxy, C₁-C₄ alkylthio, cyano, halo, C₁-C₄ alkyl, trifluoromethoxy or trifluoromethyl radicals.

12. The process according to Claim 4, comprising the additional step of:
(c) adding R₂-L' ;
wherein L is a halo, C₁-C₄ alkylsulfate, arylsulfate, trifluoromethylsulfate, C₁-C₄ alkylsulfinate, arylsulfinate, C₁-C₄ alkylsulfide, arylsulfide, C₁-C₄ alkoxy, aryloxy, C₁-C₄ alkylcarbonyloxy optionally substituted by 1-3 halogen radicals, arylcarbonyloxy, N-oxosuccinate, N-oxobenzotriazole, or cyanide radical;
and wherein
R₂ is a C₁-C₈ alkyl radical optionally substituted by (a) 1-3 radicals of halo, aroyl, C₁-C₄ alkoxycarbonyl, aryloxycarbonyl, C₁-C₄ alkylaminosulfonyl, di-(C₁-C₄ alkyl)aminosulfonyl, C₁-C₄ alkylsulfonyl, arylsulfonyl, heteroarylsulfonyl, C₁-C₄ alkoxy, cyano, and (b) 1-2 radicals of heterocyclyl, aryl or heteroaryl optionally substituted by 1-3 radicals of amino, C₁-C₄ alkylamino, di-(C₁-C₄ alkyl)amino, hydroxy, C₁-C₄ alkoxy, halo, C₁-C₄ alkyl, or trifluoromethyl radicals;
L' is a halo, C₁-C₄ alkylsulfate, arylsulfate or trifluoromethylsulfate radical,

13. The process according to Claim 5, comprising the additional step of:
c) adding R₂-L'; wherein
R₂ is a C₁-C₄ allyl radical optionally substituted by 1-3 radicals of hydrogen, a hydrogen radical, Na⁺ or K⁺; and
L' is a halo, trifluoromethylsulphate or mesylate radical.

14. The process according to Claim 6, comprising the additional step of:
c) adding R₂-L'; wherein
R₂ is a methyl or ethyl radical; and
L' is iodide.

15. The process according to Claim 7, comprising the additional step of:
c) adding R₂-L'; wherein
R₂ is a methyl or ethyl radical; and
L' is iodide.

16. The process according to Claim 8, comprising the additional step of:
c) adding R₂-L'; wherein
R₂ is a methyl or ethyl radical; and
L' is iodide.

## Patentansprüche

1. Verfahren zum Herstellen von Verbindungen der Formel I 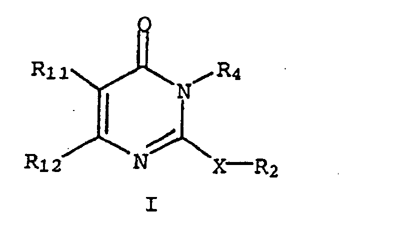 welches umfaßt:
(a) Mischen von R₁₁-CH₂-C(O)L und R₁₂-CN in einem geeigneten Lösungsmittel und in der Gegenwart einer Base; und
(b) Zufügen von R₄-N=C=X;
wobei L ein Halogen-, C₁-C₄-Alkylsulfat-, Arylsulfat-, Trifluormethylsulfat-, C₁-C₄-Alkylsulfinat-, Arylsulfinat-, C₁-C₄-Alkylsulfid-, Arylsulfid-, C₁-C₄-Alkoxy-, Aryloxy-, C₁-C₄-Alkylcarbonyloxy-, optional substituiert mit 1-3 Halogenresten, Arylcarbonyloxy-, N-Oxosuccinat-, N-Oxobenzotriazol- oder Cyanid-Rest ist;
X S oder O ist;
R₂ ein Wasserstoffrest oder Q ist;
Q ein Li⁺-, Na⁺-, K⁺-, Mg⁺²-, Zn⁺²-, Cu⁺²- oder ein Tetra-C₁-C₄-Alkylammonium-Kation ist;
R₄ -Z-Y ist; vorausgesetzt, daß die Gesamtzahl an Aryl-, Heteroaryl-, Cycloalkyl- und Heterocyclyl-Resten in -Z-Y 0-3 ist;
wobei Z ein
(1) Alkyl-, Alkenyl- oder Alkynyl-Rest, optional substituiert mit (a) 1-3 Resten von Amino, Alkylamino, Dialkylamino, Alkanoylamino, Alkoxycarbonylamino, Alkylsulfonylamino, Hydroxy, Alkoxy, Alkylthio oder Halogen, und (b) 1-2 Resten von Heterocyclyl, Aryl oder Heteroaryl, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Halogen, Alkyl oder Halogenalkyl;
(2) Heterocyclyl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Alkyl oder Halogenalkyl; oder
(3) Aryl oder Heteroaryl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Halogen, Alkyl oder Halogenalkyl ist;
Y ein
(1) Wasserstoffrest;
(2) Halogen- oder Nitro-Rest;
(3) -C(O)-R₂₀- oder -C(NR₅)-NR₅R₂₁-Rest;
(4) -OR₂₁-, -O-C(O)-R₂₁-, -O-C(O)-NR₅R₂₁- oder-O-C(O)-NR₂₂-S(O)₂-R₂₀-Rest;
(5) -SR₂₁-, -S(O)-R₂₀-, -S(O)₂-R₂₀-, -S(O)₂-NR₅R₂₁-, -S(O)₂₋NR₂₂-C(O)-R₂₁-, -S(O)₂-NR₂₂-C(O)-OR₂₀- oder -S(O)₂-NR₂₂-C(O)-NR₅R₂₁-Rest; oder
(6) -NR₅R₂₁-, -NR₂₂-C(O)-R₂₁, -NR₂₂-C(O)-OR₂₀-, -NR₂₂-C(O)-NR₅R₂₁-, -NR₂₂-C(NR₅)-NR₅R₂₁-, -NR₂₂-S(O)₂-R₂₀ oder -NR₂₂-S(O)₂-NR₅R₂₁-Rest ist;
wobei jedes R₅ unabhängig
(1) Wasserstoffreste;
(2) Alkyl-, Alkenyl- oder Alkynyl-Reste, optional substituiert mit 1-3 Resten von Amino, Alkylamino, Dialkylamino, Hydroxy, Alkoxy, Alkylthio, -SO₃H oder Halogen; oder
(3) Aryl-, Heteroaryl-, Aralkyl-, Heteroaralkyl-, Heterocyclyl-, Heterocyclylalkyl-, Cycloalkyl- oder Cycloalkylalkyl-Reste, optional substituiert mit 1-3 Resten mit Amino, Alkylamino, Dialkylamino, Hydroxy, Alkoxy, Alkylthio, Alkyl oder Halogenalkyl, ist;
wobei jedes R₂₀ unabhängig
(1) Alky-, Alkenyl- oder Alkynyl-Reste, optional substituiert mit 1-3 Resten von Amino, Alkylamino, Dialkylamino, Alkanoylamino, Alkoxycarbonylamino, N-(Alkoxycarbonyl)-N-(alkyl)amino, Aminocarbonylamino, Alkysulfonylamino, Hydroxy, Alkoxy, Alkylthio, Alkylsulfinyl, Alkylsulfonyl, Halogen oder Aralkoxy, Aralkylthio, Aralkylsulfonyl, Cycloalkyl, Heterocyclyl, Aryl oder Heteroaryl-Reste, optional substituiert mit 1-3 Resten von Amino, Alkylamino, Dialkylamino, Alkanoylamino, Alkoxycarbonylamino, Alkysulfonylamino, Alkanoyl, Hydroxy, Alkoxy, Alkylthio, Alkylsulfinyl, Alkylsulfonyl, Halogen, Alkyl oder Halogenalkyl;
(2) Heterocyclyl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Alkyl oder Halogenalkyl; oder
(3) Aryl oder Heteroaryl-Reste, optional substituiert mit 1-3 Resten von Amino, Alkylamino, Dialkylamino, Alkanoylamino, Alkoxycarbonylamino, Alkysulfonylamino, Alkoxycarbonyl, Hydroxy, Alkoxy, Alkylthio, Cyano, Halogen, Azido, Alkyl oder Halogenalkyl, ist;
jedes R₂₁ unabhängig Wasserstoffrest oder R₂₀ ist;
jedes R₂₂ unabhängig
(1) Wasserstoffrest;
(2) Alkylrest, optional substituiert mit einem Rest von Heterocyclyl, Aryl oder Heteroaryl, optional substituiert mit 1-3 Resten von Amino, Alkylamino, Dialkylamino, Alkanoylamino, Alkoxycarbonylamino, Alkysulfonylamino, Hydroxy, Alkoxy, Alkylthio, Alkylsulfinyl, Alkylsulfonyl, Cyano, Halogen, Alkyl oder Halogenalkyl; oder
(3) Heterocyclyl-, Aryl- oder Heteroaryl-Reste, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Alkylsulfinyl, Alkylsulfonyl, Cyano, Halogen, Alkyl oder Halogenalkyl, ist;
R₁₁ und R₂₂ unabhängig ein Alkyl-, Alkenyl-, Alkynyl-, Cycloalkyl-, Heterocyclyl-, Aryloder Heteroaryl-Rest, optional substituiert mit 1-3 Resten von
(1) R₃₀;
(2) Halogenresten;
(3) -C(O)-R₃₀-, -C(O)-OR₂₉-, -C(O)-NR₃₁R₃₂- oder -C(NR₃₁)-NR₃₁R₃₂-Resten;
(4) -OR₂₉-, -O-C(O)-R₂₉-, -O-C(O)-NR₃₁R₃₂- oder -O-C(O)-NR₃₃-S(O)₂-R₃₀-Resten;
(5)-SR₂₉-, -S(O)-R₃₀-, -S(O)-R₃₀-,-S(O)₂-NR₃₁R₃₂, -S(O)₂-NR₃₃-C(O)-R₃₀-, S(O)₂-NR₃₃-C(O)-OR₃₀- oder -S(O)₂-NR₃₃-C(O)-NR₃₁R₃₂-Resten; oder
(6) -NR₃₁R₃₂-, -NR₃₃-C(O)-R₂₉-, -NR₃₃-C(O)-OR₃₀-, -NR₃₃-C(O)-NR₃₁R₃₂-, -NR₃₃-C(NR₃₁)-NR₃₁R₃₂-, -NR₃₃-S(O)₂-R₃₀- oder -NR₃₃-S(O)₂-NR₃₁R₃₂-Resten ist;
vorausgesetzt, daß die Gesamtzahl an Aryl-, Heteroaryl-, Cycloaryl- und Heterocyclyl-Resten, die in jedem R₁₁ und R₁₂ substituiert sind, 0-1 ist;
wobei jedes R₃₀ unabhängig
(1) Alkyl-, Alkenyl- oder Alkynyl-Reste, optional substituiert mit 1-3 Resten von -NR₃₁R₃₁, Hydroxy, Alkoxy, Alkylthio, Alkylsulfinyl, Alkylsulfonyl, Cyano, Halogen oder Aralkoxy, Aralkylthio, Aralkylsulfonyl, Heterocyclyl, Aryl oder Heteroaryl, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Alkylsulfinyl, Alkylsulfonyl, Cyano, Halogen, Alkyl oder Halogenalkyl;
(2) Heterocyclyl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl; oder
(3) Aryl- oder Heteroaryl-Reste, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Halogen, Alkyl oder Halogenalkyl, ist;
jedes R₂₉ unabhängig Wasserstoffrest oder R₃₀ ist;
jedes R₃₁ unabhängig
(1) Wasserstoffreste;
(2) Alkyl-Rest, optional substituiert mit einem Cycloalkyl-, Aryl-, Heterocyclyl- oder Heteroaryl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl; oder
(3) Aryl-, Heteroaryl-, Heterocyclyl- oder Cycloalkyl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl, ist;
jedes R₃₂ unabhängig
(1) Wasserstoffreste;
(2) Alkyl-Rest, optional substituiert mit einem Cycloalkyl-, Aryl-, Heterocyclyl- oder Heteroaryl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl; oder
(3) Aryl-, Heteroaryl-, Heterocyclyl- oder Cycloalkyl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl, ist; und
wobei jedes R₃₃ unabhängig
(1) Wasserstoffrest; oder
(2) Alkyl-Rest, optional substituiert mit einem Rest von Heterocyclyl, Aryl oder Heteroaryl, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Alkyl oder Halogenalkyl ist.

2. Verfahren nach Anspruch 1, bei welchem
Z ein
(1) C₁-C₈-Alkyl-, C₂-C₈--Alkenyl- oder C₂-C₈-Alkynyl-Rest, optional substituiert mit (a) 1-3 Resten von Amino, C₁-C₄-Alkylamino, Di-(C₁-C₄-alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio oder Halogen, und (b) 1-2 Resten von Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Halogen, C₁-C₄-Alkyl oder C₁-C₄-Halogenalkyl von 1-3 Halogenresten;
(2) Heterocyclyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkyl oder C₁-C₄-Halogenalkyl von 1-3 Halogenresten; oder
(3) Aryl- oder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl oder C₁-C₄-Halogenalkyl von 1-3 Halogenresten, ist;
jedes R₅ unabhängig
(1) Wasserstoffreste;
(2) C₁-C₈-Alkyl-, C₂-C₈-Alkenyl- oder C₂-C₈-Alkynyl-Reste, optional substituiert mit 1-3 Resten von Amino, C₁-C₄-Alkylamino, Di-(C₁-C₄-Alkyl)amino, Hydroxy, C₁-C₄-Alkoxy, C₁-C₄-Alkylthio, -SO₃H oder Halogen; oder
(3) Aryl-, Heteroaryl-, Aryl-C₁-C₄-Alkyl-, Heteroaryl-C₁-C₄-Alkyl, Heterocyclyl-, Heterocyclyl-C₁-C₄-Alkyl, C₃-C₈-Cycloalkyl oder C₃-C₈-Cycloalkyl-C₁-C₄-Alkyl-Reste, optional substituiert mit 1-3 Resten von Amino, C₁-C₄-Alkylamino, Di-(C₁-C₄-Alkyl)amino, Hydroxy, C₁-C₄-Alkoxy, C₁-C₄-Alkylthio, C₁-C₄-Alkyl oder C₁-C₄-Halogenalkyl von 1-3 Halogenresten, ist;
jedes R₂₀ unabhängig
(1) C₁-C₈-Alkyl-, C₂-C₈-Alkenyl- oder C₂-C₈-Alkynyl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, N-((C₁-C₄-Alkoxy)carbonyl)-N-(C₁-C₄-alkyl)amino, Aminocarbonylamino, C₁-C₄-alkysulfonylamino, hydroxy, C₁-C₄-Alkoxy, C₁-C₄-Alkylthio, C₁-C₄-Alkylsulfinyl, C₁-C₄-Alkylsulfonyl, Halogen oder Aryl-C₁-C₄-Alkoxy, Aryl-C₁-C₄-Alkylthio, Aryl-C₁-C₄-Alkylsulfonyl, C₃-C₈-Cycloalkyl, Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, C₁-C₅-Alkanoyl, Hydroxy, C₁-C₄-Alkoxy, C₁-C₄-Alkylthio, C₁-C₄-Alkylsulfinyl, C₁-C₄-Alkylsulfonyl, Halogen, C₁-C₄-Alkyl oder C₁-C₄-Halogenalkyl von 1-3 Halogenresten;
(2) Heterocycyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkyl oder C₁-C₄-Halogenalkyl von 1-3 Halogenresten; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, (C₁-C₄-Alkoxy)carbonyl, Hydroxy, C₁-C₄-Alkoxy, C₁-C₄-Alkylthio, Cyano, Halogen, Azido, C₁-C₄-Alkyl oder C₁-C₄-Halogenalkyl von 1-3 Halogenresten, ist;
jedes R₂₁ unabhängig Wasserstoffrest oder R₂₀ ist;
jedes R₂₂ unabhängig
(1) Wasserstoffrest;
(2) C₁-C₄-Alkyl-Rest, optional substituiert mit einem Rest von Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkylsulfinyl, C₁-C₄-Alkylsulfonyl, Cyano, Halogen, C₁-C₄-Alkyl oder C₁-C₄-Halogenalkyl von 1-3 Halogenresten; oder
(3) Heterocyclyl-, Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkylsulfinyl, C₁-C₄-Alkylsulfonyl, Cyano, Halogen, C₁-C₄-Alkyl oder C₁-C₄-Halogenalkyl von 1-3 Halogenresten, ist;
R₁₁ und R₁₂ jeweils unabhängig ein C₁-C₆-Alkyl-, C₂-C₆-Alkenyl-, C₂-C₆-Alkynyl-, C₃-C₈-Cycloalkyl-, Hetercyclyl-, Aryl- oder Heteroaryl-Rest, optional substituiert mit 1-2 Resten von
(1) R₃₀;
(2) Halogenreste;
(3) -C(O)-R₃₀-, -C(O)-OR₂₉-, -C(O)-NR₃₁R₃₂- oder -C(NR₃₁)-NR₃₁R₃₂-Resten;
(4) -OR₂₉-, -O-C(O)-R₂₉-, -O-C(O)-NR₃₁R₃₂- oder O-C(O)-NR₃₃-S(O)₂-R₃₀-Resten;
(5) -SR₂₉-, -S(O)-R₃₀-, -S(O)-R₃₀-, -S(O)₂-NR₃₁R₃₂, -S(O)₂-NR₃₃-C(O)-R₃₀-, S(O)₂-NR₃₃-C(O)-OR₃₀- oder -S(O)₂-NR₃₃-C(O)-NR₃₁R₃₂-Resten; oder
(6) -NR₃₁R₃₂-, -NR₃₃-C(O)-R₂₉-, -NR₃₃-C(O)-OR₃₀-, -NR₃₃-C(O)-NR₃₁R₃₂-, -NR₃₃-C(NR₃₁)-NR₃₁R₃₂-, -NR₃₃-S(O)₂-R₃₀- oder -NR₃₃-S(O)₂-NR₃₁R₃₂-Resten;
vorausgesetzt, daß die Gesamtzahl an Aryl-, Heteroaryl-, Cycloalky- und Heterocyclyl-Resten, die in jedem R₁₁ und R₁₂ substituiert ist, 0-1 ist;
wobei jedes R₃₀ unabhängig
(1) C₁-C₄-Alkyl, C₂-C₄-Alkenyl- oder C₂-C₄-Alkynyl-Reste, optional substituiert mit 1-3 Resten von -NR₃₁R₃₁, Hydroxy, C₁-C₄-Alkoxy-, C₁-C₄-Alkylthio, C₁-C₄-Alkylsulfinyl, C₁-C₄-Alkylsulfonyl, Cyano, Halogen oder Aryl-C₁-C₄-Alkoxy, Aryl-C₁-C₄-Alkylthio, Aryl-C₁-C₄-Alkylsulfonyl, Heterocyclyl, Aryl oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkylsulfinyl, C₁-C₄-Alkylsulfonyl, Cyano, Halogen, C₁-C₄-Alkyl oder C₁-C₄-Halogenalkyl von 1-3 Halogenresten;
(2) Heterocyclyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl oder C₁-C₄-Halogenalkyl von 1-3 Halogenresten; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl oder C₁-C₄-Halogenalkyl von 1-3 Halogenresten;
jedes R₂₉ unabhängig Wasserstoffrest oder R₃₀ ist;
jedes R₃₁ unabhängig
(1) Wasserstoffreste;
(2) C₁-C₄-Alkyl-Rest, optional substituiert mit einem C₃-C₈-Cycloalkyl-, Aryl-, Heterocyclyloder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl, oder C₁-C₄-Halogenalkyl von 1-3 Halogenresten; oder
(3) Aryl-, Heteroaryl-, Heterocyclyl- oder C₃-C₈-Cycloalkyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl, oder C₁-C₄-Halogenalkyl von 1-3 Halogenresten, ist;
jedes R₃₂ unabhängig
(1) Wasserstoffreste;
(2) C₁-C₄-Alkyl-Rest, optional substituiert mit einem C₃-C₈-Cycloalkyl-, Aryl-, Heterocyclyloder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl, oder C₁-C₄-Halogenalkyl von 1-3 Halogenresten; oder
(3) Aryl-, Heteroaryl-, Heterocyclyl- oder C₃-C₈-Cycloalkyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl, oder C₁-C₄-Halogenalkyl von 1-3 Halogenresten; und
jedes R₃₃ unabhängig
(1) Wasserstoffrest; oder
(2) C₁-C₄-Alkyl-Rest, optional substituiert mit einem Rest von Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl, oder C₁-C₄-Halogenalkyl von 1-3 Halogenresten, ist.

3. Verfahren nach Anspruch 2, bei welchem
Z ein
(1) C₁-C₈-Alkyl-, C₂-C₈-Alkenyl- oder C₂-C₈-Alkynyl-Rest optional substituiert mit (1) 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio oder Halogen, und (b) 1-2 Resten von Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Halogen, C₁-C₄-Alkyl oder C₁-C₄-Halogenalkyl von 1-3 Halogenresten;
(2) Heterocyclyl-Rest, optional substituiert mit 1-2 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkyl oder C₁-C₄-Halogenalkyl von 1-3 Halogenresten; oder
(3) Aryl- oder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl oder C₁-C₄-Halogenalkyl von 1-3 Halogenresten, ist;
Y ein
(1) Wasserstoffrest;
(2) Halogenrest;
(3) -C(O)-R₂₀- oder -C(NR₅)-NR₅R₂₁-Rest;
(4) -OR₂₁-, -O-C(O)-R₂₁- oder -O-C(O)-NR₅-R₂₁ Rest;
(5) -SR₂₁-, -S(O)-R₂₀-, -S(O)₂-R₂₀- oder -S(O)-NR₅R₂₁-Rest; oder
(6) -NR₅-R₂₁-, -NR₂₂-C(O)-R₂₁-, -NR₂₂-C(O)-OR₂₀-, -NR₂₂-C(O)-NR₅R₂₁-, -NR₂₂-C(NR₅)-NR₅R₂₁-, -NR₂₂-S(O)₂-R₂₀- oder -NR₂₂-S(O)₂-NR₅R₂₁-Rest ist;
jedes R₅ unabhängig
(1) Wasserstoffreste;
(2) C₁-C₄-Alkyl-, C₂-C₅-Alkenyl- oder C₂-C₅-Alkynyl-Reste, optional substituiert mit 1-3 Resten von Amino, C₁-C₄-Alkylamino, Di-(C₁-C₄-Alkyl)amino, Hydroxy, C₁-C₄-Alkoxy, C₁-C₄-Alkylthio, -SO₃H oder Halogen; oder
(3) Aryl-, Heteroaryl-, Aryl-C₁-C₄-Alkyl-, Heteroaryl-C₁-C₄-Alkyl, Heterocyclyl-, Heterocyclyl-C₁-C₄-Alkyl, C₃-C₈-Cycloalkyl oder C₃-C₈-Cycloalkyl-C₁-C₄-Alkyl-Reste, optional substituiert mit 1-3 Resten von Amino, C₁-C₄-Alkylamino, Di-(C₁-C₄-Alkyl)amino, Hydroxy, C₁-C₄-Alkoxy, C₁-C₄-Alkylthio, C₁-C₄-Alkyl oder C₁-C₄-Halogenalkyl von 1-3 Halogenradikalen, ist;
jedes R₂₀ unabhängig
(1) C₁-C₈-Alkyl-, C₂-C₅-Alkenyl- oder C₂-C₅-Alkynyl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, N-((C₁-C₄-Alkoxy)carbonyl)-N-(C₁-C₄-alkyl)amino, Aminocarbonylamino, C₁-C₄-alkysulfonylamino, hydroxy, C₁-C₄-Alkoxy, C₁-C₄-Alkylthio, C₁-C₄-Alkysulfinyl, C₁-C₄-Alkylsulfonyl, Halogen oder Aryl-C₁-C₄-Alkoxy, Aryl-C₁-C₄-Alkylthio, Aryl-C₁-C₄-Alkylsulfonyl, C₃-C₈-Cycloalkyl, Heterocyclyl-, Aryloder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, C₁-C₅-Alkanoyl, Hydroxy, C₁-C₄-Alkoxy, C₁-C₄-Alkylthio, C₁-C₄-Alkylsulfinyl, C₁-C₄-Alkylsulfonyl, Halogen, C₁-C₄-Alkyl oder C₁-C₄-Halogenalkyl von 1-3 Halogenresten;
(2) Heterocyclyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, C₁-C₄-Alkyl, oder C₁-C₄-Halogenalkyl von 1-3 Halogenresten; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, (C₁-C₄-Alkoxy)carbonyl, Hydroxy, C₁-C₄-Alkoxy, C₁-C₄-Alkylthio, Cyano, Halogen, Azido, C₁-C₄-Alkyl oder C₁-C₄-Halogenalkyl von 1-3 Halogenresten, ist;
jedes R₂₁ unabhängig Wasserstoffrest oder R₂₀ ist;
R₁₁ und R₁₂ jeweils unabhängig ein C₁-C₄-Alkyl-, C₂-C₃-Alkenyl-, C₂-C₄-Alkynyl-, C₃-C₆-Cycloalkyl-, Heterocyclyl-, Aryl- oder Heteroaryl-Rest, optional substiuiert mit 1-2 Resten von
(1) R₃₀;
(2) Halogenresten;
(3) -C(O)-R₃₀-, -C(O)-OR₂₉-, -C(O)-NR₃₁R₃₂- oder -C(NR₃₁)-NR₃₁R₃₂-Resten; oder
(4) -OR₂₉-, -SR₂₉-, -S(O)-R₃₀-, -S(O)₂-R₃₀-, -S(O)₂-NR₃₁R₃₂-, -NR₃₁R₃₂-, -NR₃₃-C(O)-R₂₉oder -NR₃₃-C(O)-OR₃₀-Resten ist;
wobei jedes R₃₀ unabhängig
(1) C₁-C₄-Alkyl-Rest, optional substituiert mit 1-3 Resten von
(a) -NR₃₁R₃₁;
(b) C₁-C₄-Alkoxy-Carbonyl oder Phenoxycarbonyl oder Phenylmethoxycarbonyl, optional substituiert mit 1-3 Resten von Amino, Alkylamino, Di-(C1-C4-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl oder Trifluormethyl; oder
(c) Hydroxy, C₁-C₄-Alkoxy, C₁-C₄-Alkylthio, oder Phenyl-C₁-C₄-Alkoxy, Phenyl-C₁-C₄-Alkylthio-, Heterocyclyl-, Phenyl- oder Heteroaryl-Reste, optional substituiert mit 1-3 Resten von Amino, C₁-C₄-Alkylamino, Di-(C₁-C₄-Alkyl)amino, C₁-C₅Alkanoylamino, (C₁-C₄-Alkoxy)carbonylamino, Hydroxy, C₁-C₄-Alkoxy, C₁-C₄-Alkylthio, Cyano, Halogen, C₁-C₄-Alkyl oder C₁-C₄-Halogenalkyl von 1-3 Halogenresten;
(2) C₁-C₄-Halogenalkyl von 1-3 Halogenresten; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio, Cyano, Halogen, C₁-C₄-Alkyl oder Trifluormethyl, ist;
jedes R₂₉ unabhängig Wasserstoffrest oder R₃₀ ist;
jedes R₃₁ unabhängig
(1) Wasserstoffreste; oder
(2) C₁-C₄-Alkylrest, optional substituiert mit einem Phenyl- oder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio, Cyano, C₁-C₄-Alkyl oder Trifluoromethyl, ist; und
jedes R₃₂ unabhängig
(1) Wasserstoffreste;
(2) C₁-C₄-Alkyl-Rest, optional substituiert mit einem C₃-C₆-Cycloalkyl-, Aryl-, Heterocyclyloder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl oder C₁-C₄-Halogenalkyl von 1-3 Halogenresten; oder
(3) Aryl-, Heteroaryl-, Heterocyclyl- oder C₃-C₆-Cycloalkyl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, C₁-C₄-Alkyl oder C₁-C₄-Halogenalkyl von 1-3 Halogenresten.

4. Verfahren nach Anspruch 3, bei welchem
R₄ -Z-Y ist; vorausgesetzt, daß die Gesamtzahl an Aryl-, Heteroaryl-, Cycloalkyl- und Heterocyclyl-Resten in -Z-Y 0-2 ist;
Z ein
(1) C₁-C₈-Alkyl- oder C₂-C₈-Alkenyl-Rest, optional substituiert mit (a) 1-3 Resten von Amino, C₁-C₄-Alkylamino, Di-(C₁-C₄-alkyl)amino, C₁-C₅-Alkanoylamino, (C₁-C₄-Alkoxy)carbonylamino, Hydroxy, C₁-C₄-Alkoxy, C₁-C₄-Alkylthio oder Halogen, und (b) 1-2 Resten von Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio, Halogen, C₁-C₄-Alkyl oder C₁-C₂-Halogenalkyl von 1-3 Halogenresten;
(2) Heterocyclyl-Rest, optional substituiert mit 1-2 Resten von Amino, Di-(C₁-C₄-Alkyl)amino, (C₁-C₄-Alkoxy)carbonylamino, Hydroxy, C₁-C₄-Alkoxy, C₁-C₄-Alkylthio, C₁-C₄-Alkyl; oder
(3) Aryl- oder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl oder C₁-C₂-Halogenalkyl von 1-3 Halogenresten, ist;
Y ein
(1) Wasserstoffrest;
(2) -C(O)-R₂₀-Rest;
(3) -OR₂₁-, -SR₂₁-, -S(O)-R₂₀, -S(O)₂-R₂₀ oder -S(O)₂-NR₅R₂₁ -Rest; oder
(4) -NR₅R₂₁-, -NR₂₂-C(O)-R₂₁-, NR₂₂-C(O)-OR₂₀-, -NR₂₂-C(O)-NR₅R₂₁-, -NR₂₂-S(O)₂-R₂₀oder -NR₂₂-S(O)₂-NR₅R₂₁ -Rest, ist;
jedes R₅ unabhängig
(1) Wasserstoffreste;
(2) C₁-C₄-Alkyl- oder C₂-C₅-Alkenyl-Reste, optional substituiert mit 1-3 Resten von Amino, Di-(C₁-C₄-Alkyl)amino, Hydroxy, C₁-C₄-Alkoxy, C₁-C₄-Alkylthio, -SO₃H oder Halogen; oder
(3) Phenyl-C₁-C₂-Alkyl, Heteroaryl-C₁-C₂-Alkyl, Heterocyclyl-C₁-C₂-Alkyl oder C₃-C₆-Cycloalkyl-C₁-C₂-Alkyl-Reste, optional substituiert mit 1-3 Resten von Amino, Di-(C₁-C₄-Alkyl)amino, Hydroxy, C₁-C₄-Alkoxy, C₁-C₄-Alkylthio, C₁-C₄-Alkyl oder C₁-C₂-Halogenalkyl von 1-3 Halogenresten, ist;
jedes R₂₀ unabhängig
(1) C₁-C₈-Alkyl- oder C₂-C₅-Alkenylreste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, N-((C₁-C₄-Alkoxy)carbonyl)-N-(C₁-C₄-alkyl)amino, Aminocarbonylamino, hydroxy, C₁-C₄-Alkoxy, C₁-C₄-Alkylthio, C₁-C₄-Alkylsulfinyl, C₁-C₄-Alkylsulfonyl, Halogen, oder Aryl-C₁-C₄-Alkoxy, Aryl-C₁-C₄-Alkylthio, Aryl-C₁-C₄-Alkylsulfonyl, C₃-C₆-Cycloalkyl, Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, C₁-C₅-Alkanoyl, Hydroxy, C₁-C₄-Alkoxy, C₁-C₄-Alkylthio, Halogen, C₁-C₄-Alkyl oder C₁-C₂-Halogenalkyl von 1-3 Halogenresten;
(2) Heterocyclyl-Rest, optional substituiert mit 1-2 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio oder C₁-C₄-Alkyl; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, (C₁-C₄-Alkoxy)carbonyl, Hydroxy, C₁-C₄-Alkoxy, C₁-C₄-Alkylthio, Cyano, Halogen, Azido, C₁-C₄-Alkyl oder C₁-C₂-Halogenalkyl von 1-3 Halogenresten, ist;
jedes R₂₁ unabhängig Wasserstoffrest oder R₂₀ ist;
jedes R₂₂ unabhängig
(1) Wasserstoffrest; oder
(2) C₁-C₄-Alkylrest, optional substituiert mit einem Rest von Phenyl oder Heteroaryl, optional substituiert mit 1-3 Resten von Amino, Di-(C₁-C₂-Alkyl)amino, C₁-C₅-Alkanoylamino, (C₁-C₄-Alkoxy)carbonylamino, Hydroxy, C₁-C₄-Alkoxy, C₁-C₄-Alkylthio, Cyano, Halogen, C₁-C₄-Alkyl oder C₁-C₂-Halogenalkyl von 1-3 Halogenresten;
R₁₁ ein Aryl-Rest und R₁₂ ein Heteroarylrest ist, wobei die Aryl- und Heteroaryl-Reste optional substituiert sind mit 1-2 Resten von
(1) R₃₀;
(2) Halogenresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂oder -NR₃₃-C(O)-R₂₉ -Reste;
jedes R₃₀ unabhängig
(1) C₁-C₄-Alkylrest, optional substituiert mit
(a) Amino-, C₁-C₄-Alkylamino- oder Di-(C₁-C₄-Alkyl)amino-Resten; oder
(b) Hydroxy-, C₁-C₄-Alkoxy-, Heterocyclyl-, Phenyl- oder Heteroaryl-Resten, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio, Cyano, Halogen, C₁-C₄-Alkyl oder Trifluormethyl;
(2) C₁-C₂-Halogenalkyl von 1-3 Halogenenresten; oder
(3) Aryl- oder Heteroaryl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkylthio, Cyano, Halogen, C₁-C₄-Alkyl oder Trifluormethyl;
jedes R₂₉ unabhängig Wasserstoffrest oder R₃₀ ist;
Jedes R₂ unabhängig
(1) Wasserstoffreste;
(2) C₁-C₄-Alkylradikal optional substituiert mit Phenyl- oder Heteroaryl-Rest, optional substituiert mit 1-3 Resten von Amino, C₁-C₄-Alkylamino, Di-( C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkyl oder Trifluormethyl; oder
(3) Phenyl- oder Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, Hydroxy, C₁-C₄-Alkoxy, C₁-C₄-Alkyl oder Trifluormethyl; und
jedes R₃₃ unabhängig Wasserstoff oder C₁-C₄-Alkyl-Rest ist.

5. Verfahren nach Anspruch 4, bei welchem
L C₁-C₄-Alkoxy oder Aryloxy ist, optional substituiert mit 1-3 Resten von Halogen;
X S ist;
R₂ ein Wasserstoffrest, Na⁺ oder K⁺ ist;
Z ein
(1) C₁-C₄-Alkyl oder C₂-C₅-Alkenyl-Rest, optional substituiert mit (a) 1-3 Resten von Amino, Di-(C₁-C₂-Alkyl)amino, C₁-C₅-Alkanoylamino, (C₁-C₄-Alkoxy)carbonylamino, Hydroxy, C₁-C₂-Alkoxy, C₁-C₂-Alkylthio oder Halogen, und (b) 1-2 Resten von Heterocyclyl, Aryl oder Heteroaryl, optional substituiert mit 1-3 Resten von Amino, C₁-C₄-Alkylamino, Di-(C₁-C₂-Alkyl)amino, C₁-C₅-Alkanoylamino, (C₁-C₄-Alkoxy)carbonylamino, Hydroxy, C₁-C₄-Alkoxy, C₁-C₄-Alkylthio, Halogen, C₁-C₄-Alkyl oder Trifluormethyl;
(2) Heterocyclyl-Rest, optional substituiert mit 1-2 Resten von Amino, Di-(C₁-C₂-Alkyl)amino, (C₁-C₄-Alkoxy)carbonylamino, hydroxy, C₁-C₂-Alkoxy, C₁-C₂-Alkylthio oder C₁-C₄-Alkyl; oder
(3) Aryl- oder Heteroaryl-Rest, optional substituiert mit 1-3 Resten von Amino, Di-(C₁-C₂-Alkyl)amino, , C₁-C₅-Alkanoylamino, (C₁-C₄-Alkoxy)carbonylamino, Hydroxy, C₁-C₂-Alkoxy, C₁-C₂-Alkylthio, Cyano, Halogen, C₁-C₄-Alkyl oder Trifluormethyl ist;
Y ein
(1) Wasserstoffrest;
(2) -C(O)-R₂₀-Rest;
(3) -OR₂₁-, -SR₂₁-, -S(O)-R₂₀-, -S(O)₂-R₂₀ oder -S(O)₂-NR₅R₂₁ Rest; oder
(4) -NR₅R₂₁-, -NR₂₂-C(O)-R₂₁ oder -NR₂₂-S(O)₂-R₂₀-Rest ist;
jedes R₅ unabhängig
(1) Wasserstoffrest;
(2) C₁-C₄-Alkyl-Rest, optional substituiert mit 1-3 Resten von Amino, Di-(C₁-C₂-Alkyl)amino, Hydroxy, C₁-C₂-Alkoxy, C₁-C₂-Alkylthio oder Halogen;
(3) Phenyl-C₁-C₂-Alkyl, Heteroaryl-C₁-C₂-Alkyl, Heterocyclyl-C₁-C₂-Alkyl oder C₃-C₆-Cycloalkyl-C₁-C₂-Alkyl-Reste, optional substituiert mit 1-3 Resten von Amino, Di-(C₁-C₂-Alkyl)amino, Hydroxy, C₁-C₂-Alkoxy, C₁-C₂-Alkylthio, Methoxy, Methylthio, C₁-C₄-Alkyl oder Trifluormethyl, ist;
jedes R₂₀ unabhängig
(1) C₁-C₈-Alkyl-oder C₂-C₅-Alkenyl-Reste, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, N-((C₁-C₄-Alkoxy)carbonyl)-N-(C₁-C₄-alkyl)amino, Aminocarbonylamino, Hydroxy, C₁-C₄-Alkoxy, C₁-C₄-Alkylthio, C₁-C₄-Alkylsulfinyl, C₁-C₄-Alkylsulfonyl, Halogen, oder Aryl-C₁-C₄-Alkoxy, Aryl-C₁-C₄-Alkylthio, Aryl-C₁-C₄-Alkylsulfonyl, C₃-C₆-Cycloalkyl, Heterocyclyl, Aryl oder Heteroaryl, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, C₁-C₅-Alkanoyl, Hydroxy, C₁-C₄-Alkoxy, C₁-C₄-Alkylthio, Halogen, C₁-C₄-Alkyl oder C₁-C₄-Halogenalkyl von 1-3 Halogenresten;
(2) Heterocyclyl-Rest, optional substituiert mit 1-2 Resten von Amino, Di-(C₁-C₄-Alkyl)amino, (C₁-C₄-Alkoxy)carbonylamino, Hydroxy, C₁-C₄-Alkoxy, C₁-C₄-Alkylthio oder C₁-C₄-Alkyl; oder
(3) Aryl- oder Heteroaryl-Reste, optional substituiert mit 1-2 Resten von Amino, C₁-C₄-Alkylamino, Di-(C₁-C₄-Alkyl)amino, Acetamido, (C₁-C₄-Alkoxy)carbonylamino, C₁-C₄-Alkylsulfonylamino, (C₁-C₄-Alkoxy)carbonyl, Hydroxy, C₁-C₄-Alkoxy, C₁-C₄-Alkylthio, Cyano, Halogen, Azido, C₁-C₄-Alkyl oder Trifluormethyl, ist;
jedes R₂₁ unabhängig Wasserstoffrest oder R₂₀ ist;
R₁₁ ein Aryl-Rest ist, optional substituiert mit 1-2 Resten von (1) R₃₀; (2) Halogenresten, oder (3) -C(O)-NR₃₁R₃₂, -OR₂₉-, -SR₂₉-, -S(O)-R₃₀-, -S(O)₂-R₃₀-, -S(O)₂-NR₃₁R₃₂-, -NR₃₁R₃₂-oder -NR₃₃-C(O)-R₂₉-Resten;
R₁₂ ein Heteroaryl-Rest ist, optional substitutiert mit 1-2 Resten von (1) R₃₀; (2) Halogenresten, oder (3) -C(O)-NR₃₁R₃₂, -OR₂₉-, -SR₂₉-, -NR₃₁R₃₂-, oder -NR₃₃-C(O)-R₂₉-Resten;
jedes R₃₀ unabhängig
(1) C₁-C₄-Alkyl-Rest optional substituiert mit einem Phenyl- oder Heteroaryl-Rest, optional substituiert mit 1-3 Resten von Amino, Di-(C₁-C₂-Alkyl)amino, Acetamido, Hydroxy, C₁-C₂-Alkoxy, Halogen, C₁-C₄-Alkyl oder Trifluormethyl;
(2) Trifluormethyl-Rest; oder
(3) Aryl- oder Heteroaryl-Resten, optional substituiert mit 1-3 Resten von Amino, Di-(C₁-C₂-Alkyl)amino, Acetamido, Hydroxy, C₁-C₂-Alkoxy, Halogen, C₁-C₄-Alkyl oder Trifluormethyl;
jedes R₂₉ unabhängig Wasserstoffrest oder R₃₀ ist;
jedes R₃₁ unabhängig Wasserstoff oder C₁-C₄-Alkyl-Reste ist; und
jedes R₃₂ unabhängig
(1) Wasserstoffreste;
(2) C₁-C₄-Alkyl-Rest oder C₁-C₂-Alkyl-Rest, substituiert mit Phenyl- oder Heteroaryl-Rest, optional substiuiert mit 1-3-Resten von Amino, Dimethylamino, Acetamido, Hydroxy, Methoxy, Methyl oder Trifluormethyl; oder
(3) Phenyl oder Heteroaryl-Rest optional substituiert mit 1-3-Resten von Amino, Dimethylamino, Acetamido, Hydroxy, Methoxy, Methyl oder Trifluormethyl ist.

6. Verfahren nach Anspruch 5, bei welchem
R₄ -Z-Y ist; vorausgesetzt, daß die Gesamtzahl an Aryl-, Heteroaryl-, Cycloalkyl- und Heterocyclyl-Resten in -Z-Y 0-1 ist;
Z ein
(1) C₁-C₄-Alkyl oder C₂-C₅-Alkenyl-Rest, optional substituiert mit (a) 1-3 Resten von Amino, Di-(C₁-C₂-Alkyl)amino, (C₁-C₄-Alkoxy)carbonylamino, Hydroxy, C₁-C₂-Alkoxy, C₁-C₂-Alkylthio oder Halogen, und (b) 1-2 Resten von Aryl oder Heteroaryl, optional substituiert mit 1-2 Resten von Amino, Di-(C₁-C₂-Alkyl)amino, Acetamido, (C₁-C₄-Alkoxy)carbonylamino, Hydroxy, C₁-C₂-Alkoxy, C₁-C₂-Alkylthio, Halogen, C₁-C₄-Alkyl oder Trifluormethyl; oder
(2) Aryl- oder Heteroaryl-Rest, optional substituiert mit 1-3 Resten von Amino, Di-(C₁-C₂-Alkyl)amino, Acetamido, (C₁-C₄-Alkoxy)carbonylamino, Hydroxy, C₁-C₂-Alkoxy, C₁-C₂-Alkylthio, Cyano, Halogen, C₁-C₄-Alkyl oder Trifluormethyl ist;
jedes R₅ unabhängig
(1) Wasserstoffrest;
(2) C₁-C₄-Rest, optional substituiert mit 1-3 Halogenresten; oder
(3) Phenyl-C₁-C₂-Alkyl- oder Heteroaryl-C₁-C₂-Alkyl-Reste, optional substituiert mit 1-3 Resten von Amino, Dimethylamino, Hydroxy, Methoxy, Methylthio, Methyl oder Trifluormethyl ist:
jedes R₂₀ unabhängig
(1) C₁-C₆-Alkyl-Reste optional substituiert mit 1-3-Resten von Amino, Methylamino, Dimethylamino, t-Butoxycarbonylamino, N-((t-Butoxy)carbonyl)-N-(methyl)amino, Aminocarbonylamino, Hydroxy, Butoxy, Methoxy, Butylthio, Methylthio, Methylsulfinyl, Methylsulfonyl, Halogen, oder C₅-C₆-Cycloalkyl-, Heterocyclyl-, Phenyloder Heteroaryl-Reste, optional substituiert mit 1-2 Resten von Amino, Dimethylamino, Acetamino, Hydroxy, Methoxy, Methylthio, Halogen, Methyl oder Tritluormethyl;
(2) Heterocyclyl-Rest, optional substituiert mit 1-2-Resten von Hydroxy oder C₁-C₄-Alkyl; oder
(3) Aryl- oder Heteroaryl-Reste, optional substituiert mit 1-2-Restsen von Amino, Dimethylamino, Hydroxy, Methoxy, Methylthio, Halogen, Methyl oder Trifluormethyl ist;
jedes R₂₁ unabhängig Wasserstoffrest oder R₂₀ ist;
jedes R₂₂ unabhängig Wasserstoff- oder C₁-C₄-Alkyl-Rest ist;
R₁₁ ein Aryl-Rest und R₁₂ ein Heteroaryl-Rest ist, wobei die Aryl- und Heteroaryl-Reste optional substituiert sind mit 1-2-Resten von
(1) R₃₀;
(2) Halogenresten; oder
(3) -C(O)-NR₃₁R₃₂-, -OR₂₉-, -SR₂₉-, -S(O)-R₃₀-, -S(O)₂-R₃₀-, -S(O)₂-NR₃₁R₃₂-, -NR₃₁R₃₂-, oder -NR₃₃-C(O)-R₂₉-Resten;
R₁₁ ein Aryl-Rest ist, optional substituiert mit 1-2 Resten von Amino, Dimethylamino, Acetamido, Hydroxy, Halogen, Cyano, Methoxy, Methylthio, Methylsulfinyl, Methylsulfonyl, Aminocarbonyl, Methyl oder Trifluormethyl;
R₁₂ ein Heteroaryl-Rest ist, optional substituiert mit 1-2 Resten von Amino, Dimethylamino, Acetamido, Hydroxy, Halogen, Cyano, Methoxy, Methyl oder Trifluormethyl;
jedes R₃₀ unabhängig
(1) C₁-C₄-Alkyl-Rest, optional substituiert mit einem Phenyl- oder Heteroaryl-Rest, optional substituiert mit 1-3 Resten von Amino, Dimethylamino, Acetamido, Hydroxy, Halogen, Methoxy, Methyl oder Trifluormethyl;
(2) Trifluormethyl-Rest;
(3) Aryl- oder Heteroaryl-Reste, optional substituiert mit 1-3 Resten von Amino, Dimethylamino, Acetamido, Hydroxy, Halogen, Methoxy, Methyl oder Trifluormethyl;
jedes R₂₉ unabhängig Wasserstoffrest oder R₃₀ ist;
R₃₂ unabhängig
(1) Wasserstoff oder C₁-C₄-Alkyl-Rest ist; oder
(2) Phenyl- oder Heteroaryl-Rest, optional substituiert mit 1-2 Resten von Amino, Dimethylamino, Acetamido, Hydroxy, Methoxy, Methyl oder Trifluormethyl; und
jedes R₃₃ unabhängig Wasserstoff oder Methyl-Rest ist.

7. Verfahren nach Anspruch 6, bei welchem
Z ein C₁-C₄-Alkyl-Rest, optional substituiert mit 1-2 Resten von Amino, Di-(C₁-C₂-Alkyl)amino, (C₁-C₄-Alkoxy)carbonylamino, Hydroxy, C₁-C₂-Alkoxy, C₁-C₂-Alkylthio, Halogen, oder Aryl oder Heteroaryl, optional substituiert mit 1-2 Resten von Hydroxy, C₁-C₂-Alkoxy, C₁-C₂-Alkylthio, Halogen, C₁-C₄-Alkyl oder Trifluormethyl ist;
Y ein
(1) -C(O)-R₂₀-Rest;
(2) -OR₂₁-, -SR₂₁-, -S(O)-R₂₀-, -S(O)₂-R₂₀- oder -S(O)₂-NR₅R₂₁-Rest; oder
(3) -NR₅R₂₁-, -NR₂₂-C(O)-R₂₁- oder -NR₂₂-S(O)₂-R₂₀-Rest ist;
jedes R₅ unabhängig Wasserstoff oder C₁-C₄-Alkyl-Rest ist;
jedes R₂₀ unabhängig
(1) C₁-C₆-Alkyl-Reste, optional substituiert mit 1-3-Resten von Amino, Methylamino, Dimethylamino, t-Butoxycarbonylamino, N-((t-Butoxy)carbonyl)-N-(methyl)amino, Aminocarbonylamino, Hydroxy, Butoxy, Methoxy, Butylthio, Methylthio, Methylsulfinyl, Methylsulfonyl, Halogen oder C₅-C₆-Cycloalkyl-, Heterocyclyl-, Phenyloder Heteroaryl-Reste, optional substituiert mit 1-2 Resten von Amino, Dimethylamino, Acetamino, Hydroxy, Methoxy, Methylthio, Halogen, Methyl oder Trifluormethyl;
(2) Heterocyclyl-Rest;
(3) Aryl- oder Heteroaryl-Reste, optional substituiert mit 1-2-Resten von Amino, Dimethylamino, Hydroxy, Methoxy, Methylthio, Halogen, Methyl oder Trifluormethyl ist;
jedes R₂₁ unabhängig Wasserstoffrest oder R₂₀ ist;
R₁₁ ein unsubstituierter Phenyl- oder Naphthyl-Rest oder ein Phenyl-Rest ist, substituiert mit 1-2 Resten von Amino, Dimethylamino, Acetamido, Hydroxy, Halogen, Cyano, Methoxy, Methylthio, Methylsulfinyl, Methylsulfonyl, Aminocarbonyl, Methyl oder Trifluormethyl-Resten;
R₁₂ ein 4-Pyridyl, 4-Quinolinyl, 4-Imidazolyl oder 4-Pyrimidinyl-Rest ist, optional substituiert mit einem Rest von Amino, Dimethylamino, Acetamido, Hydroxy, Halogen, Cyano, Methoxy, Methyl oder Trifluormethyl;
R₃₀ unabhängig
(1) C₁-C₄-Alkyl-Rest, optional substituiert mit einem Phenyl- oder Heteroaryl-Rest, optional substituiert mit 1-2 Resten von Amino, Dimethylamino, Acetamido, Hydroxy, Halogen, Methoxy, Methyl oder Trifluormethyl;
(2) Trifluormethyl-Rest; oder
(3) Aryl oder Heteroaryl-Reste, optional substituiert mit 1-3 Resten von Amino, Dimethylamino, Acetamido, Hydroxy, Halogen, Methoxy, Methyl oder Trifluormethyl ist;
jedes R₂₉ unabhängig Wasserstoffrest oder R₃₀ ist; und
jedes R₃₁ unabhängig Wasserstoff, Methyl- oder Ethyl-Reste ist.

8. Verfahren nach Anspruch 7, bei welchem
Z ein C₁-C₄-Alkyl-Rest ist, optional substituiert mit 1-2 Resten von Amino, t-Butoxycarbonylamino, Dimethylamino, Hydroxy, Methoxy, Methylthio oder Halogen;
Y ein -OR₂₁-, -SR₂₁- oder -NR₅R₂₁-Rest ist;
jedes R₅ ein Wasserstoffrest ist;
jedes R₂₀ unabhängig
(1) C₁-C₆-Alkyl-Reste, optional substituiert mit 1-3 Resten von Amino, Methylamino, Dimethylamino, Hydroxy, oder Phenyl- oder Heteroaryl-Reste, optional substituiert mit 1-2 Resten von Amino, Dimethylamino, Hydroxy, Methoxy, Methylthio, Halogen, Methyl oder Trifluormethyl;
(2) Heterocyclyl-Rest; oder
(3) Aryl- oder Heteroaryl-Reste, optional substituiert mit 1-2 Resten von Amino, Dimethylamino, Hydroxy, Methoxy, Methylthio, Halogen, Methyl oder Trifluormethyl, ist;
jedes R₂₁ unabhängig Wasserstoffrest oder R₂₀ ist;
jedes R₂₂ unabhängig Wasserstoff oder Methyl-Rest ist;
R₁₁ ein unsubstituierter Phenyl-Rest oder ein Phenyl-Rest ist, substituiert mit 1-2 Resten von Amino, Dimethylamino, Acetamido, Hydroxy, Halogen, Cyano, Methoxy, Methylthio, Methylsulfonyl, Methyl oder Trifluormethyl;
R₁₂ ein 4-Pyridyl-Rest ist, optional substituiert mit einem Rest von Amino, Dimethylamino, Acetamido, Hydroxy, Halogen, Cyano, Methoxy, Methyl oder Trifluormethyl;
R₂₉ ein Aryl- oder Heteroaryl-Rest ist, optional substituiert mit 1-2 Resten von Amino, Dimethylamino, Acetamido, Hydroxy, Halogen, Methoxy, Methyl oder Trifluormethyl.

9. Verfahren nach Anspruch 1, welches den zusätzlichen Schritt umfaßt:
(c) Zufügen von R₂-L';
wobei L und L' jeweils unabhängig ein Halogen, C₁-C₄-Alkylsulfat, Arylsulfat, Trifluormethylsulfat, C₁-C₄-Alkylsulfinat, Arylsulfinat, C₁-C₄-Alkylsulfid, Arylsulfid, C₁-C₄-Alkoxy, Aryloxy, C₁-C₄-Alkylcarbonyloxy, optional substituiert mit 1-3 Halogenresten, Arylcarbonyloxy, N-Oxosuccinat, N-Oxobenzotriazol oder Cyanid-Rest ist;
und wobei
R₂ ein Alkyl- oder Alkenyl-Rest ist, optional substituiert mit
(a) 1-3 Resten von Halogen, Alkanoyl, Aroyl, Alkoxycarbonyl, Aryloxycarbonyl, Aminocarbonyl, Alkylaminocarbonyl, Dialkylaminocarbonyl, Aminosulfonyl, Alkylaminosulfonyl, Dialkylsulfonyl, Alkylsulfonyl, Arylsulfonyl, Heterarylsulfonyl, Hydroxy, Alkoxy, Cyano, und (b) 1-2 Resten von Heterocyclyl, Aryl oder Heteraryl, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Halogen, Alkyl, Trifluormethoxy oder Trifluormethyl; oder
(4) Heteroaryl-Rest, optional substituiert mit 1-3 Resten von Amino, Alkylamino, Dialkylamino, Alkanoylamino, Alkoxycarbonylamino, Alkylsulfonylamino, Hydroxy, Alkoxy, Alkylthio, Cyano, Halogen, Alkyl, Trifluormethoxy oder Trifluormethyl.

10. Verfahren nach Anspruch 2, welches den zusätzlichen Schritt umfaßt von:
(c) Zufügen von R₂-L';wobei L und L' jeweils unabhängig ein Halogen, C₁-C₄-Alkylsulfat, Arylsulfat, Trifluormethylsulfat, C₁-C₄-Alkylsulfinat, Arylsulfinat, C₁-C₄-Alkylsulfid, Arylsulfid, C₁-C₄-Alkoxy, Aryloxy, C₁-C₄-Alkylcarbonyloxy, optional substituiert mit 1-3 Halogenresten, Arylcarbonyloxy, N-Oxosuccinat, N-Oxobenzotriazol oder Cyanid-Rest ist;
und wobei R₂ ein C₁-C₈-Alkyl- oder C₂-C₈-Alkenyl-Rest ist, optional substituiert mit (a) 1-3 Resten von Halogen, C₁-C₅-Alkanoyl, Aroyl, C₁-C₄-Alkoxycarbonyl, Aryloxycarbonyl, Aminocarbonyl, C₁-C₄-Alkylaminocarbonyl, Di-( C₁-C₄-Alkyl)aminocarbonyl, Aminosulfonyl, C₁-C₄-Alkylaminosulfonyl, di-(C₁-C₄-alkyl)aminosulfonyl, C₁-C₄-Alkylsulfonyl, Arylsulfonyl, Heteroarylsulfonyl, Hydroxy, C₁-C₄-Alkoxy, Cyano, und (b) 1-2 Resten von Heterocyclyl, Aryl oder Heteroaryl, optional substituiert mit 1-3 Resten von Amino, C₁-C₄-Alkylamino, Di-( C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Halogen, C₁-C₄-Alkyl, Trifluormethoxy oder Trifluormethyl; oder (4) Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl, Trifluormethoxy oder Trifluormethyl.

11. Verfahren nach Anspruch 3, welches den zusätzlichen Schritt umfaßt:
Zufügen von R₂-L';wobei L und L' jeweils unabhängig ein Halogen, C₁-C₄-Alkylsulfat, Arylsulfat, Trifluormethylsulfat, C₁-C₄-Alkylsulfmat, Arylsulfinat, C₁-C₄-Alkylsulfid, Arylsulfid, C₁-C₄-Alkoxy, Aryloxy, C₁-C₄-Alkylcarbonyloxy, optional substituiert mit 1-3 Halogenresten, Arylcarbonyloxy, N-Oxosuccinat, N-Oxobenzotriazol oder Cyanid-Rest ist;
und wobei R₂ ein C₁-C₈-Alkyl- oder C₂-C₈-Alkenyl-Rest ist, optional substituiert mit (a) 1-3 Resten von Halogen, C₁-C₅-Alkanoyl, Aroyl, C₁-C₄-Alkoxycarbonyl, Aryloxycarbonyl, Aminocarbonyl, C₁-C₄-Alkylaminocarbonyl, Di-(C₁-C₄-Alkyl)aminocarbonyl, Aminosulfonyl, C₁-C₄-Alkylaminosulfonyl, Di-(C₁-C₄-Alkyl)aminosulfonyl, C₁-C₄-Alkylsulfonyl, Arylsulfonyl, Heteroarylsulfonyl, Hydroxy, C₁-C₄-Alkoxy, Cyano, und (b) 1-2 Resten von Heterocyclyl, Aryl oder Heteroaryl optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Halogen, C₁-C₄-Alkyl, Trifluormethoxy oder Trifluormethyl; oder (4) Heteroaryl-Rest, optional substituiert mit 1-3 Resten von Amino, C₁-C₄-Alkylamino, Di-(C₁-C₄-Alkyl)amino, C₁-C₅-Alkanoylamino, (C₁-C₄-Alkoxy)carbonylamino, C₁-C₄-Alkylsulfonylamino, Hydroxy, C₁-C₄-Alkoxy, C₁-C₄-Alkylthio, Cyano, Halogen, C₁-C₄-Alkyl, Trifluormethoxy oder Trifluormethyl.

12. Verfahren nach Anspruch 4, welches den zusätzlichen Schritt umfaßt von
(c) Zufügen von R₂-L';wobei L unabhängig ein Halogen, C₁-C₄-Alkylsulfat, Arylsulfat, Trifluormethylsulfat, C₁-C₄-Alkylsulfinat, Arylsulfinat, C₁-C₄-Alkylsulfid, Arylsulfid, C₁-C₄-Alkoxy, Aryloxy, C₁-C₄-Alkylcarbonyloxy optional substituiert mit 1-3 Halogenresten, Arylcarbonyloxy, N-Oxosuccinat, N-Oxobenzotriazol oder Cyanid-Rest ist;
und wobei
R₂ ein C₁-C₈-Alkyl-Rest ist, optional substituiert mit (a) 1-3 Resten von Halogen, Aroyl, C₁-C₄-Alkoxycarbonyl, Aryloxycarbonyl, C₁-C₄-Alkylaminosulfonyl, Di-(C₁-C₄-Alkyl)aminosulfonyl, C₁-C₄-Alkylsulfonyl, Arylsulfonyl, Heteroarylsulfonyl, C₁-C₄-Alkoxy, Cyano, und (b) 1-2 Resten von Heterocyclyl, Aryl oder Heteroaryl, optional substituiert mit 1-3 Resten von Amino, C₁-C₄-Alkylamino, Di-(C₁-C₄-Alkyl)amino, Hydroxy, C₁-C₄-Alkoxy, Halogen, C₁-C₄-Alkyl oder Trifluormethyl ist:
L' ein Halogen-, C₁-C₄-Alkylsufat, Arylsulfat oder Triluormethylsulfat-Rest ist.

13. Verfahren nach Anspruch 5, welches den zusätzlichen Schritt umfaßt von:
c) Zufügen von R₂-L'; wobei
R₂ ein C₁-C₄-Alky-Rest ist, optional substituiert mit 1-3 Resten von Wasserstoff, einem Wasserstoffrest, Na⁺ oder K⁺; und
L' ein Halogen-, Trifluormethylsulfat- oder Mesylat-Rest ist.

14. Verfahren nach Anspruch 6, welches den zusätzlichen Schritt umfaßt von:
c) Zufügen von R₂-L'; wobei
R₂ ein Methyl- oder Ethyl-Rest ist; und
L' Iodid ist.

15. Verfahren nach Anspruch 7, welches den zusätzlichen Schritt umfaßt:
c) Zufügen von R₂-L'; wobei
R₂ ein Methyl- oder Ethyl-Rest ist; und
L' Iodid ist.

16. Verfahren nach Anspruch 8, welches den zusätzlichen Schritt umfaßt:
c) Zufügen von R₂-L'; wobei
R₂ ein Methyl- oder Ethyl-Rest ist; und
L' Iodid ist.

## Revendications

1. Un procédé de préparation de composés de formule I 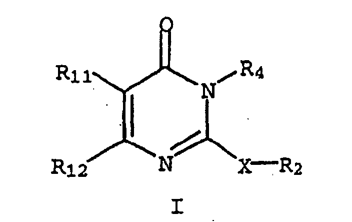 comprenant
(a) le mélange de R₁₁-CH₂-C(O)L et de R₁₂-CN dans un solvant approprié et en présence de base ; et
(b) l'addition de R₄-N=C=X ;
dans lequel L est un radical halo, C₁-C₄ alkylsulfate, arylsulfate, trifluorométhylsulfate, C₁-C₄ alkylsulfinate, arylsulfinate, C₁-C₄ alkylsulfure, arylsulfure C₁-C₄ alkoxy, aryloxy, C₁-C₄ alkylcarbonyloxy éventuellement substitué par des radicaux 1-3 halogène, arylcarbonyloxy, N-oxosuccinate, N-oxobenzotriazole, ou cyanure ;
X est S ou O ;
R₂ est un radical hydrogène ou Q ;
Q est un cation Li⁺, Na⁺, K⁺, Mg⁺², Zn⁺², Cu⁺², ou un cation tetra C₁-C₄ alkylammonium ;
R₄ est -Z-Y ; pourvu que le nombre total de radicaux aryl, hétéroaryl, cycloalkyl et hétérocyclyl dans -Z-Y est 0-3 ;
dans lequel Z est un
(1) radical alkyl, alkényl ou alkynyl éventuellement substitué par (a) 1-3 radicaux de amino, alkylamino, dialkylamino, alkanoylamino, alkoxycarbonylamino, alkylsulfonylamino, hydroxy, alkoxy, alkylthio ou halo, et (b) 1-2 radicaux de hétérocyclyl, aryl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, halo, alkyl ou haloalkyl ;
(2) radical hétérocyclyl éventuellement substitué par 1-3 radicaux de amino, alkylamino, dialkylamino, alkanoylamino, alkoxycarbonylamino, alkylsulfonylamino, hydroxy, alkoxy, alkylthio, alkyl ou haloalkyl ; ou
(3) radical aryl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, cyano, halo, alkyl ou haloalkyl ;
Y est un
(1) radical hydrogène ;
(2) radical halo ou nitro ;
(3) radical -C(O)-R₂₀ ou -C(NR₅)-NR₅R₂₁ ;
(4) radical -OR₂₁, -O-C(O)-R₂₁, -O-C(O)-NR₅R₂₁ ou -O-C(O)-NR₂₂-S(O)₂-R₂₀ ;
(5) radical -SR₂₁, -S(O)-R₂₀, -S(O)₂-R₂₀, -S(O)₂-NR₅R₂₁,-S(O)₂-NR₂₂-C(O)-R₂₁, -S(O)₂-NR₂₂-C(O)-OR₂₀ ou -S(O)₂-NR₂₂-C(O)- NR₅R₂₁ ; ou
(6) radical -NR₅R₂₁, -NR₂₂-C(O)-R₂₁, -NR₂₂-C(O)-OR₂₀, -NR₂₂-C(O)-NR₅R₂₁, -NR₂₂-C(NR₅)-NR₅R₂₁, -NR₂₂-S(O)₂-R₂₀ ou -NR₂₂-S(O)₂-NR₅R₂₁ ;
dans lequel chaque R₅ est indépendamment
(1) des radicaux hydrogène ;
(2) des radicaux alkyl, alkényl ou alkynyl éventuellement substitués par 1-3 radicaux de amino, alkylamino, dialkylamino, hydroxy, alkoxy, alkylthio, -SO₃H ou halo ; ou
(3) des radicaux aryl, hétéroaryl, aralkyl, hétéroaralkyl, hétérocyclyl, hétérocyclylalkyl, cycloalkyl ou cycloalkylalkyl éventuellement substitués par 1-3 radicaux de amino, alkylamino, dialkylamino, hydroxy, alkoxy, alkylthio, alkyl ou haloalkyl ;
dans lequel chaque R₂₀ est indépendamment
(1) des radicaux alkyl, alkényl ou alkynyl éventuellement substitués par 1-3 radicaux de amino, alkylamino, dialkylamino, alkanoylamino, alkoxycarbonylamino, N-(alkoxycarbonyl)-N-(alkyl)amino, aminocarbonylamino, alkylsulfonylamino, hydroxy, alkoxy, alkylthio, alkylsulfinyl, alkylsulfonyl, halo ou aralkoxy, aralkylthio, aralkylsulfonyl, cycloalkyl, hétérocyclyl, aryl ou hétéroaryl éventuellement substitués par 1-3 radicaux de amino, alkylamino, dialkylamino, alkanoylamino, alkoxycarbonylamino, alkylsulfonylamino, alkanoyl, hydroxy, alkoxy, alkylthio, alkylsulfinyl, alkylsulfonyl, halo, alkyl ou haloalkyl ;
(2) un radical hétérocyclyl éventuellement substitué par 1-3 radicaux de amino, alkylamino, dialkylamino, alkanoylamino, alkoxycarbonylamino, alkylsulfonylamino, hydroxy, alkoxy, alkylthio, alkyl ou haloalkyl ; ou
(3) des radicaux aryl ou hétéroaryl éventuellement substitués par 1-3 radicaux de amino, alkylamino, dialkylamino, alkanoylamino, alkoxycarbonylamino, alkylsulfonylamino, alkoxycarbonyl, hydroxy, alkoxy, alkylthio, cyano, halo, azido, alkyl ou haloalkyl ;
chaque R₂₁ est indépendamment un radical hydrogène ou R₂₀ ;
chaque R₂₂ est indépendamment
(1) un radical hydrogène ;
(2) un radical alkyl éventuellement substitué par un radical de hétérocyclyl, aryl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl ou haloalkyl ; ou
(3) des radicaux hétérocyclyl, aryl ou hétéroaryl éventuellement substitués par 1-3 radicaux de amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl ou haloalkyl ;
R₁₁ et R₁₂ sont chacun indépendamment un radical alkyl, alkényl, alkynyl, cycloalkyl, hétérocyclyl, aryl ou hétéroaryl éventuellement substitué par 1-3 radicaux de
(1) R₃₀ ;
(2) radicaux halo ;
(3) radicaux -C(O)-R₃₀, -C(O)-OR₂₉, -C(O)-NR₃₁R₃₂ ou -C(NR₃₁)-NR₃₁R₃₂ ;
(4) radicaux -OR₂₉, -O-C(O)-R₂₉, -O-C(O)-NR₃₁R₃₂ ou -O-C(O)-NR₃₃ -S(O)₂R₃₀ ;
(5) radicaux -SR₂₉, -S(O)-R₃₀, -S(O)₂-R₃₀, -S(O)₂-NR₃₁R₃₂, -S(O)₂-NR₃₃-C(O) -R₃₀, -S(O)₂-NR₃₃-C(O)-OR₃₀ ou -S(O)₂-NR₃₃-C(O)-NR₃₁R₃₂ ; ou
(6) radicaux -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃-S(O)₂-R₃₀ ou -NR₃₃-S(O)₂-NR₃₁R₃₂ ;
pourvu que le nombre total de radicaux aryl, hétéroaryl, cycloalkyl et hétérocyclyl substitués, dans chaque R₁₁ et R₁₂ est 0-1 ;
dans lequel chaque R₃₀ est indépendamment
(1) des radicaux alkyl, alkényl ou alkynyl éventuellement substitués par 1-3 radicaux de -NR₃₁R₃₁, hydroxy, alkoxy, alkylthio, alkylsulfinyl, alkylsulfonyl, cyano, halo ou aralkoxy, aralkylthio, aralkylsulfonyl, hétérocyclyl, aryl ou hétéréoaryl éventuellement substitués par 1-3 radicaux de amino, alkylamino, dialkylamino, alkanoylamino, alkoxycarbonylamino, alkylsulfonylamino, hydroxy, alkoxy, alkylthio, alkylsulfinyl, alkylsulfonyl, cyano, halo, alkyl ou haloalkyl ;
(2) un radical hétérocyclyl éventuellement substitué par 1-3 radicaux de amino, alkylamino, dialkylamino, alkanoylamino, alkoxycarbonylamino, alkylsulfonylamino, hydroxy, alkoxy, alkylthio, cyano, alkyl ou haloalkyl ; ou
(3) des radicaux aryl ou hétéroaryl éventuellement substitués par 1-3 radicaux de amino, alkylamino, dialkylamino, alkanoylamino, alkoxycarbonylamino, alkylsulfonylamino, hydroxy, alkoxy, alkylthio, cyano, halo, alkyl ou haloalkyl ;
chaque R₂₉ est indépendamment un radical hydrogène ou R₃₀ ;
chaque R₃₁ est indépendamment
(1) des radicaux hydrogène ;
(2) un radical alkyl éventuellement substitué par un radical cycloalkyl, aryl, hétérocyclyl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, cyano, alkyl ou haloalkyl ; ou
(3) un radical aryl, hétéroaryl, hétérocyclyl ou cycloalkyl éventuellement substitué par 1-3 radicaux de amino, alkylamino, dialkylamino, alkanoylamino, alkoxycarbonylamino, alkylsulfonylamino, hydroxy, alkoxy, alkylthio, cyano, halo, alkyl ou haloalkyl ;
chaque R₃₂ est indépendamment
(1) des radicaux hydrogène ;
(2) un radical alkyl éventuellement substitué par un radical cycloalkyl, aryl, hétérocyclyl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, cyano, alkyl ou haloalkyl ; ou
(3) un radical aryl, hétéroaryl, hétérocyclyl ou cycloalkyl éventuellement substitué par 1-3 radicaux de amino, alkylamino, dialkylamino, alkanoylamino, alkoxycarbonylamino, alkylsulfonylamino, hydroxy, alkoxy, alkylthio, cyano, alkyl ou haloalkyl ; et
dans lequel chaque R₃₃ est indépendamment
(1) un radical hydrogène ; ou
(2) un radical alkyl éventuellement substitué par un radical de hétérocyclyl, aryl ou hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, cyano, alkyl ou haloalkyl.

2. Le procédé de la revendication 1, dans lequel
Z est un
(1) radical C₁-C₈ alkyl, C₂-C₈ alkényl ou C₂-C₈ alkynyl éventuellement substitué par (a) 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio ou halo, et (b) 1-2 radicaux de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl ou C₁-C₄ haloalkyl de 1-3 radicaux halo ;
(2 radical hétérocyclyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl ou C₁-C₄ haloalkyl de 1-3 radicaux halo ; ou
(3) radical aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl ou C₁-C₄ haloalkyl de 1-3 radicaux halo ;
chaque R₅ est indépendamment
(1) des radicaux hydrogène ;
(2) des radicaux C₁-C₈ alkyl, C₂-C₈ alkényl ou C₂-C₈ alkynyl éventuellement substitués par 1-3 radicaux de amino, C₁-C₄ alkylamino, di-(C₁-C₄-alkyl)amino, hydroxy, C₁-C₄ alkoxy, C₁-C₄ alkylthio, -SO₃H ou halo ; ou
(3) des radicaux aryl, hétéroaryl, aryl-C₁-C₄-alkyl, hétéroaryl-C₁-C₄-alkyl, hétérocyclyl, hétérocyclyl-C₁-C₄-alkyl, C₃-C₈ cycloalkyl ou C₃-C₈-cycloalkyl-C₁-C₄-alkyl éventuellement substitués par 1-3 radicaux de amino, C₁-C₄ alkylamino, di-(C₁-C₄-alkyl)amino, hydroxy, C₁-C₄ alkoxy, C₁-C₄ alkylthio, C₁-C₄ alkyl ou C₁-C₄ haloalkyl de 1-3 radicaux halo ;
chaque R₂₀ est indépendamment
(1) des radicaux C₁-C₈ alkyl, C₂-C₈ alkényl ou C₂-C₈ alkynyl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo ou aryl- C₁-C₄ alkoxy, aryl-C₁-C₄ alkylthio, aryl-C₁-C₄-alkylsulfonyl, C₃-C₈ cycloalkyl, hétérocyclyl, aryl ou des radicaux hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, C₁-C₄ alkylsulfinyl, C₁-C₄ alkylsulfonyl, halo, C₁-C₄ alkyl ou C₁-C₄ haloalkyl de 1-3 radicaux halo ;
(2) un radical hétérocyclyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl ou C₁-C₄ haloalkyl de 1-3 radicaux halo ; ou
(3) radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl ou C₁-C₄ haloalkyl de 1-3 radicaux halo ;
chaque R₂₁ est indépendamment un radical hydrogène ou R₂₀ ;
chaque R₂₂ est indépendamment
(1) un radical hydrogène ;
(2) un radical C₁-C₄ alkyl éventuellement substitué par un radical de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo C₁-C₄ alkyl ou C₁-C₄ haloalkyl de 1-3 radicaux halo ;
(3) de radicaux hétérocyclyl, aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl ou C₁-C₄ haloalkyl de 1-3 radicaux halo ;
R₁₁ et R₁₂ sont chacun indépendamment un radical C₁-C₆ alkyl, C₂-C₆ alkényl, C₂-C₆ alkynyl, C₃-C₈ cycloalkyl, hétérocyclyl, aryl ou hétéroaryl éventuellement substitué, par 1-2 radicaux de
(1) R₃₀ ;
(2) radicaux halo ;
(3) radicaux -C(O)-R₃₀, -C(O)-OR₂₉, -C(O)-NR₃₁R₃₂ ou -C(NR₃₁)-NR₃₁R₃₂ ;
(4) radicaux -OR₂₉, -O-C(O)-R₂₉, -O-C(O)-NR₃₁R₃₂ ou -O-C(O)-NR₃₃-S(O)₂ -R₃₀ ;
(5) radicaux -SR₂₉, -S(O)-R₃₀, -S(O)₂-R₃₀, -S(O)₂-NR₃₁R₃₂, -S(O)₂-NR₃₃ -C(O) - R₃₀, -S(O)₂-NR₃₃-C(O)-OR₃₀ ou -S(O)₂-NR₃₃-C(O)-NR₃₁R₃₂ ; ou
(6) radicaux -NR₃₁R₃₂, -NR₃₃-C(O)-R₂₉, -NR₃₃-C(O)-OR₃₀, -NR₃₃-C(O)-NR₃₁R₃₂, -NR₃₃-C(NR₃₁)-NR₃₁R₃₂, -NR₃₃- S(O)₂-R₃₀ ou -NR₃₃- S(O)₂-NR₃₁R₃₂ ;
pourvu que le nombre total de radicaux aryl, hétéroaryl, cycloalkyl et hétérocyclyl substitués sur chaque R₁₁ et R₁₂ est 0-1 ;
chaque R₃₀ est indépendamment
(1) des radicaux C₁-C₄ alkyl, C₂-C₄ alkényl ou C₂-C₄ alkynyl éventuellement substitués par 1-3 radicaux de -NR₃₁R₃₁, hydroxy, C₁-C₄ alkoxy, C₁-C₄ alkylthio, C₁-C₄ alkylsulfinyl, C₁-C₄ alkylsulfonyl, cyano, halo ou aryl -C₁-C₄-alkoxy, aryl-C₁-C₄-alkylthio, aryl-C₁-C₄-alkylsulfonyl, hétérocyclyl, aryl ou des radicaux hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, cyano, halo, C₁-C₄ alkyl ou C₁-C₄ haloalkyl de 1-3 radicaux halo ;
(2) radical hétérocyclyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ; ou
(3) des radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl ou C₁-C₄ haloalkyl de 1-3 radicaux halo ;
chaque R₂₉ est indépendamment le radical hydrogène ou R₃₀ ;
chaque R₃₁ est indépendamment
(1) des radicaux hydrogène ;
(2) un radical C₁-C₄ alkyl éventuellement substitué par un radical C₃-C₈ cycloalkyl, aryl, hétérocyclyl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ; ou
(3) un radical aryl, hétéroaryl, hétérocyclyl ou C₃-C₈ cycloalkyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ;
chaque R₃₂ est indépendamment
(1) des radicaux hydrogène ;
(2) un radical C₁-C₄ alkyl éventuellement substitué par un radical C₃-C₈ cycloalkyl, aryl, hétérocyclyl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ; ou
(3) un radical aryl, hétéroaryl, hétérocyclyl ou C₃-C₈ cycloalkyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonyiamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ; et
chaque R₃₃ est indépendamment
(1) un radical hydrogène ; ou
(2) un radical C₁-C₄ alkyl éventuellement substitué par un radical de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo.

3. Le procédé de la revendication 2, dans lequel
Z est un
(1) radical C₁-C₈ alkyl, C₂-C₈ alkényl ou C₂-C₈ alkynyl éventuellement substitué par (a) 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, ou halo, et (b) 1-2 radicaux de hétérocyclyl, aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl ou C₁-C₄ haloalkyl de 1-3 radicaux halo ;
(2) radical hétérocyclyl éventuellement substitué par 1-2 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl ou C₁-C₄ haloalkyl de 1-3 radicaux halo ; ou
(3) radical aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl ou C₁-C₄ haloalkyl de 1-3 radicaux halo ;
Y est un
(1) radical hydrogène ;
(2) radical halo ;
(3) radical -C(O)-R₂₀ ou -C(NR₅)-NR₅R₂₁ ;
(4) radical -OR₂₁, -O-C(O)-R₂₁ ou -O-C(O)-NR₅R₂₁ ;
(5) radical -SR₂₁, -S(O)-R₂₀, -S(O)₂- R₂₀ ou -S(O)₂-NR₅R₂₁ ; ou
(6) -NR₅R₂₁, -NR₂₂-C(O)-R₂₁, -NR₂₂-C(O)-OR₂₀, -NR₂₂-C(O)-NR₅R₂₁, -NR₂₂ -C(NR₅)-NR₅R₂₁, -NR₂₂-S(O)₂- R₂₀ ou -NR₂₂-S(O)₂- NR₅R₂₁ ;
chaque R₅ est indépendamment
(1) des radicaux hydrogène
(2) des radicaux C₁-C₄ alkyl, C₂-C₅ alkényl ou C₂-C₅ alkynyl éventuellement substitués par 1-3 radicaux de amino, C₁-C₄ alkylamino, di-(C₁-C₄ alkyl)amino, hydroxy, C₁-C₄ alkoxy, C₁-C₄ alkylthio, -SO₃H ou halo ; ou
(3) des radicaux aryl, hétéroaryl, aryl-C₁-C₄-alkyl, hétéroaryl-C₁-C₄-alkyl, hétérocyclyl, hétérocyclyl-C₁-C₄-alkyl, C₃-C₈ cycloalkyl ou C₃-C₈-cycloalkyl-C₁-C₄-alkyl éventuellement substitués par 1-3 radicaux de amino, C₁-C₄ alkylamino, di-(C₁-C₄ alkyl)amino, hydroxy, C₁-C₄ alkoxy, C₁-C₄ alkylthio, C₁-C₄ alkyl ou C₁-C₄ haloalkyl de 1-3 radicaux halo ;
chaque R₂₀ est indépendamment
(1) des radicaux C₁-C₈ alkyl, C₂-C₅ alkényl ou C₂-C₅ alkynyl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, C₁-C₄ alkylsulfonylamino, hydroxy, C₁-C₄ alkoxy, C₁-C₄ alkylthio, C₁-C₄ alkylsulfinyl, C₁-C₄ alkylsulfonyl, halo ou aryl-C₁-C₄-alkoxy, aryl-C₁-C₄-alkylthio, aryl-C₁-C₄-alkylsulfonyl, C₃-C₈ cycloalkyl, hétérocyclyl, aryl ou des radicaux hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkonoyl, hydroxy, C₁-C₄ alkoxy, C₁-C₄ alkylthio, C₁-C₄ alkylsulfinyl, C₁-C₄ alkylsulfonyl, halo, C₁-C₄ alkyl ou C₁-C₄ haloalkyl de 1-3 radicaux halo ;
(2) un radical hétérocyclyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, C₁-C₄ alkyl ou C₁-C₄ haloalkyl de 1-3 radicaux halo ; ou
(3) des radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl ou C₁-C₄ haloalkyl de 1-3 radicaux halo ;
chaque R₂₁ est indépendamment un radical hydrogène ou R₂₀ ;
R₁₁ et R₁₂ sont chacun indépendamment un radical C₁-C₄ alkyl, C₂-C₄ alkényl, C₂-C₄ alkynyl, C₃-C₆ cycloalkyl, hétérocyclyl, aryl ou hétéroaryl éventuellement substitué par 1-2 radicaux de
(1) R₃₀ ;
(2) radicaux halo ;
(3) radicaux -C(O)-R₃₀, -C(O)-OR₂₉, -C(O)-NR₃₁R₃₂ ou -C(NR₃₁)-NR₃₁R₃₂ ; ou
(4) radicaux -OR₂₉, -SR₂₉, -S(O)-R₃₀, -S(O)₂-R₃₀, -S(O)₂-NR₃₁R₃₂, -NR₃₁R₃₂, -NR₃₃-C(O)-R₂₉ ou -NR₃₃-C(O)-OR₃₀ ;
chaque R₃₀ est indépendamment
(1) le radical C₁-C₄ alkyl, éventuellement substitué par 1-3 radicaux de
(a) -NR₃₁R₃₁ ;
(b) C₁-C₄ alkoxy-carbonyl ou phénoxycarbonyl ou phénylméthoxycarbonyl éventuellement substitué par 1-3 radicaux de amino, alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl ou trifluorométhyl ; ou
(c) des radicaux hydroxy, C₁-C₄ alkoxy, C₁-C₄ alkylthio, ou phényl-C₁-C₄-alkoxy, phényl-C₁-C₄-alkylthio, hétérocyclyl, phényl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl ou C₁-C₄ haloalkyl de 1-3 radicaux halo ;
(2) C₁-C₄ haloalkyl de 1-3 radicaux halo ; ou
(3) des radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl ou des radicaux trifluorométhyl ;
chaque R₂₉ est indépendamment le radical hydrogène ou R₃₀ ;
chaque R₃₁ est indépendamment
(1) des radicaux hydrogène ; ou
(2) un radical C₁-C₄ alkyl éventuellement substitué par un radical phényl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, C₁-C₄ alkyl ou des radicaux trifluorométhyl ; et
chaque R₃₂ est indépendamment
(1) des radicaux hydrogène ;
(2) un radical C₁-C₄ alkyl éventuellement substitué par un radical C₃-C₆ cycloalkyl, aryl, hétérocyclyl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo ; ou
(3) un radical aryl, hétéroaryl, hétérocyclyl ou C₃-C₆ cycloalkyl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, C₁-C₄ alkyl ou C₁-C₄ haloalkyl de 1-3 radicaux halo.

4. Le procédé de la revendication 3 dans lequel
R₄ est -Z-Y ; pourvu que le nombre total de radicaux aryl, hétéroaryl, cycloalkyl et hétérocyclyl dans -Z-Y est 0-2 ;
Z est un
(1) radical C₁-C₈ alkyl ou C₂-C₈ alkényl éventuellement substitué par (a) 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, ou halo, et (b) 1-2 radicaux de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl ou C₁-C₂ haloalkyl de 1-3 radicaux halo ;
(2) radical hétérocyclyl éventuellement substitué par 1-2 radicaux de amino, di-(C₁-C₄ alkyl)amino, (C₁-C₄ alkoxy)carbonylamino, hydroxy, C₁-C₄ alkoxy, C₁-C₄ alkylthio ou des radicaux C₁-C₄ alkyl ; ou
(3) radical aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl ou C₁-C₂ haloalkyl de 1-3 radicaux halo ;
Y est un
(1) radical hydrogène ;
(2) radical -C(O)-R₂₀ ;
(3) radical -OR₂₁, -SR₂₁, -S(O)- R₂₀, -S(O)₂- R₂₀ ou -S(O)₂- NR₅R₂₁ ; ou
(4) radical -NR₅R₂₁, -NR₂₂-C(O)-R₂₁, -NR₂₂-C(O)-OR₂₀, -NR₂₂-C(O)-NR₅R₂₁, -NR₂₂-S(O)₂- R₂₀ ou NR₂₂-S(O)₂- NR₅R₂₁ ;
chaque R₅ est indépendamment
(1) des radicaux hydrogène
(2) des radicaux C₁-C₄ alkyl, C₂-C₅ alkényl éventuellement substitués par 1-3 radicaux de amino, di-(C₁-C₄ alkyl)amino, hydroxy, C₁-C₄ alkoxy, C₁-C₄ alkylthio, -SO₃H ou halo ; ou
(3) des radicaux phényl-C₁-C₂-alkyl, hétéroaryl-C₁-C₂-alkyl, hétérocyclyl-C₁-C₂-alkyl, ou C₃-C₆-cycloalkyl-C₁-C₂-alkyl éventuellement substitués par 1-3 radicaux de amino, di-(C₁-C₄ alkyl)amino, hydroxy, C₁-C₄ alkoxy, C₁-C₄ alkylthio, C₁-C₄ alkyl ou C₁-C₂ haloalkyl de 1-3 radicaux halo ;
chaque R₂₀ est indépendamment
(1) des radicaux C₁-C₈ alkyl, C₂-C₅ alkényl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo ou aryl-C₁-C₄-alkoxy, aryl-C₁-C₄-alkylthio, aryl-C₁-C₄-alkylsulfonyl, C₃-C₆ cycloalkyl, hétérocyclyl, aryl ou des radicaux hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl ou C₁-C₂ haloalkyl de 1-3 radicaux halo ;
(2) un radical hétérocyclyl éventuellement substitué par 1-2 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio ou C₁-C₄ alkyl ; ou
(3) des radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl ou C₁-C₂ haloalkyl de 1-3 radicaux halo ;
chaque R₂₁ est indépendamment un radical hydrogène ou R₂₀ ;
chaque R₂₂ est indépendamment
(1) un radical hydrogène ; ou
(2) un radical C₁-C₄ alkyl éventuellement substitué par un radical de phényl ou hétéroaryl éventuellement substitué par 1-3 radicaux de amino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl ou C₁-C₂ haloalkyl de 1-3 radicaux halo ;
R₁₁ est un radical aryl et R₁₂ est un radical hétéroaryl dans lequel les radicaux aryl et hétéroaryl sont éventuellement substitués par 1-2 radicaux de
(1) R₃₀ ;
(2) radicaux halo ; ou
(3) radicaux -C(O)-NR₃₁R₃₂, -OR₂₉, -SR₂₉, -S(O)-R₃₀, -S(O)₂-R₃₀, -S(O)₂ -NR₃₁R₃₂, -NR₃₁R₃₂ ou -NR₃₃-C(O)-R₂₉ ;
chaque R₃₀ est indépendamment
(1) un radical C₁-C₄ alkyl, éventuellement substitué par
(a) des radicaux amino, C₁-C₄ alkylamino ou di-(C₁-C₄-alkyl)amino ; ou
(b) des radicaux hydroxy, C₁-C₄ alkoxy, hétérocyclyl, phényl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl ou des radicaux trifluorométhyl ; ou
(2) un radical C₁-C₂ haloalkyl de 1-3 halo ; ou
(3) des radicaux aryl ou hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkylthio, cyano, halo, C₁-C₄ alkyl ou des radicaux trifluorométhyl ;
chaque R₂₉ est indépendamment un radical hydrogène ou R₃₀ ;
chaque R₃₂ est indépendamment
(1) des radicaux hydrogène ;
(2) un radical C₁-C₄ alkyl éventuellement substitué par un radical phényl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl ou des radicaux trifluorométhyl ; ou
(3) un radical phényl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, hydroxy, C₁-C₄ alkoxy, C₁-C₄ alkyl ou des radicaux trifluorométhyl ; et
chaque R₃₃ est indépendamment un radical hydrogène ou C₁-C₄ alkyl.

5. Le procédé de la revendication 4, dans lequel
L est C₁-C₄ alkoxy ou aryloxy, éventuellement substitué par 1-3 radicaux d'halogène ;
X est S ;
R₂ est un radical hydrogène, Na⁺ ou K⁺ ;
Z est un
(1) radical C₁-C₄ alkyl ou C₂-C₅ alkényl éventuellement substitué par (a) 1-3 radicaux de amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio, ou halo, et
(b) 1-2 radicaux de hétérocyclyl, aryl ou hétéroaryl éventuellement substitués par 1-3 radicaux de amino, C₁-C₄ alkylamino, di-(C₁-C₂ alkyl)amino, C₁-C₅ alkanoylamino, (C₁-C₄ alkoxy)carbonylamino, hydroxy, C₁-C₄ alkoxy, C₁-C₄ alkylthio, halo, C₁-C₄ alkyl ou des radicaux trifluorométhyl ;
(2) radical hétérocyclyl éventuellement substitué par 1-2 radicaux de amino, di-(C₁-C₂ alkyl)amino, (C₁-C₄ alkoxy)carbonylamino, hydroxy, C₁-C₂ alkoxy, C₁-C₂ alkylthio ou des radicaux C₁-C₄ alkyl ; ou
(3) radical aryl ou hétéroaryl éventuellement substitué par 1-3 radicaux de amino, di-(C₁-C₂ alkyl)amino, C₁-C₅ alkanoylamino, (C₁-C₄ alkoxy)carbonylamino, hydroxy, C₁-C₂ alkoxy, C₁-C₂ alkylthio, cyano, halo, C₁-C₄ alkyl ou des radicaux trifluorométhyl ;
Y est un
(1) radical hydrogène ;
(2) radical -C(O)-R₂₀ ;
(3) radical -OR₂₁, -SR₂₁, -S(O)- R₂₀, -S(O)₂- R₂₀ ou -S(O)₂- NR₅R₂₁ ; ou
(4) radical -NR₅R₂₁, -NR₂₂-C(O)-R₂₁ ou -NR₂₂-S(O)₂- R₂₀ ;
chaque R₅ est indépendamment
(1) un radical hydrogène
(2) un radical C₁-C₄ alkyl éventuellement substitué par 1-3 radicaux de amino, di-(C₁-C₂ alkyl)amino, hydroxy, C₁-C₂ alkoxy, C₁-C₂ alkylthio ou halo ; ou
(3) des radicaux phényl-C₁-C₂-alkyl, hétéroaryl-C₁-C₂-alkyl, hétérocyclyl-C₁-C₂-alkyl, ou C₃-C₆-cycloalkyl-C₁-C₂-alkyl éventuellement substitués par 1-3 radicaux de amino, di-(C₁-C₂ alkyl)amino, hydroxy, C₁-C₂ alkoxy, C₁-C₂ alkylthio, méthoxy, méthylthio, C₁-C₄ alkyl ou des radicaux trifluorométhyl ;
chaque R₂₀ est indépendamment
(1) des radicaux C₁-C₈ alkyl ou C₂-C₅ alkényl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, N-((C₁-C₄ alkoxy)carbonyl)-N-(C₁-C₄ alkyl)amino, aminocarbonylamino, hydroxy, C₁-C₄ alkoxy, C₁-C₄ alkylthio, C₁-C₄ alkylsulfinyl, C₁-C₄ alkylsulfonyl, halo ou aryl-C₁-C₄-alkoxy, aryl-C₁-C₄-alkylthio, aryl-C₁-C₄-alkylsulfonyl, C₃-C₆ cycloalkyl, hétérocyclyl, aryl ou des radicaux hétéroaryl éventuellement substitués par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, C₁-C₅ alkanoyl, hydroxy, C₁-C₄ alkoxy, C₁-C₄ alkylthio, halo, C₁-C₄ alkyl ou C₁-C₂ haloalkyl de 1-3 radicaux halo ;
(2) un radical hétérocyclyl éventuellement substitué par 1-2 radicaux de amino, di-(C₁-C₄ alkyl)amino, (C₁-C₄ alkoxy)carbonylamino, hydroxy, C₁-C₄ alkoxy, C₁-C₄ alkylthio ou C₁-C₄ alkyl ; ou
(3) des radicaux aryl ou hétéroaryl éventuellement substitués par 1-3 radicaux de amino, C₁-C₄ alkylamino, di-(C₁-C₄ alkyl)amino, acétamido, (C₁-C₄ alkoxy)carbonylamino, C₁-C₄ alkylsulfonylamino, (C₁-C₄ alkoxy)carbonyl, hydroxy, C₁-C₄ alkoxy, C₁-C₄ alkylthio, cyano, halo, azido, C₁-C₄ alkyl ou des radicaux trifluorométhyl ;
chaque R₂₁ est indépendamment un radical hydrogène ou R₂₀ ;
R₁₁ est un radical aryl éventuellement substitué par 1-2 radicaux de (1) R₃₀ ; (2) radicaux halo ; ou (3) radicaux -C(O)-NR₃₁R₃₂, -OR₂₉, -SR₂₉, -S(O)-R₃₀, -S(O)₂-R₃₀, -S(O)₂-NR₃₁R₃₂, -NR₃₁R₃₂ ou -NR₃₃-C(O)-R₂₉ ;
R₁₂ est un radical hétéroaryl éventuellement substitué par 1-2 radicaux de (1) R₃₀ ; (2) radicaux halo ; ou (3) radicaux -C(O)-NR₃₁R₃₂, -OR₂₉, -SR₂₉, -NR₃₁R₃₂, ou -NR₃₃-C(O)-R₂₉ ;
chaque R₃₀ est indépendamment
(1) un radical C₁-C₄ alkyl, éventuellement substitué par un radical phényl ou hétéroaryl éventuellement substitué par 1-3 radicaux de amino, di-(C₁-C₂-alkyl)amino, acétamido, hydroxy, C₁-C₂ alkoxy, halo, C₁-C₄ alkyl ou des radicaux trifluorométhyl ;
(2) un radical trifluorométhyl ; ou
(3) des radicaux aryl ou hétéroaryl éventuellement substitués par 1-3 radicaux de amino, di-(C₁-C₂ alkyl)amino, acétamido, hydroxy, C₁-C₂ alkoxy, halo, C₁-C₄ alkyl ou des radicaux trifluorométhyl ;
chaque R₂₉ est indépendamment un radical hydrogène ou R₃₀ ;
chaque R₃₁ est indépendamment des radicaux hydrogène ou C₁-C₄ alkyl ; et
chaque R₃₂ est indépendamment
(1) des radicaux hydrogène ;
(2) un radical C₁-C₄ alkyl ou C₁-C₂ alkyl éventuellement substitué par un radical phényl ou hétéroaryl éventuellement substitué par 1-3 radicaux de amino, diméthylamino, acétamido, hydroxy, méthoxy, méthyl ou des radicaux trifluorométhyl ; ou
(3) un radical phényl ou hétéroaryl éventuellement substitué par 1-3 radicaux de amino, diméthylamino, acétamido, hydroxy, méthoxy, méthyl ou des radicaux trifluorométhyl.

6. Le procédé de la revendication 5, dans lequel
R₄ est -Z-Y ; pourvu que le nombre total de radicaux aryl, hétéroaryl, cycloalkyl et hétérocyclyl dans -Z-Y est 0-1 ;
Z est un
(1) radical C₁-C₄ alkyl ou C₂-C₅ alkényl éventuellement substitué par (a) 1-3 radicaux de amino, di-(C₁-C₂ alkyl)amino, (C₁-C₄ alkoxy)carbonylamino, hydroxy, C₁-C₂ alkoxy, C₁-C₂ alkylthio, ou halo, et (b) 1-2 radicaux de aryl ou hétéroaryl éventuellement substitué par 1-2 radicaux de amino, di-(C₁-C₂ alkyl)amino, acétamido, (C₁-C₄ alkoxy)carbonylamino, hydroxy, C₁-C₂ alkoxy, C₁-C₂ alkylthio, halo, C₁-C₄ alkyl ou des radicaux trifluorométhyl ; ou
(2) radical aryl ou hétéroaryl éventuellement substitué par 1-3 radicaux de amino, di-(C₁-C₂ alkyl)amino, acétamido (C₁-C₄ alkoxy)carbonylamino, hydroxy, C₁-C₂ alkoxy, C₁-C₂ alkylthio, cyano, halo, C₁-C₄ alkyl ou des radicaux trifluorométhyl ;
chaque R₅ est indépendamment
(1) un radical hydrogène
(2) un radical C₁-C₄ alkyl éventuellement substitué par 1-3 radicaux halo ; ou
(3) des radicaux phényl-C₁-C₂-alkyl, hétéroaryl-C₁-C₂-alkyl éventuellement substitués par 1-3 radicaux de amino, diméthylamino, hydroxy, méthoxy, méthylthio, méthyl ou des radicaux trifluorométhyl ;
chaque R₂₀ est indépendamment
(1) des radicaux C₁-C₆ alkyl éventuellement substitués par 1-3 radicaux de amino, méthylamino, diméthylamino, t-butoxycarbonylamino, N-((t-butoxy)carbonyl)-N-(méthyl)amino, aminocarbonylamino, hydroxy, butoxy, méthoxy, butylthio, méthylthio, méthylsulfinyl, méthylsulfonyl, halo ou C₅-C₆ cycloalkyl, hétérocyclyl, phényl ou des radicaux hétéroaryl éventuellement substitués par 1-2 radicaux de amino, diméthylamino, acétamino, hydroxy, méthoxy, méthylthio, halo, méthyl, ou des radicaux trifluorométhyl ;
(2) un radical hétérocyclyl éventuellement substitué par 1-2 radicaux de hydroxy ou C₁-C₄ alkoxy ; ou
(3) des radicaux aryl ou hétéroaryl éventuellement substitués par 1-2 radicaux de amino, diméthylamino, hydroxy, méthoxy, méthylthio, halo, méthyl ou des radicaux trifluorométhyl ;
chaque R₂₁ est indépendamment un radical hydrogène ou R₂₀ ;
chaque R₂₂ est indépendamment un radical hydrogène ou C₁-C₄ alkyl ;
R₁₁ est un radical aryl et R₁₂ est un radical hétéroaryl dans lequel les radicaux aryl et hétéroaryl sont éventuellement substitués par 1-2 radicaux de
(1) R₃₀ ;
(2) radicaux halo ; ou
(3) radicaux -C(O)-NR₃₁R₃₂, -OR₂₉, -SR₂₉, -S(O)-R₃₀, -S(O)₂-R₃₀, -S(O)₂ -NR₃₁R₃₂, -NR₃₁R₃₂ ou -NR₃₃-C(O)-R₂₉ ;
R₁₁ est un radical aryl éventuellement substitué par 1-2 radicaux de amino, diméthylamino, acétamido, hydroxy, halo, cyano, méthoxy, méthylthio, méthylsulfinyl, méthylsulfonyl, aminocarbonyl, méthyl ou des radicaux trifluorométhyl ;
R₁₂ est un radical hétéroaryl éventuellement substitué par 1-2 radicaux de amino, diméthylamino, acétamido, hydroxy, halo, cyano, méthoxy, méthyl ou des radicaux trifluorométhyl ;
chaque R₃₀ est indépendamment
(1) un radical C₁-C₄ alkyl, éventuellement substitué par un radical phényl ou hétéroaryl éventuellement substitué par 1-3 radicaux de amino, diméthylamino, acétamido, hydroxy, halo, méthoxy, méthyl ou des radicaux trifluorométhyl ;
(2) un radical trifluorométhyl ; ou
(3) des radicaux aryl ou hétéroaryl éventuellement substitués par 1-3 radicaux de amino, diméthylamino, acétamido, hydroxy, halo, méthoxy, méthyl ou des radicaux trifluorométhyl ;
chaque R₂₉ est indépendamment un radical hydrogène ou R₃₀ ;
chaque R₃₂ est indépendamment
(1) un radical hydrogène ou C₁-C₄ alkyl ; ou
(2) un radical phényl ou hétéroaryl éventuellement substitué par 1-2 radicaux de amino, diméthylamino, acétamido, hydroxy, méthoxy, méthyl ou des radicaux trifluorométhyl ; et
chaque R₃₃ est indépendamment le radical hydrogène ou méthyl.

7. Le procédé de la revendication 6, dans lequel
Z est un radical C₁-C₄ alkyl éventuellement substitué par 1-2 radicaux de amino, di-(C₁-C₂ alkyl)amino, (C₁-C₄ alkoxy)carbonylamino, hydroxy, C₁-C₂ alkoxy, C₁-C₂ alkylthio, halo ou aryl ou hétéroaryl éventuellement substitué par 1-2 radicaux de hydroxy, C₁-C₂ alkoxy, C₁-C₂ alkylthio, halo, C₁-C₄ alkyl ou des radicaux trifluorométhyl ; ou
Y est un
(1) radical -C(O)-R₂₀ ;
(2) radical -OR₂₁, -SR₂₁, -S(O)- R₂₀, -S(O)₂- R₂₀ ou -S(O)₂- NR₅R₂₁ ; ou
(3) radical -NR₅R₂₁, -NR₂₂-C(O)-R₂₁ ou NR₂₂-S(O)₂- R₂₀ ;
chaque R₅ est indépendamment un radical hydrogène ou C₁-C₄ alkyl ;
chaque R₂₀ est indépendamment
(1) des radicaux C₁-C₆ alkyl éventuellement substitués par 1-3 radicaux de amino, méthylamino, diméthylamino, t-butoxycarbonylamino, N-((t-butoxy)carbonyl)-N-(méthyl)amino, aminocarbonylamino, hydroxy, butoxy, méthoxy, butylthio, méthylthio, méthylsulfinyl, méthylsulfonyl, halo ou des radicaux C₅-C₆ cycloalkyl, hétérocyclyl, phényl ou hétéroaryl éventuellement substitués par 1-2 radicaux de amino, diméthylamino, acétamino, hydroxy, méthoxy, méthylthio, halo, méthyl, ou des radicaux trifluorométhyl ;
(2) un radical hétérocyclyl ; ou
(3) des radicaux aryl ou hétéroaryl éventuellement substitués par 1-2 radicaux de amino, diméthylamino, hydroxy, méthoxy, méthylthio, halo, méthyl ou des radicaux trifluorométhyl ;
chaque R₂₁ est indépendamment un radical hydrogène ou R₂₀ ;
R₁₁ est un radical phényl non substitué ou un radical naphthyl ou un radical phényl substitué par 1-2 radicaux de amino, diméthylamino, acétamido, hydroxy, halo, cyano, méthoxy, méthylthio, méthylsulfinyl, méthylsulfonyl aminocarbonyl, méthyl ou des radicaux trifluorométhyl ;
R₁₂ est un radical 4-pyridyl, 4-quinolinyl, 4-imidazolyl ou 4-pyrimidinyl éventuellement substitué par un radical de amino, diméthylamino, acétamido, hydroxy, halo, cyano, méthoxy, méthyl ou des radicaux trifluorométhyl ;
chaque R₃₀ est indépendamment
(1) un radical C₁-C₄ alkyl, éventuellement substitué par un radical phényl ou hétéroaryl éventuellement substitué par 1-2 radicaux de amino, diméthylamino, acétamido, hydroxy, halo, méthoxy, méthyl ou des radicaux trifluorométhyl ;
(2) un radical trifluorométhyl ; ou
(3) des radicaux aryl ou hétéroaryl éventuellement substitués par 1-3 radicaux de amino, diméthylamino, acétamido, hydroxy, halo, méthoxy, méthyl ou des radicaux trifluorométhyl ;
chaque R₂₉ est indépendamment un radical hydrogène ou R₃₀ ; et
chaque R₃₁ est indépendamment des radicaux hydrogène, méthyl ou éthyl.

8. Le procédé de la revendication 7, dans lequel
Z est un radical C₁-C₄ alkyl éventuellement substitué par 1-2 radicaux de amino, t-butoxycarbonylamino, diméthylamino, hydroxy, méthoxy, méthylthio ou halo ;
Y est un radical -OR₂₁, -SR₂₁, ou -NR₅R₂₁ ;
chaque R₅ est un radical hydrogène ;
chaque R₂₀ est indépendamment
(1) des radicaux C₁-C₆ alkyl éventuellement substitués par 1-3 radicaux de amino, méthylamino, diméthylamino, hydroxy ou des radicaux phényl ou hétéroaryl éventuellement substitués par 1-2 radicaux de amino, diméthylamino, hydroxy, méthoxy, méthylthio, halo, méthyl, ou des radicaux trifluorométhyl ;
(2) un radical hétérocyclyl ; ou
(3) des radicaux aryl ou hétéroaryl éventuellement substitués par 1-2 radicaux de amino, diméthylamino, hydroxy, méthoxy, méthylthio, halo, méthyl ou des radicaux trifluorométhyl ;
chaque R₂₁ est indépendamment un radical hydrogène ou R₂₀ ;
chaque R₂₂ est indépendamment un radical hydrogène ou méthyl ;
R₁₁ est un radical phényl non substitué ou un radical phényl substitué par 1-2 radicaux de amino, diméthylamino, acétamido, hydroxy, halo, cyano, méthoxy, méthylthio, méthylsulfonyl, méthyl ou des radicaux trifluorométhyl ;
R₁₂ est un radical 4-pyridyl, éventuellement substitué par un radical de amino, diméthylamino, acétamido, hydroxy, halo, cyano, méthoxy, méthyl ou des radicaux trifluorométhyl ;
R₂₉ est des radicaux aryl ou hétéroaryl éventuellement substitués par 1-2 radicaux de amino, diméthylamino, acétamido, hydroxy, halo, méthoxy, méthyl ou des radicaux trifluorométhyl ;

9. Un procédé selon la revendication 1,
comportant l'étape additionnelle de :
(c) ajouter R₂-L' ;
dans lequel L et L' sont chacun indépendamment un radical halo, C₁-C₄ alkylsulfate, arylsulfate, trifluorométhylsulfate, C₁-C₄ alkylsulfinate, arylsulfinate, C₁-C₄ alkylsulfure, arysulfure, C₁-C₄ alkoxy, aryloxy, C₁-C₄ alkylcarbonyloxy éventuellement substitué par 1-3 radicaux halogène, arylcarbonyloxy, N-oxosuccinate, N-oxobenzotriazole, ou cyanure ;
et dans lequel
R₂ est un radical alkyl ou alkényl éventuellement substitué par (a) 1-3 radicaux de halo, alkanoyl, aroyl, alkoxycarbonyl, aryloxycarbonyl, aminocarbonyl, alkylaminocarbonyl, dialkylaminocarbonyl, aminosulfonyl, alkylaminosulfonyl, dialkylaminosulfonyl, alkylsulfonyl, arylsulfonyl, hétéroarylsulfonyl, hydroxy, alkoxy, cyano, et (b) 1-2 radicaux de hétérocyclyl, aryl ou hétéroaryl éventuellement substitués par 1-3 radicaux de amino, alkylamino, dialkylamino, alkanoylamino, alkoxycarbonylamino, alkylsulfonylamino, hydroxy, alkoxy, alkylthio, halo, alkyl, trifluorométhoxy ou des radicaux trifluorométhyl ; ou
(4) un radical hétéroaryl éventuellement substitué par 1-3 radicaux de amino, alkylamino, dialkylamino, alkanoylamino, alkoxycarbonylamino, alkylsulfonylamino, hydroxy, alkoxy, alkylthio, cyano, halo, alkyl, trifluorométhoxy ou des radicaux trifluorométhyl.

10. Le procédé selon la revendication 2, comprenant l'étape additionnelle de :
(c) ajouter R₂-L' ; dans lequel L et L' sont chacun indépendamment un radical halo, C₁-C₄ alkylsulfate, arylsulfate, trifluorométhylsulfate, C₁-C₄ alkylsulfinate, arylsulfinate, C₁-C₄ alkylsulfure, arylsulfure, C₁-C₄ alkoxy, aryloxy, C₁-C₄ alkylcarbonyloxy éventuellement substitué par 1-3 radicaux halogène, arylcarbonyloxy, N-oxosuccinate, N-oxobenzotriazole, ou cyanure ;
et dans lequel R₂ est le radical C₁-C₈ alkyl ou C₂-C₈ alkényl éventuellement substitué par (a) 1-3 radicaux de halo, C₁-C₅ alkanoyl, aroyl, C₁-C₄ alkoxycarbonyl, aryloxycarbonyl, aminocarbonyl, C₁-C₄ alkylaminocarbonyl, di-(C₁-C₄ alkyl)aminocarbonyl, aminosulfonyl, C₁-C₄ alkylaminosulfonyl, di-(C₁-C₄ alkyl)aminosulfonyl, C₁-C₄ alkylsulfonyl, arylsulfonyl, hétéroarylsulfonyl, hydroxy, C₁-C₄ alkoxy, cyano, et (b) 1-2 radicaux de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluorométhoxy ou des radicaux trifluorométhyl ; ou
(4) un radical hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, trifluorométhoxy ou des radicaux trifluorométhyl.

11. Le procédé selon la revendication 3, comprenant l'étape additionnelle de :
(c) ajouter R₂-L' ; dans lequel L et L' sont chacun indépendamment un radical halo, C₁-C₄ alkylsulfate, arylsulfate, trifluorométhylsulfate, C₁-C₄ alkylsulfinate, arylsulfinate, C₁-C₄ alkylsulfure, arylsulfure, C₁-C₄ alkoxy, aryloxy, C₁-C₄ alkylcarbonyloxy éventuellement substitué par 1-3 radicaux halogène, arylcarbonyloxy, N-oxosuccinate, N-oxobenzotriazole, ou cyanure ;
et dans lequel R₂ est un radical C₁-C₈ alkyl ou C₂-C₈ alkényl éventuellement substitué par (a) 1-3 radicaux de halo, C₁-C₅ alkanoyl, aroyl, C₁-C₄ alkoxycarbonyl, aryloxycarbonyl, aminocarbonyl, C₁-C₄ alkylaminocarbonyl, di-(C₁-C₄ alkyl)aminocarbonyl, aminosulfonyl, C₁-C₄ alkylaminosulfonyl, di-(C₁-C₄ alkyl)aminosulfonyl, C₁-C₄ alkylsulfonyl, arylsulfonyl, hétéroarylsulfonyl, hydroxy, C₁-C₄ alkoxy, cyano, et (b) 1-2 radicaux de hétérocyclyl, aryl ou hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, halo, C₁-C₄ alkyl, trifluorométhoxy ou des radicaux trifluorométhyl ; ou
(4) un radical hétéroaryl éventuellement substitué par 1-3 radicaux de amino, C₁-C₄ alkylamino, di-(C₁-C₄ alkyl)amino, C₁-C₅ alkanoylamino, (C₁-C₄ alkoxy)carbonylamino, C₁-C₄ alkylsulfonylamino, hydroxy, C₁-C₄ alkoxy, C₁-C₄ alkylthio, cyano, halo, C₁-C₄ alkyl, trifluorométhoxy ou des radicaux trifluorométhyl.

12. Le procédé selon la revendication 4, comprenant l'étape additionnelle de :
(c) ajouter R₂-L' ;
dans lequel L est un radical halo, C₁-C₄ alkylsulfate, arylsulfate, trifluorométhylsulfate, C₁-C₄ alkylsulfinate, arylsulfinate, C₁-C₄ alkylsulfure, arylsulfure, C₁-C₄ alkoxy, aryloxy, C₁-C₄ alkylcarbonyloxy éventuellement substitué par 1-3 radicaux halogène, arylcarbonyloxy, N-oxosuccinate, N-oxobenzotriazole, ou cyanure ;
et dans lequel
R₂ est un radical C₁-C₈ alkyl éventuellement substitué par (a) 1-3 radicaux de halo, aroyl, C₁-C₄ alkoxycarbonyl, aryloxycarbonyl, C₁-C₄ alkylaminosulfonyl, di-(C₁-C₄ alkyl)aminosullfonyl, C₁-C₄ alkylsulfonyl, arylsulfonyl, hétéroarylsulfonyl, C₁-C₄ alkoxy, cyano, et (b) 1-2 radicaux de hétérocyclyl, aryl ou hétéroaryl éventuellement substitué par 1-3 radicaux de amino, C₁-C₄ alkylamino, di-(C₁-C₄ alkyl)amino, hydroxy, C₁-C₄ alkoxy, halo, C₁-C₄ alkyl, ou des radicaux trifluorométhyl ;
L' est un radical halo, C₁-C₄ alkylsulfate, arylsulfate ou trifluorométhylsulfate.

13. Le procédé selon la revendication 5, comprenant l'étape additionnelle de :
(c) ajouter R₂-L' : dans lequel
R₂ est un radical C₁-C₄ alkyl éventuellement substitué par 1-3 radicaux d'hydrogène, un radical hydrogène, Na⁺ ou K⁺ ; et
L' est un radical halo, trifluorométhylsulphate ou mésylate.

14. Le procédé selon la revendication 6, comprenant l'étape additionnelle de :
(c) ajouter R₂-L' : dans lequel
R₂ est un radical méthyl ou éthyl et
L' est un iodure.

15. Le procédé selon la revendication 7, comprenant l'étape additionnelle de :
(c) ajouter R₂-L' : dans lequel
R₂ est un radical méthyl ou éthyl ; et
L' est un iodure.

16. Le procédé selon la revendication 8, comprenant l'étape additionnelle de :
(c) ajouter R₂-L' : dans lequel
R₂ est un radical méthyl ou éthyl ; et
L' est un iodure.
